(19) Europäisches Patentamt
European Patent Office
Office européen des brevets

(11) EP 2 869 847 B1

(12) **EUROPEAN PATENT SPECIFICATION**

(45) Date of publication and mention of the grant of the patent:
**06.12.2017 Bulletin 2017/49**

(21) Application number: **13737118.3**

(22) Date of filing: **08.07.2013**

(51) Int Cl.:
***A61K 47/68*** *(2017.01)* ***A61P 35/00*** *(2006.01)*

(86) International application number:
**PCT/US2013/049519**

(87) International publication number:
**WO 2014/011521 (16.01.2014 Gazette 2014/03)**

(54) **IMMUNOCONJUGATES COMPRISING ANTI-CD79B ANTIBODIES**

IMMUNKONJUGATE MIT ANTI-CD79B-ANTIKÖRPERN

IMMUNOCONJUGUÉS COMPRENANT DES ANTICORPS ANTI-CD79B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.07.2012 US 201261669268 P**
**15.11.2012 US 201261726742 P**

(43) Date of publication of application:
**13.05.2015 Bulletin 2015/20**

(73) Proprietor: **Genentech, Inc.**
**South San Francisco, CA 94080 (US)**

(72) Inventors:
- **POLAKIS, Paul**
**South San Francisco, California 94080 (US)**
- **POLSON, Andrew**
**South San Francisco, California 94080 (US)**
- **SPENCER, Susan Diane**
**South San Francisco, California 94080 (US)**
- **YU, Shang-Fan**
**South San Francisco, California 94080 (US)**
- **ZHENG, Bing**
**South San Francisco, California 94080 (US)**

(74) Representative: **Woolley, Lindsey Claire**
**Mewburn Ellis LLP**
**City Tower**
**40 Basinghall Street**
**London EC2V 5DE (GB)**

(56) References cited:
**US-A1- 2011 076 287** **US-B2- 8 088 378**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 869 847 B1

SEQUENCE LISTING

<110> Genentech, Inc.

<120> ANTI-CD79B ANTIBODIES AND IMMUNOCONJUGATES

<130> GENE-32773/WO-1/ORD

<150> US 61/726,742
<151> 2012-11-15

<150> US 61/669,268
<151> 2012-07-09

<160> 56

<170> PatentIn version 3.5

<210> 1
<211> 113
<212> PRT
<213> Homo sapiens

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Val Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Asp Tyr Trp Gly Gln Gly Thr Leu Val Thr Val Ser
            100                 105                 110

Ser
```

<210> 2
<211> 108
<212> PRT
<213> Homo sapiens

<400> 2

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Leu Pro Trp
                85                  90                  95

Thr Phe Gly Gln Gly Thr Lys Val Glu Ile Lys Arg
            100                 105
```

<210> 3
<211> 117
<212> PRT
<213> Artificial sequence

<220>
<223> Synthetic

<400> 3

```
Glu Val Gln Leu Gln Gln Ser Gly Ala Glu Leu Met Lys Pro Gly Ala
1               5                   10                  15

Ser Val Lys Ile Ser Cys Lys Ala Thr Gly Tyr Thr Phe Ser Ser Tyr
            20                  25                  30

Trp Ile Glu Trp Val Lys Gln Arg Pro Gly His Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Lys Ala Thr Phe Thr Ala Asp Thr Ser Ser Asn Thr Ala Tyr
65                  70                  75                  80

Met Gln Leu Ser Ser Leu Thr Ser Glu Asp Ser Ala Val Tyr Tyr Cys
                85                  90                  95
```

Thr Arg Arg Val Pro Val Tyr Phe Asp Tyr Trp Gly Gln Gly Thr Ser
100 105 110

Val Thr Val Ser Ser
115

<210> 4
<211> 112
<212> PRT
<213> Artificial sequence

<220>
<223> Synthetic

<400> 4

Asp Ile Val Leu Thr Gln Ser Pro Ala Ser Leu Ala Val Ser Leu Gly
1 5 10 15

Gln Arg Ala Thr Ile Ser Cys Lys Ala Ser Gln Ser Val Asp Tyr Asp
20 25 30

Gly Asp Ser Phe Leu Asn Trp Tyr Gln Gln Lys Pro Gly Gln Pro Pro
35 40 45

Lys Leu Phe Ile Tyr Ala Ala Ser Asn Leu Glu Ser Gly Ile Pro Ala
50 55 60

Arg Phe Ser Gly Ser Gly Ser Gly Thr Asp Phe Thr Leu Asn Ile His
65 70 75 80

Pro Val Glu Glu Glu Asp Ala Ala Thr Tyr Tyr Cys Gln Gln Ser Asn
85 90 95

Glu Asp Pro Leu Thr Phe Gly Ala Gly Thr Glu Leu Glu Leu Lys Arg
100 105 110

<210> 5
<211> 117
<212> PRT
<213> Artificial sequence

<220>
<223> Synthetic

<400> 5

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Val
        35                  40                  45

Gly Glu Ile Leu Pro Gly Gly Gly Asp Thr Asn Tyr Asn Glu Ile Phe
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Val Tyr Phe Asp Tyr Trp Gly Gln Gly Thr Leu
            100                 105                 110

Val Thr Val Ser Ser
        115

<210> 6
<211> 112
<212> PRT
<213> Artificial sequence

<220>
<223> Synthetic

<400> 6

```
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Asp
            20                  25                  30

Gly Asp Ser Phe Leu Asn Trp Tyr Gln Gln Lys Pro Gly Lys Ala Pro
        35                  40                  45

Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110
```

<210> 7
<211> 117
<212> PRT
<213> Artificial sequence

<220>
<223> Synthetic

<400> 7

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Arg Ala Thr Phe Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Val Tyr Phe Asp Tyr Trp Gly Gln Gly Thr Leu
            100                 105                 110

Val Thr Val Ser Ser
        115
```

<210> 8
<211> 112
<212> PRT
<213> Artificial sequence

<220>
<223> Synthetic

<400> 8

```
Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Asp
            20                  25                  30

Gly Asp Ser Phe Leu Asn Trp Tyr Gln Gln Lys Pro Gly Lys Ala Pro
        35                  40                  45
```

```
Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110
```

<210> 9
<211> 117
<212> PRT
<213> Artificial sequence

<220>
<223> Synthetic

<400> 9

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Arg Ala Thr Phe Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Ile Arg Leu Asp Tyr Trp Gly Gln Gly Thr Leu
            100                 105                 110

Val Thr Val Ser Ser
        115
```

<210> 10
<211> 112
<212> PRT

<213> Artificial sequence

<220>
<223> Synthetic

<400> 10

```
Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Asp
            20                  25                  30

Gly Asp Ser Phe Leu Asn Trp Tyr Gln Gln Lys Pro Gly Lys Ala Pro
        35                  40                  45

Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110
```

<210> 11
<211> 117
<212> PRT
<213> Artificial sequence

<220>
<223> Synthetic

<400> 11

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Arg Ala Thr Phe Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Ile Arg Leu Asp Tyr Trp Gly Gln Gly Thr Leu
            100                 105                 110

Val Thr Val Ser Ser
        115

<210> 12
<211> 112
<212> PRT
<213> Artificial sequence

<220>
<223> Synthetic

<400> 12

Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Glu
            20                  25                  30

Gly Asp Ser Phe Leu Asn Trp Tyr Gln Gln Lys Pro Gly Lys Ala Pro
        35                  40                  45

Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110

<210> 13
<211> 117
<212> PRT
<213> Artificial sequence

<220>
<223> Synthetic

<400> 13

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
20 25 30

Trp Ile Glu Trp Val Arg Gln Ala Pro Gly Lys Gly Leu Glu Trp Ile
35 40 45

Gly Glu Ile Leu Pro Gly Gly Gly Asp Thr Asn Tyr Asn Glu Ile Phe
50 55 60

Lys Gly Arg Ala Thr Phe Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Thr Arg Arg Val Pro Ile Arg Leu Asp Tyr Trp Gly Gln Gly Thr Leu
100 105 110

Val Thr Val Ser Ser
115

<210> 14
<211> 112
<212> PRT
<213> Artificial sequence

<220>
<223> Synthetic

<400> 14

Asp Ile Gln Leu Thr Gln Ser Pro Ser Ser Leu Ser Ala Ser Val Gly
1 5 10 15

Asp Arg Val Thr Ile Thr Cys Lys Ala Ser Gln Ser Val Asp Tyr Ser
20 25 30

Gly Asp Ser Phe Leu Asn Trp Tyr Gln Gln Lys Pro Gly Lys Ala Pro
35 40 45

Lys Leu Phe Ile Tyr Ala Ala Ser Asn Leu Glu Ser Gly Val Pro Ser
50 55 60

Arg Phe Ser Gly Ser Gly Ser Gly Thr Asp Phe Thr Leu Thr Ile Ser
65 70 75 80

Ser Leu Gln Pro Glu Asp Phe Ala Thr Tyr Tyr Cys Gln Gln Ser Asn
85 90 95

Glu Asp Pro Leu Thr Phe Gly Gln Gly Thr Lys Val Glu Ile Lys Arg
100 105 110

<210> 15
<211> 10
<212> PRT
<213> Artificial sequence

<220>
<223> Synthetic

<400> 15

Gly Tyr Thr Phe Ser Ser Tyr Trp Ile Glu
1 5 10

<210> 16
<211> 18
<212> PRT
<213> Artificial sequence

<220>
<223> Synthetic

<400> 16

Gly Glu Ile Leu Pro Gly Gly Gly Asp Thr Asn Tyr Asn Glu Ile Phe
1 5 10 15

Lys Gly

<210> 17
<211> 10
<212> PRT
<213> Artificial sequence

<220>
<223> Synthetic

<400> 17

Thr Arg Arg Val Pro Val Tyr Phe Asp Tyr
1 5 10

<210> 18
<211> 15
<212> PRT
<213> Artificial sequence

<220>
<223> Synthetic

<400> 18

Lys Ala Ser Gln Ser Val Asp Tyr Asp Gly Asp Ser Phe Leu Asn
1 5 10 15

<210> 19
<211> 7
<212> PRT
<213> Artificial sequence

<220>
<223> Synthetic

<400> 19

```
Ala Ala Ser Asn Leu Glu Ser
1               5
```

<210> 20
<211> 9
<212> PRT
<213> Artificial sequence

<220>
<223> Synthetic

<400> 20

```
Gln Gln Ser Asn Glu Asp Pro Leu Thr
1               5
```

<210> 21
<211> 10
<212> PRT
<213> Artificial sequence

<220>
<223> Synthetic

<400> 21

```
Gly Tyr Thr Phe Ser Ser Tyr Trp Ile Glu
1               5                   10
```

<210> 22
<211> 18
<212> PRT
<213> Artificial sequence

<220>
<223> Synthetic

<400> 22

```
Gly Glu Ile Leu Pro Gly Gly Gly Asp Thr Asn Tyr Asn Glu Ile Phe
1               5                   10                  15

Lys Gly
```

<210> 23
<211> 10
<212> PRT

<213> Artificial sequence

<220>
<223> Synthetic

<400> 23

```
Thr Arg Arg Val Pro Ile Arg Leu Asp Tyr
1               5                   10
```

<210> 24
<211> 15
<212> PRT
<213> Artificial sequence

<220>
<223> Synthetic

<400> 24

```
Lys Ala Ser Gln Ser Val Asp Tyr Glu Gly Asp Ser Phe Leu Asn
1               5                   10                  15
```

<210> 25
<211> 7
<212> PRT
<213> Artificial sequence

<220>
<223> Synthetic

<400> 25

```
Ala Ala Ser Asn Leu Glu Ser
1               5
```

<210> 26
<211> 9
<212> PRT
<213> Artificial sequence

<220>
<223> Synthetic

<400> 26

```
Gln Gln Ser Asn Glu Asp Pro Leu Thr
1               5
```

<210> 27
<211> 25
<212> PRT
<213> Artificial sequence

<220>
<223> Synthetic

<400> 27

Glu Val Gln Leu Val Glu Ser Gly Gly Gly Leu Val Gln Pro Gly Gly
1 5 10 15

Ser Leu Arg Leu Ser Cys Ala Ala Ser
20 25

<210> 28
<211> 13
<212> PRT
<213> Artificial sequence

<220>
<223> Synthetic

<400> 28

Trp Val Arg Gln Ala Pro Gly Lys Gly Leu Glu Trp Ile
1 5 10

<210> 29
<211> 30
<212> PRT
<213> Artificial sequence

<220>
<223> Synthetic

<400> 29

Arg Ala Thr Phe Ser Ala Asp Thr Ser Lys Asn Thr Ala Tyr Leu Gln
1 5 10 15

Met Asn Ser Leu Arg Ala Glu Asp Thr Ala Val Tyr Tyr Cys
20 25 30

<210> 30
<211> 11
<212> PRT
<213> Artificial sequence

<220>
<223> Synthetic

<400> 30

Trp Gly Gln Gly Thr Leu Val Thr Val Ser Ser
1 5 10

<210> 31
<211> 23
<212> PRT
<213> Artificial sequence

<220>
<223> Synthetic

<400> 31

Asp Ile Gln Leu Thr Gln Ser Pro Ser Ser Leu Ser Ala Ser Val Gly
1 5 10 15

Asp Arg Val Thr Ile Thr Cys
20

<210> 32
<211> 15
<212> PRT
<213> Artificial sequence

<220>
<223> Synthetic

<400> 32

Trp Tyr Gln Gln Lys Pro Gly Lys Ala Pro Lys Leu Leu Ile Tyr
1 5 10 15

<210> 33
<211> 32
<212> PRT
<213> Artificial sequence

<220>
<223> Synthetic

<400> 33

Gly Val Pro Ser Arg Phe Ser Gly Ser Gly Ser Gly Thr Asp Phe Thr
1 5 10 15

Leu Thr Ile Ser Ser Leu Gln Pro Glu Asp Phe Ala Thr Tyr Tyr Cys
20 25 30

<210> 34
<211> 11
<212> PRT
<213> Artificial sequence

<220>
<223> Synthetic

<400> 34

Phe Gly Gln Gly Thr Lys Val Glu Ile Lys Arg
1 5 10

<210> 35
<211> 15
<212> PRT
<213> Artificial sequence

<220>

<223> Synthetic

<400> 35

Lys Ala Ser Gln Ser Val Asp Tyr Ser Gly Asp Ser Phe Leu Asn
1 5 10 15

<210> 36
<211> 15
<212> PRT
<213> Artificial sequence

<220>
<223> Synthetic

<400> 36

Trp Tyr Gln Gln Lys Pro Gly Lys Ala Pro Lys Leu Leu Phe Tyr
1 5 10 15

<210> 37
<211> 218
<212> PRT
<213> Artificial sequence

<220>
<223> Synthetic

<400> 37

```
Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Glu
            20                  25                  30

Gly Asp Ser Phe Leu Asn Trp Tyr Gln Gln Lys Pro Gly Lys Ala Pro
        35                  40                  45

Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 38
<211> 446
<212> PRT
<213> Artificial sequence

<220>

<223> Synthetic

<400> 38

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Arg Ala Thr Phe Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Ile Arg Leu Asp Tyr Trp Gly Gln Gly Thr Leu
            100                 105                 110
```

```
Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365
```

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly
435 440 445

<210> 39
<211> 446
<212> PRT
<213> Artificial sequence

<220>
<223> Synthetic

<400> 39

Glu Val Gln Leu Val Glu Ser Gly Gly Gly Leu Val Gln Pro Gly Gly
1 5 10 15

Ser Leu Arg Leu Ser Cys Ala Ala Ser Gly Tyr Thr Phe Ser Ser Tyr
20 25 30

Trp Ile Glu Trp Val Arg Gln Ala Pro Gly Lys Gly Leu Glu Trp Ile
35 40 45

Gly Glu Ile Leu Pro Gly Gly Gly Asp Thr Asn Tyr Asn Glu Ile Phe
50 55 60

Lys Gly Arg Ala Thr Phe Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Thr Arg Arg Val Pro Ile Arg Leu Asp Tyr Trp Gly Gln Gly Thr Leu
100 105 110

Val Thr Val Ser Ser Cys Ser Thr Lys Gly Pro Ser Val Phe Pro Leu
115 120 125

```
Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380
```

```
Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445
```

<210> 40
<211> 229
<212> PRT
<213> Homo sapiens

<400> 40

```
Met Ala Arg Leu Ala Leu Ser Pro Val Pro Ser His Trp Met Val Ala
1               5                   10                  15

Leu Leu Leu Leu Leu Ser Ala Glu Pro Val Pro Ala Ala Arg Ser Glu
            20                  25                  30

Asp Arg Tyr Arg Asn Pro Lys Gly Ser Ala Cys Ser Arg Ile Trp Gln
        35                  40                  45

Ser Pro Arg Phe Ile Ala Arg Lys Arg Gly Phe Thr Val Lys Met His
    50                  55                  60

Cys Tyr Met Asn Ser Ala Ser Gly Asn Val Ser Trp Leu Trp Lys Gln
65                  70                  75                  80

Glu Met Asp Glu Asn Pro Gln Gln Leu Lys Leu Glu Lys Gly Arg Met
                85                  90                  95

Glu Glu Ser Gln Asn Glu Ser Leu Ala Thr Leu Thr Ile Gln Gly Ile
            100                 105                 110

Arg Phe Glu Asp Asn Gly Ile Tyr Phe Cys Gln Gln Lys Cys Asn Asn
        115                 120                 125

Thr Ser Glu Val Tyr Gln Gly Cys Gly Thr Glu Leu Arg Val Met Gly
    130                 135                 140

Phe Ser Thr Leu Ala Gln Leu Lys Gln Arg Asn Thr Leu Lys Asp Gly
145                 150                 155                 160
```

Ile Ile Met Ile Gln Thr Leu Leu Ile Ile Leu Phe Ile Ile Val Pro
165 170 175

Ile Phe Leu Leu Leu Asp Lys Asp Asp Ser Lys Ala Gly Met Glu Glu
180 185 190

Asp His Thr Tyr Glu Gly Leu Asp Ile Asp Gln Thr Ala Thr Tyr Glu
195 200 205

Asp Ile Val Thr Leu Arg Thr Gly Glu Val Lys Trp Ser Val Gly Glu
210 215 220

His Pro Gly Gln Glu
225

<210> 41
<211> 201
<212> PRT
<213> Homo sapiens

<400> 41

Ala Arg Ser Glu Asp Arg Tyr Arg Asn Pro Lys Gly Ser Ala Cys Ser
1 5 10 15

Arg Ile Trp Gln Ser Pro Arg Phe Ile Ala Arg Lys Arg Gly Phe Thr
20 25 30

Val Lys Met His Cys Tyr Met Asn Ser Ala Ser Gly Asn Val Ser Trp
35 40 45

Leu Trp Lys Gln Glu Met Asp Glu Asn Pro Gln Gln Leu Lys Leu Glu
50 55 60

Lys Gly Arg Met Glu Glu Ser Gln Asn Glu Ser Leu Ala Thr Leu Thr
65 70 75 80

Ile Gln Gly Ile Arg Phe Glu Asp Asn Gly Ile Tyr Phe Cys Gln Gln
85 90 95

Lys Cys Asn Asn Thr Ser Glu Val Tyr Gln Gly Cys Gly Thr Glu Leu
100 105 110

Arg Val Met Gly Phe Ser Thr Leu Ala Gln Leu Lys Gln Arg Asn Thr
115 120 125

Leu Lys Asp Gly Ile Ile Met Ile Gln Thr Leu Leu Ile Ile Leu Phe
130 135 140

Ile Ile Val Pro Ile Phe Leu Leu Leu Asp Lys Asp Asp Ser Lys Ala
145 150 155 160

Gly Met Glu Glu Asp His Thr Tyr Glu Gly Leu Asp Ile Asp Gln Thr
165 170 175

Ala Thr Tyr Glu Asp Ile Val Thr Leu Arg Thr Gly Glu Val Lys Trp
180 185 190

Ser Val Gly Glu His Pro Gly Gln Glu
195 200

<210> 42
<211> 10
<212> PRT
<213> Artificial sequence

<220>
<223> Synthetic

<400> 42

Gly Tyr Glu Phe Ser Arg Ser Trp Met Asn
1 5 10

<210> 43
<211> 18
<212> PRT
<213> Artificial sequence

<220>
<223> Synthetic

<400> 43

Gly Arg Ile Tyr Pro Gly Asp Gly Asp Thr Asn Tyr Ser Gly Lys Phe
1 5 10 15

Lys Gly

<210> 44
<211> 11
<212> PRT
<213> Artificial sequence

<220>
<223> Synthetic

<400> 44

Asp Gly Ser Ser Trp Asp Trp Tyr Phe Asp Val
1 5 10

<210> 45

<211> 16
<212> PRT
<213> Artificial sequence

<220>
<223> Synthetic

<400> 45

```
Arg Ser Ser Gln Ser Ile Val His Ser Val Gly Asn Thr Phe Leu Glu
1               5                   10                  15
```

<210> 46
<211> 7
<212> PRT
<213> Artificial sequence

<220>
<223> Synthetic

<400> 46

```
Lys Val Ser Asn Arg Phe Ser
1               5
```

<210> 47
<211> 9
<212> PRT
<213> Artificial sequence

<220>
<223> Synthetic

<400> 47

```
Phe Gln Gly Ser Gln Phe Pro Tyr Thr
1               5
```

<210> 48
<211> 120
<212> PRT
<213> Artificial sequence

<220>
<223> Synthetic

<400> 48

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Glu Phe Ser Arg Ser
            20                  25                  30

Trp Met Asn Trp Val Arg Gln Ala Pro Gly Lys Gly Leu Glu Trp Val
        35                  40                  45
```

Gly Arg Ile Tyr Pro Gly Asp Gly Asp Thr Asn Tyr Ser Gly Lys Phe
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ala Arg Asp Gly Ser Ser Trp Asp Trp Tyr Phe Asp Val Trp Gly Gln
100 105 110

Gly Thr Leu Val Thr Val Ser Ser
115 120

<210> 49
<211> 112
<212> PRT
<213> Artificial sequence

<220>
<223> Synthetic

<400> 49

Asp Ile Gln Met Thr Gln Ser Pro Ser Ser Leu Ser Ala Ser Val Gly
1 5 10 15

Asp Arg Val Thr Ile Thr Cys Arg Ser Ser Gln Ser Ile Val His Ser
20 25 30

Val Gly Asn Thr Phe Leu Glu Trp Tyr Gln Gln Lys Pro Gly Lys Ala
35 40 45

Pro Lys Leu Leu Ile Tyr Lys Val Ser Asn Arg Phe Ser Gly Val Pro
50 55 60

Ser Arg Phe Ser Gly Ser Gly Ser Gly Thr Asp Phe Thr Leu Thr Ile
65 70 75 80

Ser Ser Leu Gln Pro Glu Asp Phe Ala Thr Tyr Tyr Cys Phe Gln Gly
85 90 95

Ser Gln Phe Pro Tyr Thr Phe Gly Gln Gly Thr Lys Val Glu Ile Lys
100 105 110

<210> 50
<211> 450
<212> PRT
<213> Artificial sequence

<220>
<223> Synthetic

<400> 50

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Glu Phe Ser Arg Ser
            20                  25                  30

Trp Met Asn Trp Val Arg Gln Ala Pro Gly Lys Gly Leu Glu Trp Val
        35                  40                  45

Gly Arg Ile Tyr Pro Gly Asp Gly Asp Thr Asn Tyr Ser Gly Lys Phe
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Asp Gly Ser Ser Trp Asp Trp Tyr Phe Asp Val Trp Gly Gln
            100                 105                 110

Gly Thr Leu Val Thr Val Ser Ser Cys Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
```

```
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 51
<211> 219
<212> PRT

<213> Artificial sequence

<220>
<223> Synthetic

<400> 51

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Ile Val His Ser
            20                  25                  30

Val Gly Asn Thr Phe Leu Glu Trp Tyr Gln Gln Lys Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Phe Gln Gly
                85                  90                  95

Ser Gln Phe Pro Tyr Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 52
<211> 450
<212> PRT
<213> Artificial sequence

<220>
<223> Synthetic

<400> 52

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Glu Phe Ser Arg Ser
            20                  25                  30

Trp Met Asn Trp Val Arg Gln Ala Pro Gly Lys Gly Leu Glu Trp Val
        35                  40                  45

Gly Arg Ile Tyr Pro Gly Asp Gly Asp Thr Asn Tyr Ser Gly Lys Phe
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Asp Gly Ser Ser Trp Asp Trp Tyr Phe Asp Val Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220
```

```
Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Cys Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 53
<211> 219

<212> PRT
<213> Artificial sequence

<220>
<223> Synthetic

<400> 53

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Ile Val His Ser
            20                  25                  30

Val Gly Asn Thr Phe Leu Glu Trp Tyr Gln Gln Lys Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Phe Gln Gly
                85                  90                  95

Ser Gln Phe Pro Tyr Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Cys Thr Lys Ser Phe Asn Arg Gly Glu Cys
    210                 215
```

<210> 54
<211> 218
<212> PRT
<213> Artificial sequence

<220>

<223> Synthetic

<400> 54

```
Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Ser Val Asp Tyr Glu
            20                  25                  30

Gly Asp Ser Phe Leu Asn Trp Tyr Gln Gln Lys Pro Gly Lys Ala Pro
        35                  40                  45

Lys Leu Leu Ile Tyr Ala Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser Asn
                85                  90                  95

Glu Asp Pro Leu Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

            Cys Thr Lys Ser Phe Asn Arg Gly Glu Cys
                210                 215
```

<210> 55
<211> 447
<212> PRT

<213> Artificial sequence

<220>
<223> Synthetic

<400> 55

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Ser Ser Tyr
            20                  25                  30

Trp Ile Glu Trp Val Arg Gln Ala Pro Gly Lys Gly Leu Glu Trp Ile
        35                  40                  45

Gly Glu Ile Leu Pro Gly Gly Gly Asp Thr Asn Tyr Asn Glu Ile Phe
    50                  55                  60

Lys Gly Arg Ala Thr Phe Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Thr Arg Arg Val Pro Ile Arg Leu Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190
```

```
Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Cys
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445
```

<210> 56
<211> 450
<212> PRT
<213> Artificial sequence

<220>
<223> Synthetic

<400> 56

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Glu Phe Ser Arg Ser
            20                  25                  30

Trp Met Asn Trp Val Arg Gln Ala Pro Gly Lys Gly Leu Glu Trp Val
        35                  40                  45

Gly Arg Ile Tyr Pro Gly Asp Gly Asp Thr Asn Tyr Ser Gly Lys Phe
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Asp Gly Ser Ser Trp Asp Trp Tyr Phe Asp Val Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205
```

```
Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

## Description

### FIELD OF THE INVENTION

The present invention relates to immunoconjugates comprising anti-CD79b antibodies and methods of using the same.

### BACKGROUND

CD79 is the signaling component of the B-cell receptor consisting of a covalent heterodimer containing CD79a (Igα, mb-1) and CD79b (Igβ, B29). CD79a and CD79b each contain an extracellular immunoglobulin (Ig) domain, a transmembrane domain, and an intracellular signaling domain, an immunoreceptor tyrosine-based activation motif (ITAM) domain. CD79 is expressed on B cells and, for example, in Non-Hodgkin's Lymphoma cells (NHLs) (Cabezudo et al., Haematologica, 84:413-418 (1999); D'Arena et al., Am. J. Hematol., 64: 275-281 (2000); Olejniczak et al., Immunol. Invest., 35: 93-114 (2006)). CD79a and CD79b and sIg are all required for surface expression of the CD79 (Matsuuchi et al., Curr. Opin. Immunol., 13(3): 270-7)). The average surface expression of CD79b on NHLs is similar to that on normal B-cells, but with a greater range (Matsuuchi et al., Curr. Opin. Immunol., 13(3): 270-7 (2001)).

There is a need in the art for agents that target CD79b for the diagnosis and treatment of CD79b-associated conditions, such as cancer. The invention fulfills that need and provides other benefits.

### SUMMARY

The invention provides anti-CD79b antibodies and immunoconjugates and methods of using the same. Subject-matter which is not encompassed under the scope of the claims does not form part of the presently claimed invention.

An immunoconjugate comprising an antibody that binds CD79b covalently attached to a cytotoxic agent is provided, according to the claims. The cytotoxic agent is a nemorubicin derivative according to the claims. The antibody that binds CD79b comprises (i) HVR-H1 comprising the amino acid sequence of SEQ ID NO: 21, (ii) HVR-H2 comprising the amino acid sequence of SEQ ID NO: 22, and (iii) HVR-H3 comprising the amino acid sequence of SEQ ID NO: 23; (iv) HVR-L1 comprising an amino acid sequence selected from SEQ ID NOs: 18, 24, and 35, (v) HVR-L2 comprising the amino acid sequence of SEQ ID NO: 25, and (vi) HVR-L3 comprising the amino acid sequence of SEQ ID NO: 26. In some embodiments, the antibody comprises HVR-L1 comprising the amino acid sequence of SEQ ID NO: 24.

In some embodiments, the antibody comprises: a) a VH sequence having at least 95% sequence identity to the amino acid sequence of SEQ ID NO: 11; or b) a VL sequence having at least 95% sequence identity to the amino acid sequence of SEQ ID NO: 12; or c) a VH sequence as in (a) and a VL sequence as in (b). In some embodiments, the antibody comprises a VH sequence having an amino acid sequence selected from SEQ ID NOs: 9, 11, and 13. In some embodiments, the antibody comprises a VH sequence having the amino acid sequence of SEQ ID NO: 11. In some embodiments, the antibody comprises a VL sequence having an amino acid sequence selected from SEQ ID NOs: 8, 10, 12, and 14. In some embodiments, the antibody comprises a VL sequence having the amino acid sequence of SEQ ID NO: 12. In some embodiments, the antibody is an IgG1, IgG2a or IgG2b antibody.

In some embodiments, an immunoconjugate comprising an antibody that binds CD79b covalently attached to a cytotoxic agent is provided, according to the claims, wherein the antibody comprises (a) a VH sequence having the amino acid sequence of SEQ ID NO: 11 and a VL sequence having the amino acid sequence of SEQ ID NO: 12, and wherein the cytotoxic agent is a nemorubicin derivative, according to the claims.

In some embodiments, the immunoconjugate has the formula Ab-(L-D)p, wherein: (a) Ab is the antibody; (b) L is a linker; (c) D is the cytotoxic agent; and (d) p ranges from 1-8, according to the claims.

D has a structure selected from:

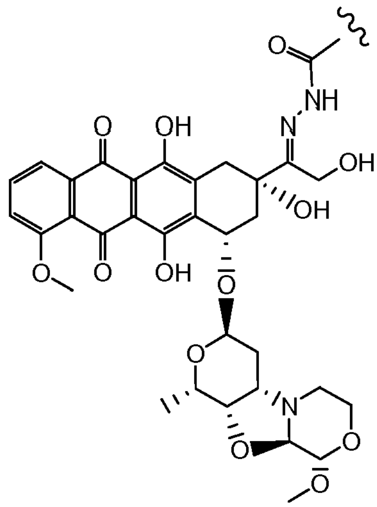

;

and

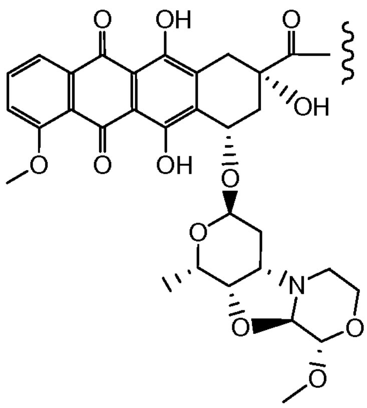

.

In some embodiments, the immunoconjugate comprises a linker that is cleavable by a protease. In some such embodiments, the linker comprises a val-cit dipeptide or a Phe-homoLys dipeptide. In some embodiments, the immunoconjugate comprises a linker that is acid-labile. In some such embodiments, the linker comprises hydrazone.

In some embodiments, the immunoconjugate has a formula selected from:

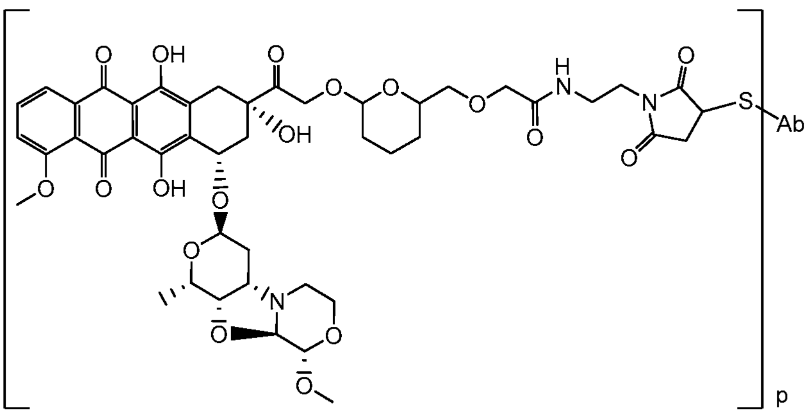

;

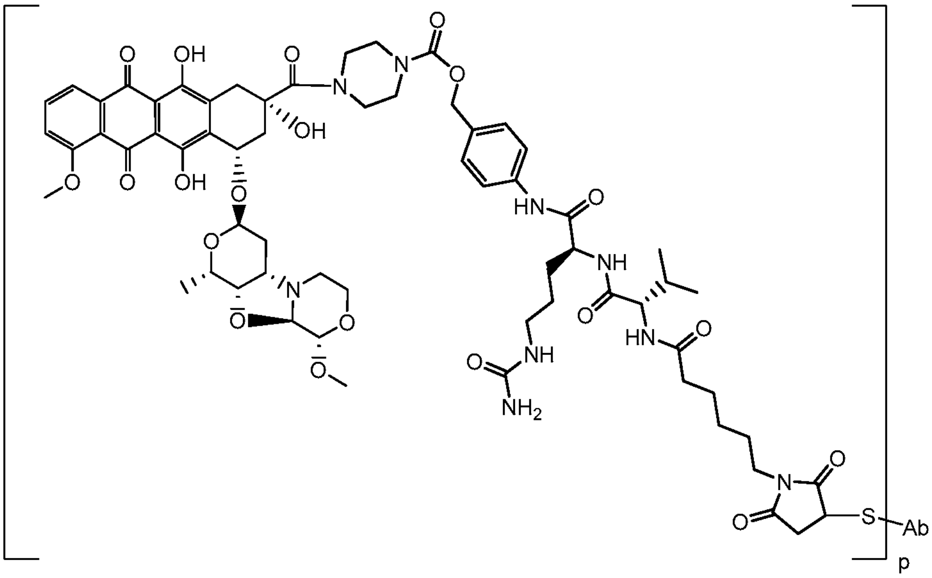

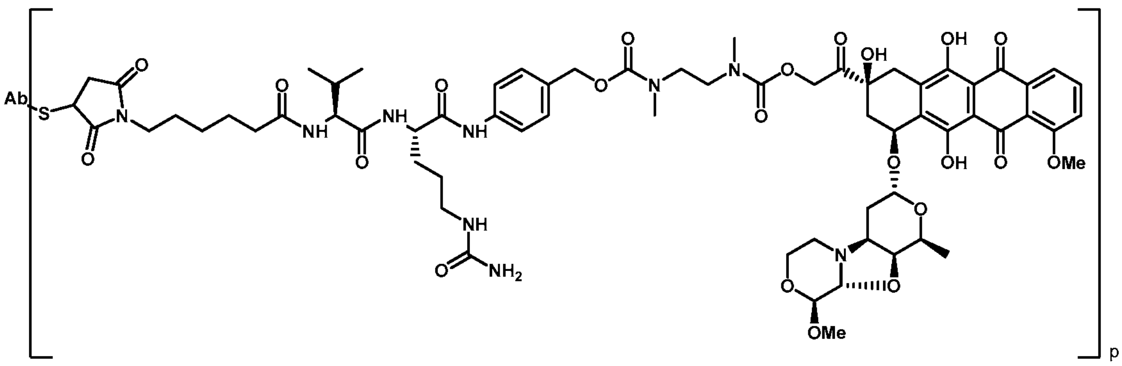

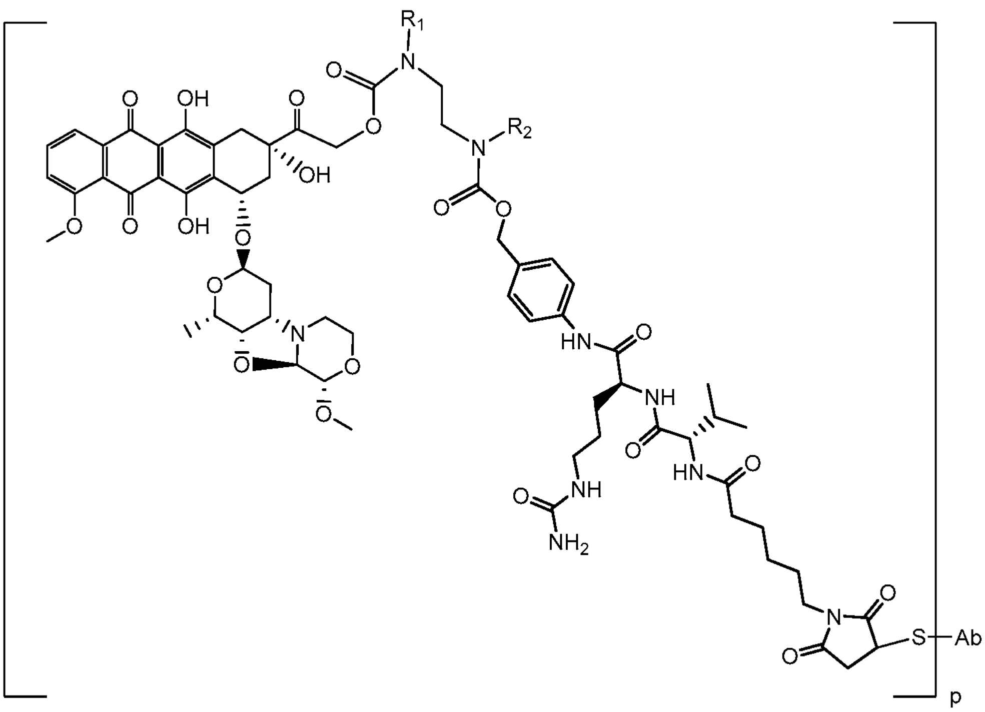

;

and

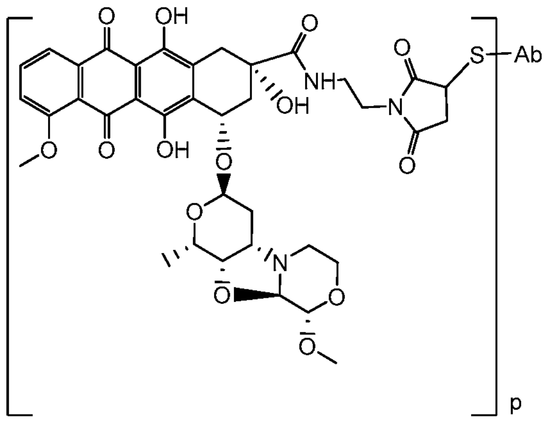

;

wherein $R_1$ and $R_2$ are independently selected from H and $C_1$-$C_6$ alkyl. In some embodiments, p ranges from 1-3.

In some embodiments, an immunoconjugate is provided, wherein the immunoconjugate has a formula selected from:

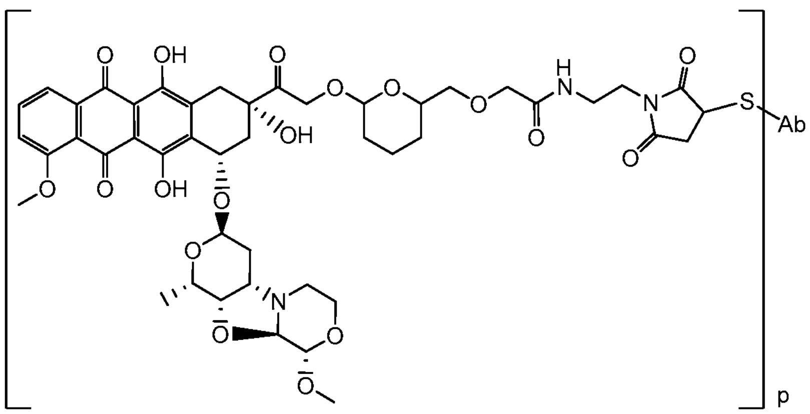

;

and

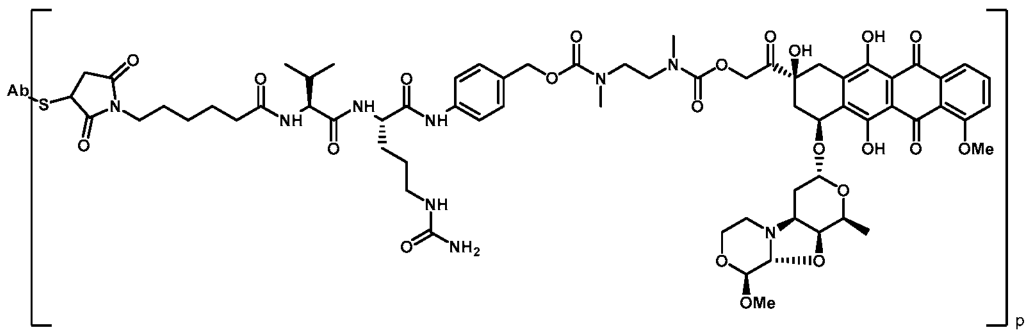

;

wherein Ab is an antibody comprising (i) HVR-H1 comprising the amino acid sequence of SEQ ID NO: 21, (ii) HVR-H2 comprising the amino acid sequence of SEQ ID NO: 22, (iii) HVR-H3 comprising the amino acid sequence of SEQ ID NO: 23, (iv) HVR-L1 comprising the amino acid sequence of SEQ ID NO: 24, (v) HVR-L2 comprising the amino acid sequence of SEQ ID NO: 25, and (vi) HVR-L3 comprising the amino acid sequence of SEQ ID NO: 26; and wherein p ranges from 1 to 3. In some embodiments, the antibody comprises a VH sequence of SEQ ID NO: 11 and a VL sequence

of SEQ ID NO: 12. In some embodiments, the antibody comprises a heavy chain of SEQ ID NO: 39 and a light chain of SEQ ID NO: 37.

In any of the embodiments discussed herein, the antibody may be a monoclonal antibody. In some embodiments, the antibody may be a human, humanized, or chimeric antibody. In some embodiments, the antibody is an antibody fragment that binds CD79b. in some embodiments, the antibody binds human CD79b. In some such embodiments, human CD79b has the sequence of SEQ ID NO: 40 or SEQ ID NO: 41.

In some embodiments, pharmaceutical formulations are provided, wherein the formulation comprises an immunoconjugate described herein and a pharmaceutically acceptable carrier. In some embodiments, the pharmaceutical formulation comprises an additional therapeutic agent.

Methods of treating an individual with a CD79b-positive cancer are disclosed. The method may comprise administering to the individual an effective amount of the immunoconjugate described herein. In some embodiments, the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, the method further comprises administering an additional therapeutic agent to the individual. In some such embodiments, the additional therapeutic agent comprises an antibody that binds CD22. In some embodiments, the additional therapeutic agent is an immunoconjugate comprising an antibody that binds CD22 covalently attached to a cytotoxic agent.

A method of treating an individual having a CD79b-positive cancer, wherein the CD79b-positive cancer is resistant to a first therapeutic is disclosed. In some embodiments, the method comprises administering to the individual an effective amount of an immunoconjugate described herein. In some embodiments, the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, the first therapeutic comprises a first antibody that binds an antigen other than CD79b. In some embodiments, the first therapeutic is a first immunoconjugate comprising a first antibody that binds an antigen other than CD79b and a first cytotoxic agent. In some embodiments, the first antibody binds CD22. In some embodiments, the first therapeutic comprises a first antibody that binds CD79b. In some embodiments, the first therapeutic is a first immunoconjugate comprising a first antibody that binds CD79b and a first cytotoxic agent. In some embodiments, the first cytotoxic agent and the cytotoxic agent of the immunoconjugate described herein are different. In some embodiments, the first cytotoxic agent is MMAE. In some embodiments, the first cytotoxic agent is a pyrrolobenzodiazepine. In some embodiments, the first cytotoxic agent is a maytansinoid. In some embodiments, the CD79b-positive cancer that is resistant to a first therapeutic expresses P-glycoprotein (P-gp).

A method of treating an individual having a CD79b-positive cancer is disclosed, wherein the CD79b-positive cancer expresses P-gp. In some embodiments, the method comprises administering to the individual an effective amount of an immunoconjugate described herein. In some embodiments, the CD79b-positive / P-gp positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, the CD79b positive / P-gp positive cancer expresses higher levels of P-gp mRNA and/or protein than control cells or tissue.

A method of inhibiting proliferation of a CD79b-positive cell is disclosed. In some such embodiments, the method comprises exposing the cell to the immunoconjugate described herein under conditions permissive for binding of the immunoconjugate to CD79b on the surface of the cell, thereby inhibiting proliferation of the cell. In some embodiments, the cell is a neoplastic B cell. In some embodiments, the cell is a lymphoma cell.

## BRIEF DESCRIPTION OF THE FIGURES

**Figures 1A-1B** show the amino acid sequence of the heavy chain variable region of murine anti-CD79b antibody MA79b aligned with the humanized MA79b graft and humanized versions 17, 18, 28, and 32 (huMA79b graft, huMA79bv17, huMA79bv18, huMA79bv28, and huMA79bv32, respectively), and aligned with the human subgroup III sequence. The HVRs are boxed (HVR-H1, HVR-H2, HVR-H3). The sequences bracketing the HVRs are the framework sequences (FR-H1 to FR-H4). The sequences are numbered according to Kabat numbering. The Kabat, Chothia, and contact CDRs are indicated about the boxed HVRs.

**Figures 2A-2B** show the amino acid sequence of the light chain variable region of murine anti-CD79b antibody MA79b aligned with the humanized MA79b graft and humanized versions 17, 18, 28, and 32 (huMA79b graft,

huMA79bv17, huMA79bv18, huMA79bv28, and huMA79bv32, respectively), and aligned with the human subgroup kappa I sequence. The HVRs are boxed. The FR-L1, FR-L2, FR-L3, and FR-L4 sequences bracket the HVRs (HVR-L1, HVR-L2, HVR-L3). The sequences are numbered according to Kabat numbering. The Kabat, Chothia, and contact CDRs are indicated about the boxed HVRs.

**Figure 3** shows the full length amino acid sequences (variable and constant regions) of the light and heavy chains of humanized anti-CD79b antibody huMA79bv28, isotype IgG1. The underlined portions are the constant domains.

**Figure 4** shows the amino acid sequences of the anti-CD79b cysteine engineered antibodies in which the light chain or heavy chain or Fc region is altered to replace an amino acid with a cysteine at selected amino acid positions. The cysteine engineered antibodies shown include Thio-huMA79bv28-HC-A118C heavy chain, in which the alanine at EU position 118 (sequential position alanine 118) is altered to a cysteine; Thio-huMA79b.v28-LC-V205C light chain in which a valine at Kabat position 205 (sequential position valine 209) is altered to a cysteine; and Thio-huMA79b.v28-HC-S400C heavy chain in which a serine at EU position 400 (sequential position serine 400) is altered to a cysteine. In each figure, the altered amino acid is shown in bold text with double underlining. Single underlining indicates constant regions. Variable regions are not underlined.

**Figure 5** shows the linker and drug structures of (A) huMA79bv28-PNU-2; and (B) huMA79bv28-PNU-1, which are described in Example A.

**Figure 6** shows efficacy of various antibody-drug conjugates in a WSU-DLCL2 mouse xenograft model, as described in Example B.

**Figure 7** shows efficacy of various antibody-drug conjugates in a Granta-519 mouse xenograft model, as described in Example C.

**Figure 8** shows efficacy of various antibody-drug conjugates in a SuDHL4-luc mouse xenograft model, as described in Example D.

**Figure 9** shows dose-dependent inhibition of tumor growth by huMA79bv28-PNU-1 in a SuDHL4-luc mouse xenograft model, as described in Example E.

**Figure 10** shows dose-dependent inhibition of tumor growth by huMA79bv28-PNU-1 in a BJAB-luc mouse xenograft model, as described in Example F.

**Figure 11** shows inhibition of tumor growth by huMA79bv28-MMAE in a BJAB-luc mouse xenograft model, as described in Example G.

**Figure 12** shows expression of P-gp in BJAB.Luc_ SN8v28-vcE(CD79b)_T1.1X1 and BJAB.Luc_SN8v28-vcE(CD79b)_T1.2X1 resistant cells and in Bjab-luc cells stably expressing P-gp as determined by FACS, as described in Example H.

**Figure 13** shows dose-dependent inhibition of Bjab-luc cells stably expressing P-gp by PNU-159682, as described in Example H.

## DETAILED DESCRIPTION

### I. DEFINITIONS

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-CD79b antibody" and "an antibody that binds to CD79b" refer to an antibody that is capable of binding CD79b with sufficient affinity such that the antibody is useful as a diagnostic and/or therapeutic agent in targeting CD79b. In one embodiment, the extent of binding of an anti-CD79b antibody to an unrelated, non-CD79b protein is less

than about 10% of the binding of the antibody to CD79b as measured, e.g., by a radioimmunoassay (RIA). In certain embodiments, an antibody that binds to CD79b has a dissociation constant (Kd) of ≤ 1μM, ≤ 100 nM, ≤ 10 nM,, ≤ 5 Nm,, ≤ 4 nM,, ≤ 3 nM,, ≤ 2 nM, ≤ 1 nM, ≤ 0.1 nM, ≤ 0.01 nM, or ≤ 0.001 nM (*e.g.,* $10^{-8}$ M or less, *e.g.* from $10^{-8}$ M to $10^{-13}$ M, *e.g.*, from $10^{-9}$ M to $10^{-13}$ M). In certain embodiments, an anti-CD79b antibody binds to an epitope of CD79b that is conserved among CD79b from different species.

The term "antibody" is used herein in the broadest sense and encompasses various antibody structures, including monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody and that binds the antigen to which the intact antibody binds. Examples of antibody fragments include Fv, Fab, Fab', Fab'-SH, $F(ab')_2$; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "cancer" and "cancerous" refer to or describe the physiological condition in mammals that is typically characterized by unregulated cell growth/proliferation. Examples of cancer include, melanoma, carcinoma, lymphoma (e.g., Hodgkin's and non-Hodgkin's lymphoma), blastoma, sarcoma, and leukemia. More particular examples of cancer include B-cell associated cancers, including for example, high, intermediate and low grade lymphomas (including B cell lymphomas such as, for example, mucosa-associated-lymphoid tissue B cell lymphoma and non-Hodgkin's lymphoma (NHL), mantle cell lymphoma, Burkitt's lymphoma, small lymphocytic lymphoma, marginal zone lymphoma, diffuse large cell lymphoma, follicular lymphoma, and Hodgkin's lymphoma and T cell lymphomas) and leukemias (including secondary leukemia, chronic lymphocytic leukemia (CLL), such as B cell leukemia (CD5+ B lymphocytes), myeloid leukemia, such as acute myeloid leukemia, chronic myeloid leukemia, lymphoid leukemia, such as acute lymphoblastic leukemia (ALL) and myelodysplasia), and other hematological and/or B cell- or T-cell-associated cancers. Also included are cancers of additional hematopoietic cells, including polymorphonuclear leukocytes, such as basophils, eosinophils, neutrophils and monocytes, dendritic cells, platelets, erythrocytes and natural killer cells. Also included are cancerous B cell proliferative disorders selected from the following: lymphoma, non-Hodgkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), and mantle cell lymphoma. The origins of B-cell cancers include as follows: marginal zone B-cell lymphoma origins in memory B-cells in marginal zone, follicular lymphoma and diffuse large B-cell lymphoma originates in centrocytes in the light zone of germinal centers, chronic lymphocytic leukemia and small lymphocytic leukemia originates in B1 cells (CDC5+), mantle cell lymphoma originates in naive B-cells in the mantle zone and Burkitt's lymphoma originates in centroblasts in the dark zone of germinal centers. Tissues which include hematopoietic cells referred herein to as "hematopoietic cell tissues" include thymus and bone marrow and peripheral lymphoid tissues, such as spleen, lymph nodes, lymphoid tissues associated with mucosa, such as the gut-associated lymphoid tissues, tonsils, Peyer's patches and appendix and lymphoid tissues associated with other mucosa, for example, the bronchial linings. Further particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

A "B-cell malignancy" herein includes non-Hodgkin's lymphoma (NHL), including low grade/follicular NHL, small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia, non-Hodgkin's lymphoma (NHL), lymphocyte predominant Hodgkin's disease (LPHD), small lymphocytic lymphoma (SLL), chronic lymphocytic leukemia (CLL), indolent NHL including relapsed indolent NHL and rituximab-refractory indolent NHL; leukemia, including acute lymphoblastic leukemia (ALL), chronic lymphocytic leukemia (CLL), Hairy cell leukemia, chronic myeloblastic leukemia; Burkitt's lymphoma; mantle cell lymphoma; and other hematologic malignancies. Such malignancies may be treated with antibodies directed against B-cell surface markers, such as CD79b. Such diseases are contemplated herein to be treated by the administration of an antibody directed against a B cell surface marker, such as CD79b, and includes the administration of an unconjugated ("naked") antibody or an antibody conjugated to a cytotoxic agent as disclosed herein. Such diseases are also contemplated herein to be treated by combination therapy including an anti-CD79b antibody or anti-CD79b antibody drug conjugate of the invention in combination with another antibody or antibody drug conjugate, another cytoxic agent,

radiation or other treatment administered simultaneously or in series. In an exemplary treatment method, an anti-CD79b immunoconjugate is administered in combination with an anti-CD22 antibody, immunoglobulin, or CD22 binding fragment thereof, either together or sequentially. The anti-CD22 antibody may be a naked antibody or an antibody drug conjugate. In another exemplary treatment method, an anti-CD79b immunoconjugate is administered in combination with an anti-CD20 antibody, immunoglobulin, or CD20 binding fragment thereof, either together or sequentially. The anti-CD20 antibody may be a naked antibody or an antibody drug conjugate. In some embodiments of the combination therapy, the anti-CD79b immunoconjugate is administered with Rituxan® (rituximab).

The term "non-Hodgkin’s lymphoma" or "NHL", as used herein, refers to a cancer of the lymphatic system other than Hodgkin’s lymphomas. Hodgkin’s lymphomas can generally be distinguished from non-Hodgkin’s lymphomas by the presence of Reed-Sternberg cells in Hodgkin’s lymphomas and the absence of said cells in non-Hodgkin’s lymphomas. Examples of non-Hodgkin’s lymphomas encompassed by the term as used herein include any that would be identified as such by one skilled in the art (e.g., an oncologist or pathologist) in accordance with classification schemes known in the art, such as the Revised European-American Lymphoma (REAL) scheme as described in Color Atlas of Clinical Hematology (3rd edition), A. Victor Hoffbrand and John E. Pettit (eds.) (Harcourt Publishers Ltd., 2000). See, in particular, the lists in Fig. 11.57, 11.58 and 11.59. More specific examples include, relapsed or refractory NHL, front line low grade NHL, Stage III/IV NHL, chemotherapy resistant NHL, precursor B lymphoblastic leukemia and/or lymphoma, small lymphocytic lymphoma, B cell chronic lymphocytic leukemia and/or prolymphocytic leukemia and/or small lymphocytic lymphoma, B-cell prolymphocytic lymphoma, immunocytoma and/or lymphoplasmacytic lymphoma, lymphoplasmacytic lymphoma, marginal zone B cell lymphoma, splenic marginal zone lymphoma, extranodal marginal zone - MALT lymphoma, nodal marginal zone lymphoma, hairy cell leukemia, plasmacytoma and/or plasma cell myeloma, low grade/follicular lymphoma, intermediate grade/follicular NHL, mantle cell lymphoma, follicle center lymphoma (follicular), intermediate grade diffuse NHL, diffuse large B-cell lymphoma, aggressive NHL (including aggressive front-line NHL and aggressive relapsed NHL), NHL relapsing after or refractory to autologous stem cell transplantation, primary mediastinal large B-cell lymphoma, primary effusion lymphoma, high grade immunoblastic NHL, high grade lymphoblastic NHL, high grade small non-cleaved cell NHL, bulky disease NHL, Burkitt’s lymphoma, precursor (peripheral) large granular lymphocytic leukemia, mycosis fungoides and/or Sezary syndrome, skin (cutaneous) lymphomas, anaplastic large cell lymphoma, angiocentric lymphoma.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "cytotoxic agent" as used herein refers to a substance that inhibits or prevents a cellular function and/or causes cell death or destruction. Cytotoxic agents include, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma 1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin,

carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., paclitaxel (TAXOL®; Bristol-Myers Squibb Oncology, Princeton, N.J.), ABRAXANE™ Cremophor-free, albumin-engineered nanoparticle formulation of paclitaxel (American Pharmaceutical Partners, Schaumberg, Illinois), and docetaxel (TAXOTERE®; Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone; CVP, an abbreviation for a combined therapy of cyclophosphamide, vincristine, and prednisolone; and FOLFOX, an abbreviation for a treatment regimen with oxaliplatin (ELOXATIN™) combined with 5-FU and leucovorin.

"Effector functions" refer to those biological activities attributable to the Fc region of an antibody, which vary with the antibody isotype. Examples of antibody effector functions include: Clq binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "epitope" refers to the particular site on an antigen molecule to which an antibody binds.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The term "glycosylated forms of CD79b" refers to naturally occurring forms of CD79b that are post-translationally modified by the addition of carbohydrate residues.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or

other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including to a cytotoxic agent.

An "individual" or "subject" is a mammal. Mammals include, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated antibody" is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated nucleic acid" refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-CD79b antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "CD79b," as used herein, refers to any native CD79b from any vertebrate source, including mammals such as primates (e.g. humans, cynomolgus monkey (cyno)) and rodents (e.g., mice and rats), unless otherwise indicated. The term encompasses "full-length," unprocessed CD79b as well as any form of CD79b that results from processing in the cell. The term also encompasses naturally occurring variants of CD79b, e.g., splice variants, allelic variants, and isoforms. The amino acid sequence of an exemplary human CD79b precursor (with signal sequence) is shown in SEQ ID NO: 40. The amino acid sequence of an exemplary human mature CD79b (without signal sequence) is shown in SEQ ID NO: 41.

The term "CD79b-positive cancer" refers to a cancer comprising cells that express CD79b on their surface.

The term "CD79b-positive cell" refers to a cell that expresses CD79b on its surface.

The term "monoclonal antibody" as used herein refers to an antibody obtained from a population of substantially

homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, a buffer, excipient,

stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, immunoconjugates of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

"Alkyl" is $C_1$-$C_{18}$ hydrocarbon containing normal, secondary, tertiary or cyclic carbon atoms. Examples are methyl (Me, $-CH_3$), ethyl (Et, $-CH_2CH_3$), 1-propyl (n-Pr, n-propyl, $-CH_2CH_2CH_3$), 2-propyl (i-Pr, i-propyl, $-CH(CH_3)_2$), 1-butyl (n-Bu, n-butyl, $-CH_2CH_2CH_2CH_3$), 2-methyl-1-propyl (i-Bu, i-butyl, $-CH_2CH(CH_3)_2$), 2-butyl (s-Bu, s-butyl, $-CH(CH_3)CH_2CH_3$), 2-methyl-2-propyl (t-Bu, t-butyl, $-C(CH_3)_3$), 1-pentyl (n-pentyl, $-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), 1-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$.

The term "$C_1$-$C_8$ alkyl," as used herein refers to a straight chain or branched, saturated or unsaturated hydrocarbon having from 1 to 8 carbon atoms. Representative "$C_1$-$C_8$ alkyl" groups include, -methyl, -ethyl, -n-propyl, -n-butyl, -n-pentyl, - n-hexyl, -n-heptyl, -n-octyl, -n-nonyl and -n-decyl; while branched $C_1$-$C_8$ alkyls include, but are not limited to, -isopropyl, -*sec*-butyl, -isobutyl, -*tert*-butyl, -isopentyl, 2-methylbutyl, unsaturated $C_1$-$C_8$ alkyls include, but are not limited to, -vinyl, -allyl, -1-butenyl, -2-butenyl, - isobutylenyl, -1-pentenyl, -2-pentenyl, -3-methyl-1-butenyl, -2-methyl-2-butenyl, - 2,3-dimethyl-2-butenyl, 1-hexyl, 2-hexyl, 3-hexyl,-acetylenyl, -propynyl, -1-butynyl, - 2-butynyl, -1-pentynyl, -2-pentynyl, -3-methyl-1 butynyl. A $C_1$-$C_8$ alkyl group can be unsubstituted or substituted with one or more groups including, $-C_1$-$C_8$ alkyl, -O-($C_1$-$C_8$ alkyl), -aryl, -C(O)R', -OC(O)R', -C(O)OR', $-C(O)NH_2$, -C(O)NHR', $-C(O)N(R')_2$ -NHC(O)R', $-SO_3R'$, $-S(O)_2R'$, -S(O)R', -OH, -halogen, $-N_3$, $-NH_2$, -NH(R'), $-N(R')_2$ and - CN; where each R' is independently selected from H, $-C_1$-$C_8$ alkyl and aryl.

The term "$C_1$-$C_6$ alkyl," as used herein refers to a straight chain or branched, saturated or unsaturated hydrocarbon having from 1 to 6 carbon atoms. Representative "$C_1$-$C_6$ alkyl" groups include, -methyl, -ethyl, -n-propyl, -n-butyl, -n-pentyl, - and n-hexyl; while branched $C_1$-$C_6$ alkyls include, -isopropyl, -*sec*-butyl, -isobutyl, -*tert*-butyl, -isopentyl, and 2-methylbutyl; unsaturated $C_1$-$C_6$ alkyls include, -vinyl, -allyl, -1-butenyl, -2-butenyl, and -isobutylenyl, -1-pentenyl, -2-pentenyl, -3-methyl-1-butenyl, -2-methyl-2-butenyl, -2,3-dimethyl-2-butenyl, 1-hexyl, 2-hexyl, and 3-hexyl. A $C_1$-$C_6$ alkyl group can be unsubstituted or substituted with one or more groups, as described above for $C_1$-$C_8$ alkyl group.

The term "$C_1$-$C_4$ alkyl," as used herein refers to a straight chain or branched, saturated or unsaturated hydrocarbon having from 1 to 4 carbon atoms. Representative "$C_1$-$C_4$ alkyl" groups include, -methyl, -ethyl, -n-propyl, -n-butyl; while branched $C_1$-$C_4$ alkyls include, -isopropyl, -*sec*-butyl, -isobutyl, - *tert*-butyl; unsaturated $C_1$-$C_4$ alkyls include, -vinyl, -allyl, -1-butenyl, - 2-butenyl, and -isobutylenyl. A $C_1$-$C_4$ alkyl group can be unsubstituted or substituted with one or more groups, as described above for $C_1$-$C_8$ alkyl group.

"Alkoxy" is an alkyl group singly bonded to an oxygen. Exemplary alkoxy groups include, methoxy ($-OCH_3$) and ethoxy ($-OCH_2CH_3$). A "$C_1$-$C_5$ alkoxy" is an alkoxy group with 1 to 5 carbon atoms. Alkoxy groups may can be unsubstituted or substituted with one or more groups, as described above for alkyl groups.

"Alkenyl" is $C_2$-$C_{18}$ hydrocarbon containing normal, secondary, tertiary or cyclic carbon atoms with at least one site of unsaturation, i.e. a carbon-carbon, $sp^2$ double bond. Examples include, ethylene or vinyl ($-CH=CH_2$), allyl ($-CH_2CH=CH_2$), cyclopentenyl ($-C_5H_7$), and 5-hexenyl ($-CH_2CH_2CH_2CH_2CH=CH_2$). A "$C_2$-$C_8$ alkenyl" is a hydrocarbon

containing 2 to 8 normal, secondary, tertiary or cyclic carbon atoms with at least one site of unsaturation, i.e. a carbon-carbon, *sp*$^2$ double bond.

"Alkynyl" is $C_2$-$C_{18}$ hydrocarbon containing normal, secondary, tertiary or cyclic carbon atoms with at least one site of unsaturation, i.e. a carbon-carbon, *sp* triple bond. Examples include, acetylenic (-C=CH) and propargyl ($-CH_2C{\equiv}CH$). A "$C_2$-$C_8$ alkynyl" is a hydrocarbon containing 2 to 8 normal, secondary, tertiary or cyclic carbon atoms with at least one site of unsaturation, i.e. a carbon-carbon, *sp* triple bond.

"Alkylene" refers to a saturated, branched or straight chain or cyclic hydrocarbon radical of 1-18 carbon atoms, and having two monovalent radical centers derived by the removal of two hydrogen atoms from the same or two different carbon atoms of a parent alkane. Typical alkylene radicals include, methylene ($-CH_2-$) 1,2-ethyl ($-CH_2CH_2-$), 1,3-propyl ($-CH_2CH_2CH_2-$), 1,4-butyl ($-CH_2CH_2CH_2CH_2-$), and the like.

A "$C_1$-$C_{10}$ alkylene" is a straight chain, saturated hydrocarbon group of the formula $-(CH_2)_{1-10}-$. Examples of a $C_1$-$C_{10}$ alkylene include methylene, ethylene, propylene, butylene, pentylene, hexylene, heptylene, ocytylene, nonylene and decalene.

"Alkenylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, 1,2-ethylene (-CH=CH-).

"Alkynylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkyne. Typical alkynylene radicals acetylene ($-C{\equiv}C-$), propargyl ($-CH_2C{\equiv}C-$), and 4-pentynyl ($-CH_2CH_2CH_2C{\equiv}C-$).

"Aryl" refers to a carbocyclic aromatic group. Examples of aryl groups include, but are not limited to, phenyl, naphthyl and anthracenyl. A carbocyclic aromatic group or a heterocyclic aromatic group can be unsubstituted or substituted with one or more groups including, but not limited to, $-C_1$-$C_8$ alkyl, -O-($C_1$-$C_8$ alkyl), -aryl, -C(O)R', -OC(O)R', -C(O)OR', $-C(O)NH_2$, -C(O)NHR', $-C(O)N(R')_2$-NHC(O)R', $-S(O)_2R'$, -S(O)R', -OH, - halogen, $-N_3$, $-NH_2$, -NH(R'), $-N(R')_2$ and -CN; wherein each R' is independently selected from H, $-C_1$-$C_8$ alkyl and aryl.

A "$C_5$-$C_{20}$ aryl" is an aryl group with 5 to 20 carbon atoms in the carbocyclic aromatic rings. Examples of $C_5$-$C_{20}$ aryl groups include, phenyl, naphthyl and anthracenyl. A $C_5$-$C_{20}$ aryl group can be substituted or unsubstituted as described above for aryl groups. A "$C_5$-$C_{14}$ aryl" is an aryl group with 5 to 14 carbon atoms in the carbocyclic aromatic rings. Examples of $C_5$-$C_{14}$ aryl groups include, phenyl, naphthyl and anthracenyl. A $C_5$-$C_{14}$ aryl group can be substituted or unsubstituted as described above for aryl groups.

An "arylene" is an aryl group which has two covalent bonds and can be in the ortho, meta, or para configurations as shown in the following structures:

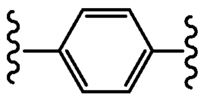

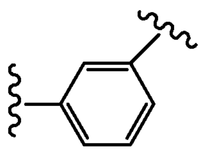

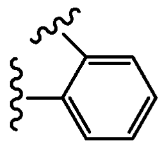

in which the phenyl group can be unsubstituted or substituted with up to four groups including, $-C_1$-$C_8$ alkyl, -O-($C_1$-$C_8$ alkyl), -aryl, -C(O)R', -OC(O)R', -C(O)OR',$-C(O)NH_2$, -C(O)NHR', $-C(O)N(R')_2$-NHC(O)R', $-S(O)_2R'$, -S(O)R', -OH, -halogen, $-N_3$,$-NH_2$, -NH(R'), $-N(R')_2$ and -CN; wherein each R' is independently selected from H, $-C_1$-$C_8$ alkyl and aryl.

"Arylalkyl" refers to an acyclic alkyl radical in which one of the hydrogen atoms bonded to a carbon atom, typically a terminal or $sp^3$ carbon atom, is replaced with an aryl radical. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. The arylalkyl group comprises 6 to 20 carbon atoms, e.g. the alkyl moiety, including alkanyl, alkenyl or alkynyl groups, of the arylalkyl group is 1 to 6 carbon atoms and the aryl moiety is 5 to 14 carbon atoms.

"Heteroarylalkyl" refers to an acyclic alkyl radical in which one of the hydrogen atoms bonded to a carbon atom, typically a terminal or $sp^3$ carbon atom, is replaced with a heteroaryl radical. Typical heteroarylalkyl groups include, 2-benzimidazolylmethyl, 2-furylethyl, and the like. The heteroarylalkyl group comprises 6 to 20 carbon atoms, e.g. the alkyl moiety, including alkanyl, alkenyl or alkynyl groups, of the heteroarylalkyl group is 1 to 6 carbon atoms and the heteroaryl moiety is 5 to 14 carbon atoms and 1 to 3 heteroatoms selected from N, O, P, and S. The heteroaryl moiety of the heteroarylalkyl group may be a monocycle having 3 to 7 ring members (2 to 6 carbon atoms or a bicycle having 7 to 10 ring members (4 to 9 carbon atoms and 1 to 3 heteroatoms selected from N, O, P, and S), for example: a bicyclo [4,5], [5,5], [5,6], or [6,6] system.

"Substituted alkyl," "substituted aryl," and "substituted arylalkyl" mean alkyl, aryl, and arylalkyl respectively, in which one or more hydrogen atoms are each independently replaced with a substituent. Typical substituents include,

-X, -R, $-O^-$, -OR, -SR, $-S^-$, $-NR_2$, $-NR_3$, =NR, $-CX_3$, -CN, -OCN, -SCN, -N=C=O, -NCS, -NO, $-NO_2$, $=N_2$, $-N_3$, NC(=O)R, -C(=O)R, $-C(=O)NR_2$, $-SO_3^-$, $-SO_3H$, $-S(=O)_2R$, $-OS(=O)_2OR$, $-S(=O)_2NR$, -S(=O)R, $-OP(=O)(OR)_2$, $-P(=O)(OR)_2$, $-PO^-_3$, $-PO_3H_2$, -C(=O)R, -C(=O)X, -C(=S)R, $-CO_2R$, $-CO_2^-$, -C(=S)OR, -C(=O)SR, -C(=S)SR, $-C(=O)NR_2$, $-C(=S)NR_2$, $-C(=NR)NR_2$, where each X is independently a halogen: F, Cl, Br, or I; and each R is independently -H, $C_2$-$C_{18}$ alkyl, $C_6$-$C_{20}$ aryl, $C_3$-$C_{14}$ heterocycle, protecting group or prodrug moiety. Alkylene, alkenylene, and alkynylene groups as described above may also be similarly substituted.

"Heteroaryl" and "heterocycle" refer to a ring system in which one or more ring atoms is a heteroatom, e.g. nitrogen, oxygen, and sulfur. The heterocycle radical comprises 3 to 20 carbon atoms and 1 to 3 heteroatoms selected from N, O, P, and S. A heterocycle may be a monocycle having 3 to 7 ring members (2 to 6 carbon atoms and 1 to 3 heteroatoms selected from N, O, P, and S) or a bicycle having 7 to 10 ring members (4 to 9 carbon atoms and 1 to 3 heteroatoms selected from N, O, P, and S), for example: a bicyclo [4,5], [5,5], [5,6], or [6,6] system.

Exemplary heterocycles are described, e.g., in Paquette, Leo A., "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566.

Examples of heterocycles include by way of example pyridyl, dihydroypyridyl, tetrahydropyridyl (piperidyl), thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thienyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, and isatinoyl.

By way of example carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline. Still more typically, nitrogen bonded heterocycles include 1-aziridyl, 1-azetedyl, 1-pyrrolyl, 1-imidazolyl, 1-pyrazolyl, and 1-piperidinyl.

A "$C_3$-$C_8$ heterocycle" refers to an aromatic or non-aromatic $C_3$-$C_8$ carbocycle in which one to four of the ring carbon atoms are independently replaced with a heteroatom from the group consisting of O, S and N. Representative examples of a $C_3$-$C_8$ heterocycle include, benzofuranyl, benzothiophene, indolyl, benzopyrazolyl, coumarinyl, isoquinolinyl, pyrrolyl, thiophenyl, furanyl, thiazolyl, imidazolyl, pyrazolyl, triazolyl, quinolinyl, pyrimidinyl, pyridinyl, pyridonyl, pyrazinyl, pyridazinyl, isothiazolyl, isoxazolyl and tetrazolyl. A $C_3$-$C_8$ heterocycle can be unsubstituted or substituted with up to seven groups including, but not limited to, $-C_1$-$C_8$ alkyl, $-O-(C_1$-$C_8$ alkyl), -aryl, -C(O)R',-OC(O)R', -C(O)OR', $-C(O)NH_2$, -C(O)NHR', $-C(O)N(R')_2$-NHC(O)R', $-S(O)_2R'$, -S(O)R',-OH, -halogen, $-N_3$, $-NH_2$, -NH(R'), $-N(R')_2$ and -CN; wherein each R' is independently selected from H, $-C_1$-$C_8$ alkyl and aryl.

"$C_3$-$C_8$ heterocyclo" refers to a $C_3$-$C_8$ heterocycle group defined above wherein one of the heterocycle group's hydrogen atoms is replaced with a bond. A $C_3$-$C_8$ heterocyclo can be unsubstituted or substituted with up to six groups including, $-C_1$-$C_8$ alkyl, $-O-(C_1$-$C_8$ alkyl), -aryl, -C(O)R', -OC(O)R', -C(O)OR', $-C(O)NH_2$, -C(O)NHR', -$C(O)N(R')_2$-NHC(O)R', $-S(O)_2R'$, -S(O)R', -OH, -halogen, $-N_3$, $-NH_2$, -NH(R'), $-N(R')_2$ and -CN; wherein each R' is independently selected from H, $-C_1$-$C_8$ alkyl and aryl.

"Carbocycle" means a saturated or unsaturated ring having 3 to 7 carbon atoms as a monocycle or 7 to 12 carbon atoms as a bicycle. Monocyclic carbocycles have 3 to 6 ring atoms, still more typically 5 or 6 ring atoms. Bicyclic carbocycles have 7 to 12 ring atoms, e.g. arranged as a bicyclo [4,5], [5,5], [5,6] or [6,6] system, or 9 or 10 ring atoms arranged as a bicyclo [5,6] or [6,6] system. Examples of monocyclic carbocycles include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl,

1-cyclohex-3-enyl, cycloheptyl, and cyclooctyl.

A "$C_3$-$C_8$ carbocycle" is a 3-, 4-, 5-, 6-, 7- or 8-membered saturated or unsaturated non-aromatic carbocyclic ring. Representative $C_3$-$C_8$ carbocycles include, -cyclopropyl, -cyclobutyl, -cyclopentyl, -cyclopentadienyl, -cyclohexyl, - cyclohexenyl, -1,3-cyclohexadienyl, -1,4-cyclohexadienyl, -cycloheptyl, -1,3-cycloheptadienyl, -1,3,5-cycloheptatrienyl, -cyclooctyl, and -cyclooctadienyl. A $C_3$-$C_8$ carbocycle group can be unsubstituted or substituted with one or more groups including, -$C_1$-$C_8$ alkyl, -O-($C_1$-$C_8$ alkyl), -aryl, -C(O)R', -OC(O)R', -C(O)OR', -C(O)$NH_2$, -C(O)NHR', -C(O)N(R')$_2$-NHC(O)R', -S(O)$_2$R', -S(O)R', -OH, -halogen, -$N_3$, -$NH_2$, - NH(R'), -N(R')$_2$ and -CN; where each R' is independently selected from H, -$C_1$-$C_8$ alkyl and aryl.

A "$C_3$-$C_8$ carbocyclo" refers to a $C_3$-$C_8$ carbocycle group defined above wherein one of the carbocycle groups' hydrogen atoms is replaced with a bond.

"Linker" refers to a chemical moiety comprising a covalent bond or a chain of atoms that covalently attaches an antibody to a drug moiety. In various embodiments, linkers include a divalent radical such as an alkyldiyl, an aryldiyl, a heteroaryldiyl, moieties such as: $-(CR_2)_nO(CR_2)_n-$, repeating units of alkyloxy (e.g. polyethylenoxy, PEG, polymethyleneoxy) and alkylamino (e.g. polyethyleneamino, Jeffamine™); and diacid ester and amides including succinate, succinamide, diglycolate, malonate, and caproamide. In various embodiments, linkers can comprise one or more amino acid residues, such as valine, phenylalanine, lysine, and homolysine.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

"Leaving group" refers to a functional group that can be substituted by another functional group. Certain leaving groups are well known in the art, and examples include, a halide (*e.g.*, chloride, bromide, iodide), methanesulfonyl (mesyl), p-toluenesulfonyl (tosyl), trifluoromethylsulfonyl (triflate), and trifluoromethylsulfonate.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include, acetyl, trifluoroacetyl, t-butoxycarbonyl (BOC), benzyloxycarbonyl (CBZ) and 9-fluorenylmethylenoxycarbonyl (Fmoc). For a general description of protecting groups and their use, see T. W. Greene, Protective Groups in Organic Synthesis, John Wiley & Sons, New York, 1991, or a later edition.

## II. COMPOSITIONS AND METHODS

In one aspect, the invention is based, in part, on antibodies that bind to CD79b and immunoconjugates comprising such antibodies. Antibodies and immunoconjugates of the invention are useful, e.g., for the diagnosis or treatment of CD79b-positive cancers.

### A. Exemplary Anti-CD79b Antibodies

In some embodiments, isolated antibodies that bind to CD79b are provided CD79b heterodimerizes with CD79a to form CD79, a B-cell-restricted receptor. CD79b is expressed in various B-cell related disorders and cancers, including

various lymphomas, such as Non-Hodgkin's lymphoma.

An exemplary naturally occurring human CD79b precursor sequence, with signal sequence (amino acids 1-28) is provided in SEQ ID NO: 40, and the corresponding mature CD79b sequence is shown in SEQ ID NO: 41 (corresponding to amino acids 29 to 229 of SEQ ID NO: 40).

In some embodiments, an anti-CD79b antibody binds human CD79b. In some embodiments, an anti-CD79b antibody binds human CD79b with an affinity of ≤ 10 nM, or ≤ 5 nM, or ≤ 4 nM, or ≤ 3 nM, or ≤ 2 nM and optionally ≥ 0.0001 nM, or ≥ 0.001 nM, or ≥ 0.01 nM. Exemplary such antibodies include huMA79bv28 and huMA79bv32, which bind to human CD79b with an affinity of 0.44 nM and 0.24 nM, respectively. See, e.g., US 8,088,378 B2.

*Assays*

Whether an anti-CD79b antibody "binds with an affinity of" ≤ 10 nM, or ≤ 5 nM, or ≤ 4 nM, or ≤ 3 nM, or ≤ 2 nM, is determined using Scatchard analysis, as described, e.g., US 8,088,378 B2. Briefly, $I^{125}$ labeled antibody is competed against serial dilutions of unlabeled antibody in the presence of BJAB cells expressing human CD79b. Following the incubation, cells are washed and cell pellet counts read by a gamma counter. *See, e.g.,* US 8,088,378 B2. Binding affinity, $K_D$, of the antibodies may be determined in accordance with standard Scatchard analysis performed utilizing a non-linear curve fitting program (see, for example, Munson et al., Anal Biochem, 107: 220-239, 1980).

Antibody MA79b and other embodiments

The invention discloses an anti-CD79b antibody or immunoconjugate comprising at least one, two, three, four, five, or six HVRs selected from (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising an amino acid sequence selected from SEQ ID NOs: 24 and 35.

The invention discloses an anti-CD79b antibody or immunoconjugate comprising at least one, two, three, four, five, or six HVRs selected from (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising an amino acid sequence of SEQ ID NO: 24; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising the amino acid sequence of SEQ ID NO: 24.

In one aspect, the invention provides an antibody or immunoconjugate comprising at least one, at least two, or all three VH HVR sequences selected from (a) HVR-H1 comprising the amino acid sequence of SEQ ID NO: 21; (b) HVR-H2 comprising the amino acid sequence of SEQ ID NO: 22; and (c) HVR-H3 comprising the amino acid sequence of SEQ ID NO: 23. In some embodiments, the antibody comprises HVR-H3 comprising the amino acid sequence of SEQ ID NO: 23. In another embodiment, the antibody comprises HVR-H3 comprising the amino acid sequence of SEQ ID NO: 23 and HVR-L3 comprising the amino acid sequence of SEQ ID NO: 26. In a further embodiment, the antibody comprises HVR-H3 comprising the amino acid sequence of SEQ ID NO: 23, HVR-L3 comprising the amino acid sequence of SEQ ID NO: 26, and HVR-H2 comprising the amino acid sequence of SEQ ID NO: 22. In a further embodiment, the antibody comprises (a) HVR-H1 comprising the amino acid sequence of SEQ ID NO: 21; (b) HVR-H2 comprising the amino acid sequence of SEQ ID NO: 22; and (c) HVR-H3 comprising the amino acid sequence of SEQ ID NO: 23.

In another aspect, the invention provides an antibody or immunoconjugate comprising at least one, at least two, or all three VL HVR sequences selected from (a) HVR-L1 comprising an amino acid sequence selected from SEQ ID NOs: 18, 24, and 35; (b) HVR-L2 comprising the amino acid sequence of SEQ ID NO: 25; and (c) HVR-L3 comprising the amino acid sequence of SEQ ID NO: 26. In another aspect, the invention provides an antibody or immunoconjugate comprising at least one, at least two, or all three VL HVR sequences selected from (a) HVR-L1 comprising the amino acid sequence of SEQ ID NO: 24; (b) HVR-L2 comprising the amino acid sequence of SEQ ID NO: 25; and (c) HVR-L3 comprising the amino acid sequence of SEQ ID NO: 26. In one embodiment, the antibody comprises (a) HVR-L1 comprising an amino acid sequence selected from SEQ ID NOs: 24 and 35; (b) HVR-L2 comprising the amino acid sequence of SEQ ID NO: 25; and (c) HVR-L3 comprising the amino acid sequence of SEQ ID NO: 26. In some embodiments, the antibody comprises an HVR-L1 comprising the amino acid sequence of SEQ ID NO: 24 or SEQ ID NO: 35. In some embodiments, the antibody comprises an HVR-L1 comprising the amino acid sequence of SEQ ID NO: 24. In some embodiments, the antibody comprises (a) HVR-L1 comprising the amino acid sequence of SEQ ID NO: 24; (b) HVR-L2 comprising the amino acid sequence of SEQ ID NO: 25; and (c) HVR-L3 comprising the amino acid sequence

of SEQ ID NO: 26. In some embodiments, the antibody comprises (a) HVR-L1 comprising the amino acid sequence of SEQ ID NO: 35; (b) HVR-L2 comprising the amino acid sequence of SEQ ID NO: 25; and (c) HVR-L3 comprising the amino acid sequence of SEQ ID NO: 26.

In another aspect, an antibody or immunoconjugate comprises (a) a VH domain comprising at least one, at least two, or all three VH HVR sequences selected from (i) HVR-H1 comprising the amino acid sequence of SEQ ID NO: 21, (ii) HVR-H2 comprising the amino acid sequence of SEQ ID NO: 22, and (iii) HVR-H3 comprising an amino acid sequence selected from SEQ ID NOs: 17 and 23; and (b) a VL domain comprising at least one, at least two, or all three VL HVR sequences selected from (i) HVR-L1 comprising an amino acid sequence selected from SEQ ID NOs: 18, 24, and 35, (ii) HVR-L2 comprising the amino acid sequence of SEQ ID NO: 25, and (iii) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising the amino acid sequence of SEQ ID NO: 24 or SEQ ID NO: 35.

In another aspect, the invention provides an antibody or immunoconjugate comprising (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: HVR-H3 comprising the amino acid sequence of SEQ ID NO: 23 and/or HVR-L1 comprising an amino acid sequence selected from SEQ ID NOs: 24 and 35. In another aspect, the invention provides an antibody or immunoconjugate comprising (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising the amino acid sequence of SEQ ID NO: 24; (e) HVR-L2 comprising the amino acid sequence of SEQ ID NO: 25; and (f) HVR-L3 comprising the amino acid sequence of SEQ ID NO: 26.

In any of the above embodiments, an anti-CD79b antibody is humanized. In one embodiment, an anti-CD79b antibody comprises HVRs as in any of the above embodiments, and further comprises a human acceptor framework, e.g. a human immunoglobulin framework or a human consensus framework. In certain embodiments, the human acceptor framework is the human VL kappa 1 ($VL_{KI}$) framework and/or the VH framework $VH_{III}$. In some embodiments, a humanized anti-CD79b antibody comprises (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some embodiments, a humanized anti-CD79b antibody comprises (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising the amino acid sequence of SEQ ID NO: 24; (e) HVR-L2 comprising the amino acid sequence of SEQ ID NO: 25; and (f) HVR-L3 comprising the amino acid sequence of SEQ ID NO: 26.

In another aspect, an anti-CD79b antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 11. In certain embodiments, a VH sequence having at least 90%, 91%, 92%, 93%, 94%, 95%, 96%, 97%, 98%, or 99% identity to the amino acid sequence of SEQ ID NO: 11 contains substitutions (e.g., conservative substitutions), insertions, or deletions relative to the reference sequence, but an anti-CD79b antibody comprising that sequence retains the ability to bind to CD79b. In certain embodiments, a total of 1 to 10 amino acids have been substituted, inserted and/or deleted in SEQ ID NO: 11. In certain embodiments, a total of 1 to 5 amino acids have been substituted, inserted and/or deleted in SEQ ID NO: 11. In certain embodiments, substitutions, insertions, or deletions occur in regions outside the HVRs (i.e., in the FRs).

Optionally, the anti-CD79b antibody comprises the VH sequence of any one of SEQ ID NOs: 5, 7, 9, 11, and 13, including post-translational modifications of that sequence. In some embodiments, the anti-CD79b antibody comprises the VH sequence of SEQ ID NO: 11, including post-translational modifications of that sequence. In a particular embodiment, the VH comprises one, two or three HVRs selected from: (a) HVR-H1 comprising the amino acid sequence of SEQ ID NO: 21, (b) HVR-H2 comprising the amino acid sequence of SEQ ID NO: 22, and (c) HVR-H3 comprising the amino acid sequence of SEQ ID NO: 17 or SEQ ID NO: 23.

In some embodiments, an anti-CD79b antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 12. In certain embodiments, a VL sequence having at least 90%, 91%, 92%, 93%, 94%, 95%, 96%, 97%, 98%, or 99% identity to the amino acid sequence of SEQ ID NO: 12 contains substitutions (e.g., conservative substitutions), insertions, or deletions relative to the reference sequence, but an anti-CD79b antibody comprising that sequence retains the ability to bind to CD79b. In certain embodiments, a total of 1 to 10 amino acids have been substituted, inserted and/or deleted in SEQ ID NO: 12. In certain embodiments, a total of 1 to 5 amino acids

have been substituted, inserted and/or deleted in SEQ ID NO: 12. In certain embodiments, the substitutions, insertions, or deletions occur in regions outside the HVRs (i.e., in the FRs). Optionally, the anti-CD79b antibody comprises the VL sequence of any one of SEQ ID NOs: 6, 8, 10, 12, and 14, including post-translational modifications of that sequence. In some embodiments, the anti-CD79b antibody comprises the VL sequence of SEQ ID NO: 12, including post-translational modifications of that sequence. In a particular embodiment, the VL comprises one, two or three HVRs selected from (a) HVR-L1 comprising an amino acid sequence selected from SEQ ID NOs: 18, 24, and 35; (b) HVR-L2 comprising the amino acid sequence of SEQ ID NO: 25; and (c) HVR-L3 comprising the amino acid sequence of SEQ ID NO: 26. In some embodiments, the VL comprises one, two or three HVRs selected from (a) HVR-L1 comprising the amino acid sequence of SEQ ID NO: 24 or SEQ ID NO: 35; (b) HVR-L2 comprising the amino acid sequence of SEQ ID NO: 25; and (c) HVR-L3 comprising the amino acid sequence of SEQ ID NO: 26.

In another aspect, an anti-CD79b antibody is provided, wherein the antibody comprises a VH as in any of the embodiments provided above, and a VL as in any of the embodiments provided above. In some embodiments, the antibody comprises the VH and VL sequences in SEQ ID NO: 11 and SEQ ID NO: 12, respectively, including post-translational modifications of those sequences. In some embodiments, the antibody comprises the VH and VL sequences in SEQ ID NO: 13 and SEQ ID NO: 14, respectively, including post-translational modifications of those sequences. In some embodiments, the antibody comprises the VH and VL sequences in SEQ ID NO: 9 and SEQ ID NO: 10, respectively, including post-translational modifications of those sequences. In some embodiments, the antibody comprises the VH and VL sequences in SEQ ID NO: 7 and SEQ ID NO: 8, respectively, including post-translational modifications of those sequences. In some embodiments, the antibody comprises the heavy chain and light chain sequences in SEQ ID NO: 38 and SEQ ID NO: 37, respectively, including post-translational modifications of those sequences. In some embodiments, the antibody comprises the heavy chain and light chain sequences in SEQ ID NO: 39 and SEQ ID NO: 37, respectively, including post-translational modifications of those sequences. In some embodiments, the antibody comprises the heavy chain and light chain sequences in SEQ ID NO: 38 and SEQ ID NO: 54, respectively, including post-translational modifications of those sequences. In some embodiments, the antibody comprises the heavy chain and light chain sequences in SEQ ID NO: 55 and SEQ ID NO: 37, respectively, including post-translational modifications of those sequences.

In a further aspect, the invention provides an antibody or immunoconjugate that binds to the same epitope as an anti-CD79b antibody provided herein. For example, in certain embodiments, an antibody or immunoconjugate is provided that binds to the same epitope as an anti-CD79b antibody comprising a VH sequence of SEQ ID NO: 11 and a VL sequence of SEQ ID NO: 12.

In a further aspect of the invention, an anti-CD79b antibody according to any of the above embodiments is a monoclonal antibody, including a chimeric, humanized or human antibody. In one embodiment, an anti-CD79b antibody is an antibody fragment, e.g., a Fv, Fab, Fab', scFv, diabody, or $F(ab')_2$ fragment. In another embodiment, the antibody is a substantially full length antibody, e.g., an IgG1 antibody or other antibody class or isotype as defined herein.

In any of the immunoconjugates described above, the antibody may be conjugated to a drug moiety. In some embodiments, the antibody is conjugated to a cytotoxic agent. In some such embodiments, the cytotoxic agent is a nemorubicin derivative, such as PNU-159682. Various exemplary nemorubicin derivatives are discussed herein.

In a further aspect, an anti-CD79b antibody or immunoconjugate according to any of the above embodiments may incorporate any of the features, singly or in combination, as described in Sections 1-7 below.

### *1. Antibody Affinity*

In certain embodiments, an antibody provided herein has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM, and optionally is $\geq 10^{-13}$ M. (e.g. $10^{-8}$ M or less, e.g. from $10^{-8}$ M to $10^{-13}$ M, e.g., from $10^{-9}$ M to $10^{-13}$ M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 $\mu$g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150

μl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE®-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with *N*-ethyl-N'-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 μl/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

### *2. Antibody Fragments*

In certain embodiments, an antibody provided herein is an antibody fragment. Antibody fragments include, Fab, Fab', Fab'-SH, $F(ab')_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including proteolytic digestion of an intact antibody as well as production by recombinant host cells (e.g. *E. coli* or phage), as described herein.

### *3. Chimeric and Humanized Antibodies*

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al.,

Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

***4. Human Antibodies***

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

***5. Library-Derived Antibodies***

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be

cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### *6. Multispecific Antibodies*

An antibody provided herein may be a multispecific antibody, e.g. a bispecific antibody. Multispecific antibodies are monoclonal antibodies that have binding specificities for at least two different sites. In certain embodiments, one of the binding specificities is for CD79b and the other is for any other antigen. In certain embodiments, bispecific antibodies may bind to two different epitopes of CD79b. Bispecific antibodies may also be used to localize cytotoxic agents to cells which express CD79b. Bispecific antibodies can be prepared as full length antibodies or antibody fragments.

Techniques for making multispecific antibodies include, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to CD79b as well as another, different antigen (see, US 2008/0069820, for example).

### *7. Antibody Variants*

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

#### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |

(continued)

| Original Residue | Exemplary Substitutions | Preferred Substitutions |
|---|---|---|
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular

are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex is used to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**b) <u>Glycosylation variants</u>**

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody

variants may have improved CDC function. Such antibody variants are described, e.g., in WO 1997/30087 (Patel et al.); WO 1998/58964 (Raju, S.); and WO 1999/22764 (Raju, S.).

**c) Fc region variants**

In certain embodiments, one or more amino acid modifications may be introduced into the Fc region of an antibody provided herein, thereby generating an Fc region variant. The Fc region variant may comprise a human Fc region sequence (*e.g.*, a human IgG1, IgG2, IgG3 or IgG4 Fc region) comprising an amino acid modification (*e.g.* a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492(1991). Examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered *(i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

**d) Cysteine engineered antibody variants**

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Nonlimiting exemplary cysteine engineered heavy chains and light chains of a huMA79bv28 antibody are shown in Figure 4 (SEQ ID NOs: 39, 54, and 55). Cysteine engineered antibodies may be generated as described, e.g., in U.S. Patent No. 7,521,541.

**e) Antibody Derivatives**

In certain embodiments, an antibody provided herein may be further modified to contain additional nonproteinaceous moieties that are known in the art and readily available. The moieties suitable for derivatization of the antibody include but are not limited to water soluble polymers. Examples of water soluble polymers include, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, wavelengths that do not harm ordinary cells, but which heat the nonproteinaceous moiety to a temperature at which cells proximal to the antibody-nonproteinaceous moiety are killed.

**B. Recombinant Methods and Compositions**

Antibodies may be produced using recombinant methods and compositions, e.g., as described in U.S. Patent No. 4,816,567. In one embodiment, isolated nucleic acid encoding an anti-CD79b antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-CD79b antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-CD79b antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms

(invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR$^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### C. Assays

Anti-CD79b antibodies provided herein may be identified, screened for, or characterized for their physical/chemical properties and/or biological activities by various assays known in the art.

In one aspect, an antibody is tested for its antigen binding activity, e.g., by known methods such as ELISA, BIACore®, FACS, or Western blot.

In another aspect, competition assays may be used to identify an antibody that competes with any of the antibodies described herein for binding to CD79b. In certain embodiments, such a competing antibody binds to the same epitope (e.g., a linear or a conformational epitope) that is bound by an antibody described herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized CD79b is incubated in a solution comprising a first labeled antibody that binds to CD79b (e.g., murine MA79b antibody, humanized MA79b.v17 antibody and/or humanized MA79b.v18 antibody and/or humanized MA79b.v28 and/or humanized MA79b.v32) and a second unlabeled antibody that is being tested for its ability to compete with the first antibody for binding to CD79b. The second antibody may be present in a hybridoma supernatant. As a control, immobilized CD79b is incubated in a solution comprising the first labeled antibody but not the second unlabeled antibody. After incubation under conditions permissive for binding of the first antibody to CD79b, excess unbound antibody is removed, and the amount of label associated with immobilized CD79b is measured. If the amount of label associated with immobilized CD79b is substantially reduced in the test sample relative to the control sample, then that indicates that the second antibody is competing with the first antibody for binding to CD79b. In certain embodiments, immobilized CD79b is present on the surface of a cell or in a membrane preparation obtained from a cell expressing CD79b on its surface. *See* Harlow and Lane (1988) Antibodies: A Laboratory Manual ch.14 (Cold Spring Harbor Laboratory, Cold Spring Harbor, NY).

### D. Immunoconjugates

The invention also discloses immunoconjugates comprising an anti-CD79b antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes (i.e., a radioconjugate).

Immunoconjugates allow for the targeted delivery of a drug moiety to a tumor, and, in some embodiments intracellular accumulation therein, where systemic administration of unconjugated drugs may result in unacceptable levels of toxicity to normal cells (Polakis P. (2005) Current Opinion in Pharmacology 5:382-387).

Antibody-drug conjugates (ADC) are targeted chemotherapeutic molecules which combine properties of both antibodies and cytotoxic drugs by targeting potent cytotoxic drugs to antigen-expressing tumor cells (Teicher, B.A. (2009) Current Cancer Drug Targets 9:982-1004), thereby enhancing the therapeutic index by maximizing efficacy and minimizing off-target toxicity (Carter, P.J. and Senter P.D. (2008) The Cancer Jour. 14(3):154-169; Chari, R.V. (2008) Acc. Chem. Res. 41:98-107.

The ADC compounds of the invention include those with anticancer activity. In some embodiments, the ADC compounds include an antibody conjugated, i.e. covalently attached, to the drug moiety. In some embodiments, the

antibody is covalently attached to the drug moiety through a linker. The antibody-drug conjugates (ADC) of the invention selectively deliver an effective dose of a drug to tumor tissue whereby greater selectivity, i.e. a lower efficacious dose, may be achieved while increasing the therapeutic index ("therapeutic window").

The drug moiety (D) of the antibody-drug conjugates (ADC) may include any compound, moiety or group that has a cytotoxic or cytostatic effect. Exemplary drug moieties include, nemorubicin and its derivatives, such as PNU-159682, that have cytotoxic activity. Examples of such immunoconjugates are discussed in further detail below.

### *1. Exemplary Antibody-drug Conjugates*

An exemplary embodiment of an antibody-drug conjugate (ADC) compound comprises an antibody (Ab) which targets a tumor cell, a drug moiety (D), and a linker moiety (L) that attaches Ab to D. In some embodiments, the antibody is attached to the linker moiety (L) through one or more amino acid residues, such as lysine and/or cysteine.

An exemplary ADC has Formula I:

$$Ab\text{-}(L\text{-}D)_p \qquad \mathbf{I}$$

where p is 1 to about 20. In some embodiments, the number of drug moieties that can be conjugated to an antibody is limited by the number of free cysteine residues. In some embodiments, free cysteine residues are introduced into the antibody amino acid sequence by the methods described herein. Exemplary ADC of Formula I include, antibodies that have 1, 2, 3, or 4 engineered cysteine amino acids (Lyon, R. et al (2012) Methods in Enzym. 502:123-138). In some embodiments, one or more free cysteine residues are already present in an antibody, without the use of engineering, in which case the existing free cysteine residues may be used to conjugate the antibody to a drug. In some embodiments, an antibody is exposed to reducing conditions prior to conjugation of the antibody in order to generate one or more free cysteine residues.

#### a) <u>Exemplary Linkers</u>

A "Linker" (L) is a bifunctional or multifunctional moiety that can be used to link one or more drug moieties (D) to an antibody (Ab) to form an antibody-drug conjugate (ADC) of Formula I. In some embodiments, antibody-drug conjugates (ADC) can be prepared using a Linker having reactive functionalities for covalently attaching to the drug and to the antibody. For example, in some embodiments, a cysteine thiol of an antibody (Ab) can form a bond with a reactive functional group of a linker or a drug-linker intermediate to make an ADC.

In one aspect, a linker has a functionality that is capable of reacting with a free cysteine present on an antibody to form a covalent bond. Exemplary such reactive functionalities include maleimide, haloacetamides, $\alpha$-haloacetyl, activated esters such as succinimide esters, 4-nitrophenyl esters, pentafluorophenyl esters, tetrafluorophenyl esters, anhydrides, acid chlorides, sulfonyl chlorides, isocyanates, and isothiocyanates. *See, e.g.,* the conjugation method at page 766 of Klussman, et al (2004), Bioconjugate Chemistry 15(4):765-773, and the Examples herein.

In some embodiments, a linker has a functionality that is capable of reacting with an electrophilic group present on an antibody. Exemplary such electrophilic groups include, aldehyde and ketone carbonyl groups. In some embodiments, a heteroatom of the reactive functionality of the linker can react with an electrophilic group on an antibody and form a covalent bond to an antibody unit. Exemplary such reactive functionalities include, hydrazide, oxime, amino, hydrazine, thiosemicarbazone, hydrazine carboxylate, and arylhydrazide.

A linker may comprise one or more linker components. Exemplary linker components include 6-maleimidocaproyl ("MC"), maleimidopropanoyl ("MP"), valine-citrulline ("val-cit" or "vc"), alanine-phenylalanine ("ala-phe"), p-aminobenzyloxycarbonyl (a "PAB"), N-Succinimidyl 4-(2-pyridylthio) pentanoate ("SPP"), and 4-(N-maleimidomethyl) cyclohexane-1 carboxylate ("MCC"). Various linker components are known in the art, some of which are described below.

A linker may be a "cleavable linker," facilitating release of a drug. Exemplary cleavable linkers include acid-labile linkers (e.g., comprising hydrazone), protease-sensitive (e.g., peptidase-sensitive) linkers, photolabile linkers, or disulfide-containing linkers (Chari et al., Cancer Research 52:127-131 (1992); US 5208020).

In certain embodiments, a linker has the following Formula II:

$$-A_a\text{-}W_w\text{-}Y_y\text{-} \qquad \mathbf{II}$$

wherein A is a "stretcher unit", and a is an integer from 0 to 1; W is an "amino acid unit", and w is an integer from 0 to 12; Y is a "spacer unit", and y is 0, 1, or 2. An ADC comprising the linker of Formula II has the Formula I(A): Ab-($A_a$-$W_w$-$Y_y$-D)p, wherein Ab, D, and p are defined as above for Formula I. Exemplary embodiments of such linkers are described in U.S. Patent No. 7,498,298.

In some embodiments, a linker component comprises a "stretcher unit" (A) that links an antibody to another

linker component or to a drug moiety. Exemplary stretcher units are shown below (wherein the wavy line indicates sites of covalent attachment to an antibody, drug, or additional linker components):

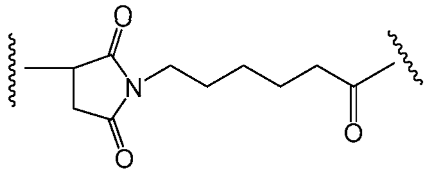

MC

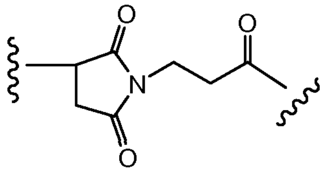

MP

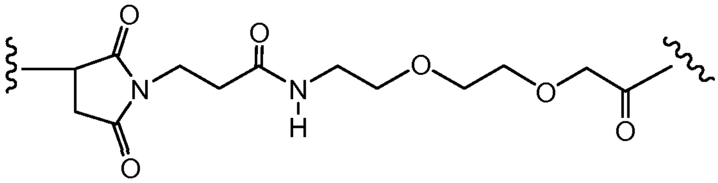

mPEG

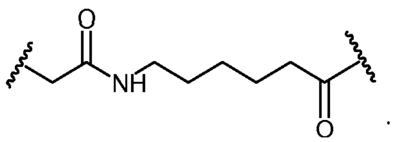

In some embodiments, a linker component comprises an "amino acid unit" (W). In some such embodiments, the amino acid unit allows for cleavage of the linker by a protease, thereby facilitating release of the drug from the immunoconjugate upon exposure to intracellular proteases, such as lysosomal enzymes (Doronina et al. (2003) Nat. Biotechnol. 21:778-784). Exemplary amino acid units include, dipeptides, tripeptides, tetrapeptides, and pentapeptides. Exemplary dipeptides include, valine-citrulline (vc or val-cit), alanine-phenylalanine (af or ala-phe); phenylalanine-lysine (fk or phe-lys); phenylalanine-homolysine (phe-homolys); and N-methyl-valine-citrulline (Me-val-cit). Exemplary tripeptides include, glycine-valine-citrulline (gly-val-cit) and glycine-glycine-glycine (gly-gly-gly). An amino acid unit may comprise amino acid residues that occur naturally and/or minor amino acids and/or non-naturally occurring amino acid analogs, such as citrulline. Amino acid units can be designed and optimized for enzymatic cleavage by a particular enzyme, for example, a tumor-associated protease, cathepsin B, C and D, or a plasmin protease.

Typically, peptide-type linkers can be prepared by forming a peptide bond between two or more amino acids and/or peptide fragments. Such peptide bonds can be prepared, for example, according to a liquid phase synthesis method (e.g., E. Schröder and K. Lübke (1965) "The Peptides", volume 1, pp 76-136, Academic Press).

In some embodiments, a linker component comprises a "spacer unit" (Y) that links the antibody to a drug moiety, either directly or through a stretcher unit and/or an amino acid unit. A spacer unit may be "self-immolative" or a "non-self-immolative." A "non-self-immolative" spacer unit is one in which part or all of the spacer unit remains bound to the drug moiety upon cleavage of the ADC. Examples of non-self-immolative spacer units include, a glycine spacer unit and a glycine-glycine spacer unit. In some embodiments, enzymatic cleavage of an ADC containing a glycine-glycine spacer unit by a tumor-cell associated protease results in release of a glycine-glycine-drug moiety from the remainder of the ADC. In some such embodiments, the glycine-glycine-drug moiety is subjected to a hydrolysis step in the tumor cell, thus cleaving the glycine-glycine spacer unit from the drug moiety.

A "self-immolative" spacer unit allows for release of the drug moiety. In certain embodiments, a spacer unit of a linker comprises a p-aminobenzyl unit. In some such embodiments, a p-aminobenzyl alcohol is attached to an amino acid unit via an amide bond, and a carbamate, methylcarbamate, or carbonate is made between the benzyl alcohol and the drug (Hamann et al. (2005) Expert Opin. Ther. Patents (2005) 15:1087-1103). In some embodiments, the spacer unit comprises p-aminobenzyloxycarbonyl (PAB). In some embodiments, an ADC comprising a self-immolative linker has the structure:

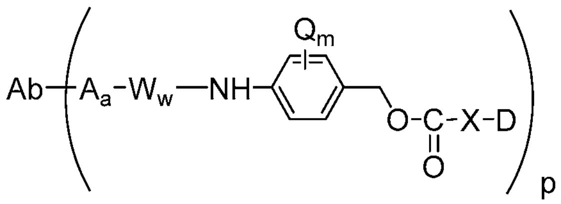

wherein Q is $-C_1-C_8$ alkyl, $-O-(C_1-C_8$ alkyl), -halogen, -nitro, or -cyano; m is an integer ranging from 0 to 4; X may be one or more additional spacer units or may be absent; and p ranges from 1 to about 20. In some embodiments, p ranges from 1 to 10, 1 to 7, 1 to 5, or 1 to 4. Exemplary X spacer units include:

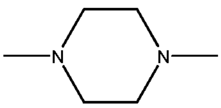 and 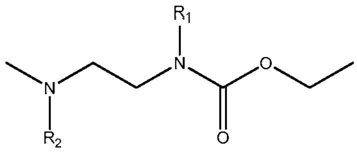 ;

wherein $R_1$ and $R_2$ are independently selected from H and $C_1-C_6$ alkyl. In some embodiments, R1 and R2 are each $-CH_3$.

Other examples of self-immolative spacers include, aromatic compounds that are electronically similar to the PAB group, such as 2-aminoimidazol-5-methanol derivatives (U.S. Patent No. 7,375,078; Hay et al. (1999) Bioorg. Med. Chem. Lett. 9:2237) and ortho- or para-aminobenzylacetals. In some embodiments, spacers can be used that undergo cyclization upon amide bond hydrolysis, such as substituted and unsubstituted 4-aminobutyric acid amides (Rodrigues et al (1995) Chemistry Biology 2:223), appropriately substituted bicyclo[2.2.1] and bicyclo[2.2.2] ring systems (Storm et al (1972) J. Amer. Chem. Soc. 94:5815) and 2-aminophenylpropionic acid amides (Amsberry, et al (1990) J. Org. Chem. 55:5867). Linkage of a drug to the $\alpha$-carbon of a glycine residue is another example of a self-immolative spacer that may be useful in ADC (Kingsbury et al (1984) J. Med. Chem. 27:1447).

In some embodiments, linker L may be a dendritic type linker for covalent attachment of more than one drug moiety to an antibody through a branching, multifunctional linker moiety (Sun et al (2002) Bioorganic & Medicinal Chemistry Letters 12:2213-2215; Sun et al (2003) Bioorganic & Medicinal Chemistry 11:1761-1768). Dendritic linkers can increase the molar ratio of drug to antibody, i.e. loading, which is related to the potency of the ADC. Thus, where an antibody bears only one reactive cysteine thiol group, a multitude of drug moieties may be attached through a dendritic linker.

Exemplary linkers are shown below in the context of an ADC of Formula I:

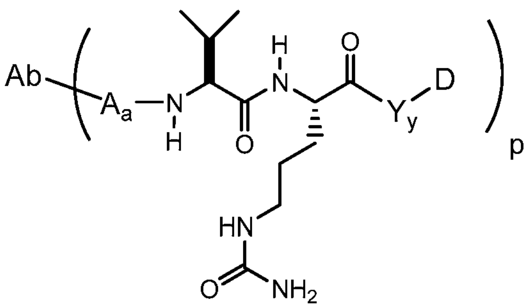

val-cit

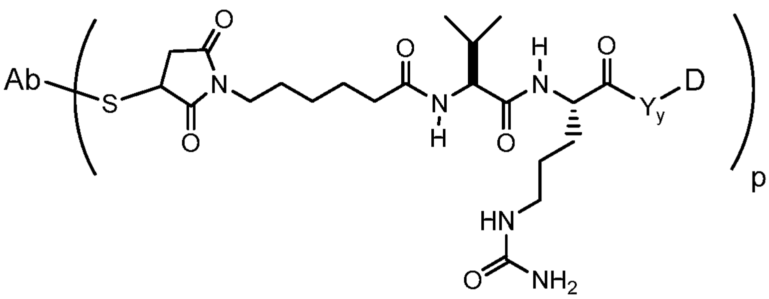

MC-val-cit

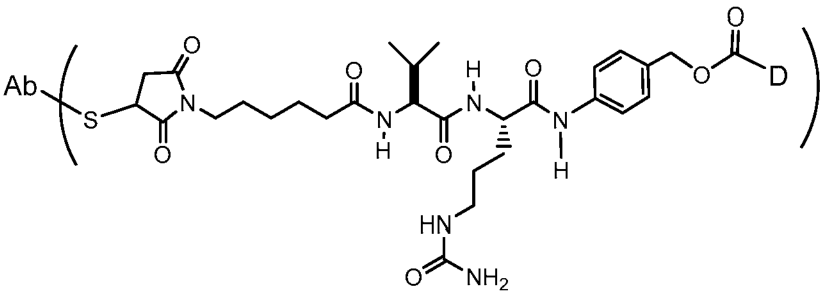

MC-val-cit-PAB

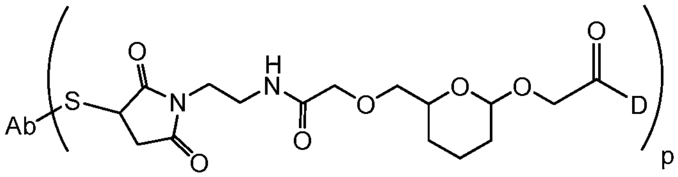

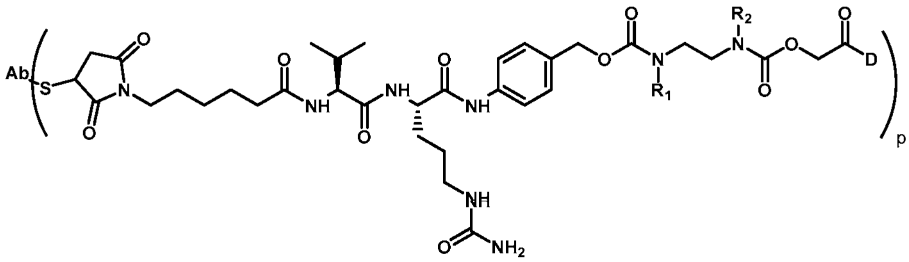

wherein $R_1$ and $R_2$ are independently selected from H and $C_1$-$C_6$ alkyl. In some embodiments, R1 and R2 are each $-CH_3$.

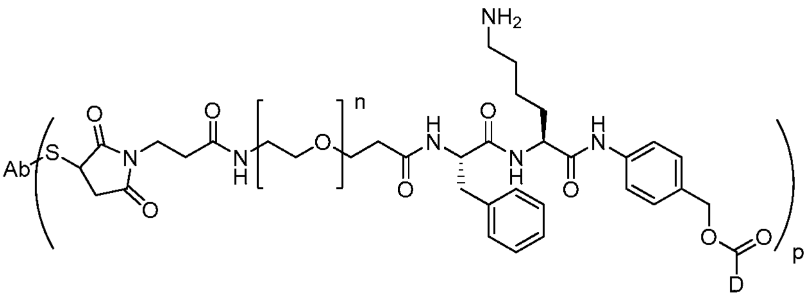

Phe-homoLys-PAB-Ab; wherein n is 0 to 12. In some embodiments, n is 2 to 10. In some embodiments, n is 4 to 8.

Further exemplary ADCs include the structures:

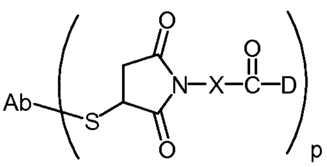
,
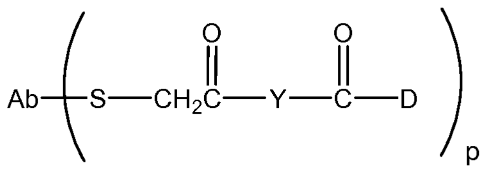
.

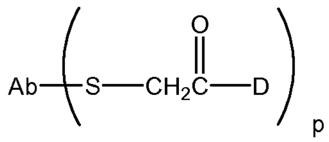,
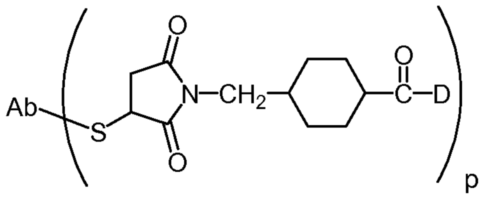.

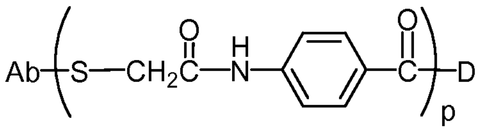.

where X is:

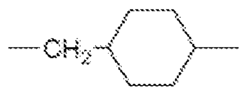,

$-(CH_2)_n-$, $-(CH_2CH_2O)_n-$,

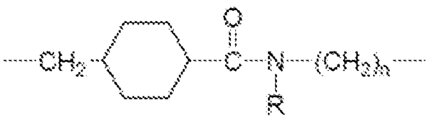, 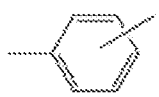,

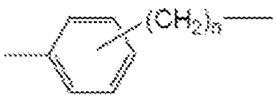, or 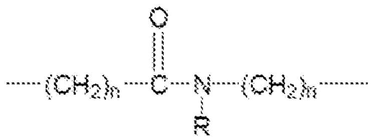

Y is:

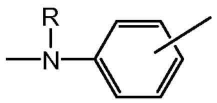 or 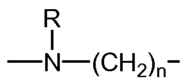 ;

each R is independently H or $C_1$-$C_6$ alkyl; and n is 1 to 12.

In some embodiments, a linker is substituted with groups that modulate solubility and/or reactivity. As an example, a charged substituent such as sulfonate ($-SO_3^-$) or ammonium may increase water solubility of the linker reagent and facilitate the coupling reaction of the linker reagent with the antibody and/or the drug moiety, or facilitate the coupling reaction of Ab-L (antibody-linker intermediate) with D, or D-L (drug-linker intermediate) with Ab, depending on the synthetic route employed to prepare the ADC. In some embodiments, a portion of the linker is coupled to the antibody and a portion of the linker is coupled to the drug, and then the Ab-(linker portion)$^a$ is coupled to drug-(linker portion)$^b$ to form the ADC of Formula I.

The compounds of the invention expressly contemplate, ADC prepared with the following linker reagents: bis-maleimido-trioxyethylene glycol (BMPEO), N-(β-maleimidopropyloxy)-N-hydroxy succinimide ester (BMPS), N-(ε-maleimidocaproyloxy) succinimide ester (EMCS), N-[γ-maleimidobutyryloxy]succinimide ester (GMBS), 1,6-hexane-bis-vinylsulfone (HBVS), succinimidyl 4-(N-maleimidomethyl)cyclohexane-1-carboxy-(6-amidocaproate) (LC-SMCC), m-maleimidobenzoyl-N-hydroxysuccinimide ester (MBS), 4-(4-N-Maleimidophenyl)butyric acid hydrazide (MPBH), succinimidyl 3-(bromoacetamido)propionate (SBAP), succinimidyl iodoacetate (SIA), succinimidyl (4-iodoacetyl)aminobenzoate (SIAB), N-succinimidyl-3-(2-pyridyldithio) propionate (SPDP), N-succinimidyl-4-(2-pyridylthio)pentanoate (SPP), succinimidyl 4-(N-maleimidomethyl)cyclohexane-1-carboxylate (SMCC), succinimidyl 4-(p-maleimidophenyl)butyrate (SMPB), succinimidyl 6-[(beta-maleimidopropionamido)hexanoate] (SMPH), iminothiolane (IT), sulfo-EMCS, sulfo-GMBS, sulfo-KMUS, sulfo-MBS, sulfo-SIAB, sulfo-SMCC, and sulfo-SMPB, and succinimidyl-(4-vinylsulfone)benzoate (SVSB), and including bis-maleimide reagents: dithiobismaleimidoethane (DTME), 1,4-Bismaleimidobutane (BMB), 1,4 Bismaleimidyl-2,3-dihydroxybutane (BMDB), bismaleimidohexane (BMH), bismaleimidoethane (BMOE), BM(PEG)$_2$ (shown below), and BM(PEG)$_3$ (shown below); bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). In some embodiments, bis-maleimide reagents allow the attachment of the thiol group of a cysteine in the antibody to a thiol-containing drug moiety, linker, or linker-drug intermediate. Other functional groups that are reactive with thiol groups include, iodoacetamide, bromoacetamide, vinyl pyridine, disulfide, pyridyl disulfide, isocyanate, and isothiocyanate.

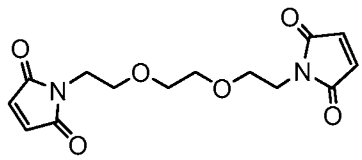

BM(PEG)$_2$

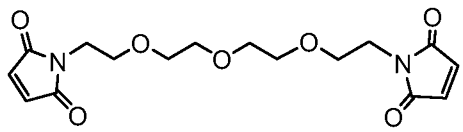

BM(PEG)$_3$

Certain useful linker reagents can be obtained from various commercial sources, such as Pierce Biotechnology, Inc. (Rockford, IL), Molecular Biosciences Inc.(Boulder, CO), or synthesized in accordance with procedures described in the art; for example, in Toki et al (2002) J. Org. Chem. 67:1866-1872; Dubowchik, et al. (1997) Tetrahedron Letters, 38:5257-60; Walker, M.A. (1995) J. Org. Chem. 60:5352-5355; Frisch et al (1996) Bioconjugate Chem. 7:180-186; US 6214345; WO 02/088172; US 2003130189; US2003096743; WO 03/026577; WO 03/043583; and WO 04/032828.

Carbon-14-labeled 1-isothiocyanatobenzyl-3-methyldiethylene triaminepentaacetic acid (MX-DTPA) is an exemplary chelating agent for conjugation of radionucleotide to the antibody. See, e.g., WO94/11026.

**b) Exemplary Drug Moieties**

An ADC may comprise an anthracycline. Anthracyclines are antibiotic compounds that exhibit cytotoxic activity. While not intending to be bound by any particular theory, studies have indicated that anthracyclines may operate to kill cells by a number of different mechanisms, including: 1) intercalation of the drug molecules into the DNA of the cell thereby inhibiting DNA-dependent nucleic acid synthesis; 2) production by the drug of free radicals which then react with cellular macromolecules to cause damage to the cells, and/or 3) interactions of the drug molecules with the cell membrane (see, e.g., C. Peterson et al., "Transport And Storage Of Anthracycline In Experimental Systems And Human Leukemia" in Anthracycline Antibiotics In Cancer Therapy; N.R. Bachur, "Free Radical Damage" id. at pp.97-102). Because of their cytotoxic potential anthracyclines have been used in the treatment of numerous cancers such as leukemia, breast carcinoma, lung carcinoma, ovarian adenocarcinoma and sarcomas (see e.g., P.H- Wiernik, in Anthracycline: Current Status And New Developments p 11).

Exemplary anthracyclines include doxorubicin, epirubicin, idarubicin, daunomycin, nemorubicin, and derivatives thereof. Immunoconjugates and prodrugs of daunorubicin and doxorubicin have been prepared and studied (Kratz et al (2006) Current Med. Chem. 13:477-523; Jeffrey et al (2006) Bioorganic & Med. Chem. Letters 16:358-362; Torgov et al (2005) Bioconj. Chem. 16:717-721; Nagy et al (2000) Proc. Natl. Acad. Sci. USA 97:829-834; Dubowchik et al (2002) Bioorg. & Med. Chem. Letters 12:1529-1532; King et al (2002) J. Med. Chem. 45:4336-4343; EP 0328147; US 6630579). The antibody-drug conjugate BR96-doxorubicin reacts specifically with the tumor-associated antigen Lewis-Y and has been evaluated in phase I and II studies (Saleh et al (2000) J. Clin. Oncology 18:2282-2292; Ajani et al (2000) Cancer Jour. 6:78-81; Tolcher et al (1999) J. Clin. Oncology 17:478-484).

PNU-159682 is a potent metabolite (or derivative) of nemorubicin (Quintieri, et al. (2005) Clinical Cancer Research 11(4):1608-1617). Nemorubicin is a semisynthetic analog of doxorubicin with a 2-methoxymorpholino group on the glycoside amino of doxorubicin and has been under clinical evaluation (Grandi et al (1990) Cancer Treat. Rev. 17:133; Ripamonti et al (1992) Brit. J. Cancer 65:703;), including phase II/III trials for hepatocellular carcinoma (Sun et al (2003) Proceedings of the American Society for Clinical Oncology 22, Abs 1448; Quintieri (2003) Proceedings of the American Association of Cancer Research, 44:1st Ed, Abs 4649; Pacciarini et al (2006) Four. Clin. Oncology 24:14116).

An exemplary ADC comprising nemorubicin or nemorubicin derivatives is shown in Formula Ia:

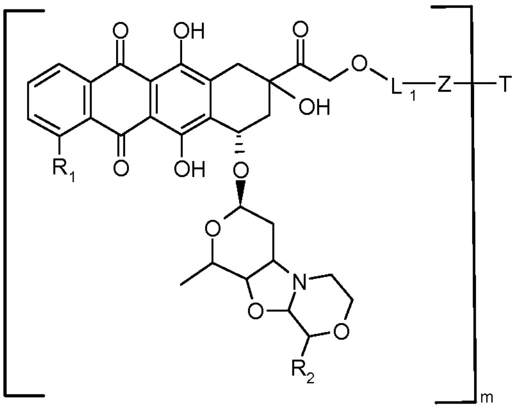

(Ia)

wherein $R_1$ is hydrogen atom, hydroxy or methoxy group and $R_2$ is a $C_1$-$C_5$ alkoxy group; or a pharmaceutically acceptable salt thereof;
$L_1$ and Z together are a linker (L) as described herein;
T is an antibody (Ab) as described herein; and
m is 1 to about 20. In some embodiments, m is 1 to 10, 1 to 7, 1 to 5, or 1 to 4.

In some embodiments, $R_1$ and $R_2$ are both methoxy (-OMe).
A further exemplary ADC comprising nemorubicin or nemorubicin derivatives is shown in Formula Ib:

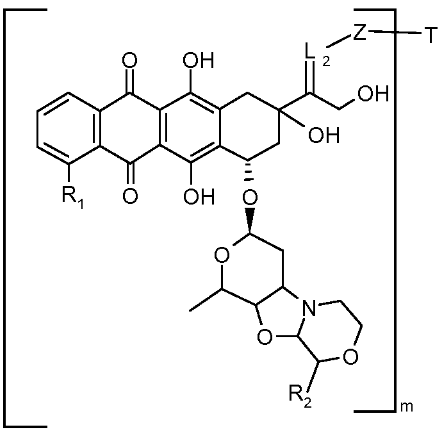

(Ib)

wherein $R_1$ is hydrogen atom, hydroxy or methoxy group and $R_2$ is a $C_1$-$C_5$ alkoxy group; or a pharmaceutically acceptable salt thereof;
$L_2$ and Z together are a linker (L) as described herein;
T is an antibody (Ab) as described herein; and
m is 1 to about 20. In some embodiments, m is 1 to 10, 1 to 7, 1 to 5, or 1 to 4.

In some embodiments, $R_1$ and $R_2$ are both methoxy (-OMe).
In some embodiments, the nemorubicin component of a nemorubicin-containing ADC is PNU-159682. In some such embodiments, the drug portion of the ADC may have one of the following structures:

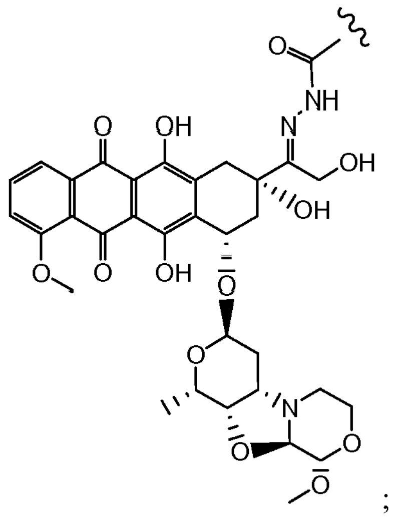

or

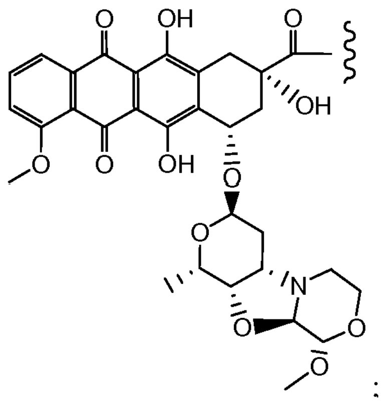

wherein the wavy line indicates the attachment to the linker (L).

Anthracyclines, including PNU-159682, may be conjugated to antibodies through several linkage sites and a variety of linkers (US 2011/0076287; WO2009/099741; US 2010/0034837; WO 2010/009124), including the linkers described herein.

Exemplary ADCs comprising a nemorubicin and linker include,

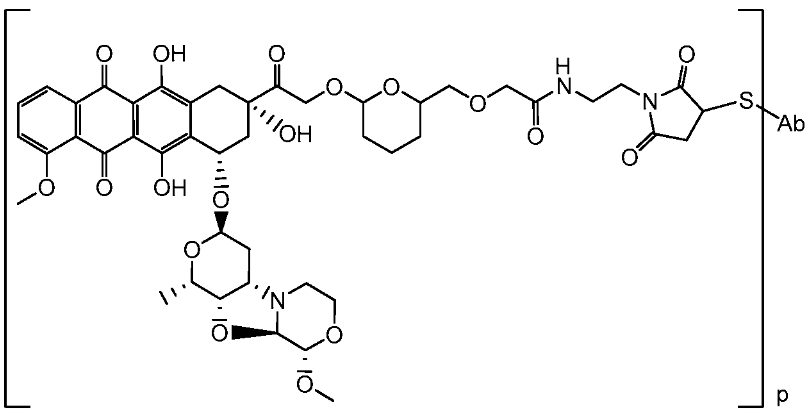

PNU-159682 maleimide acetal-Ab;

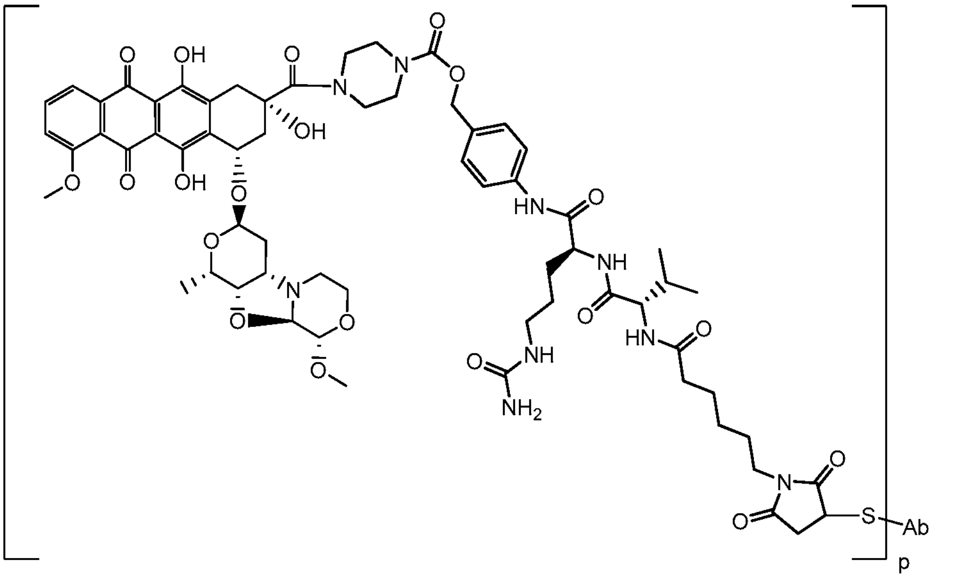

PNU-159682-val-cit-PAB-Ab;

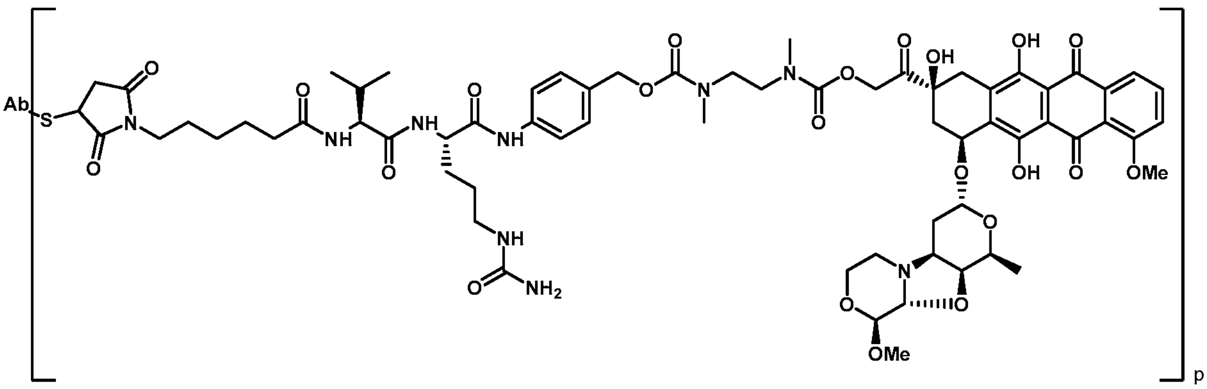

PNU-159682-val-cit-PAB-spacer-Ab;

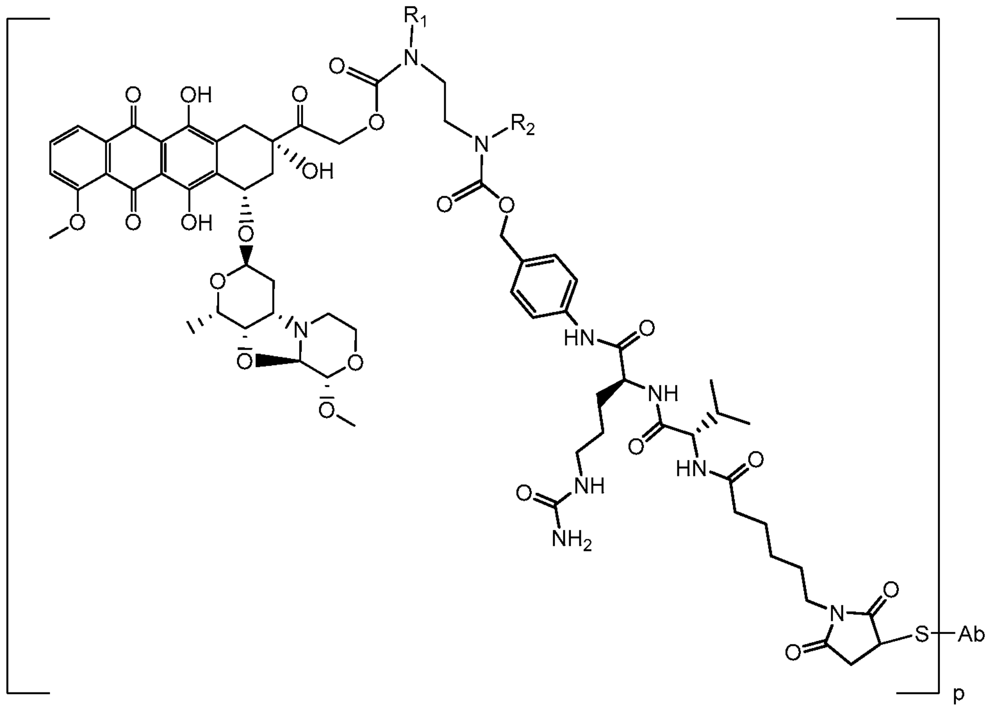

PNU-159682-val-cit-PAB-spacer($R^1R^2$)-Ab, wherein:

$R_1$ and $R_2$ are independently selected from H and $C_1$-$C_6$ alkyl; and

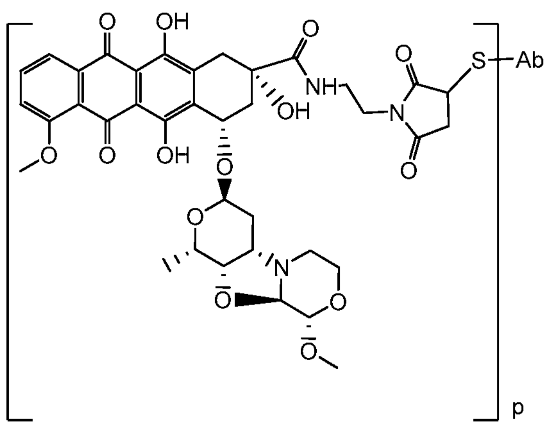

PNU-159682-maleimide-Ab.

The linker of PNU-159682 maleimide acetal-Ab is acid-labile, while the linkers of PNU-159682-val-cit-PAB-Ab, PNU-159682-val-cit-PAB-spacer-Ab, and PNU-159682-val-cit-PAB-spacer($R^1R^2$)-Ab are protease cleavable.

**c) Drug Loading**

Drug loading is represented by p, the average number of drug moieties per antibody in a molecule of Formula I. Drug loading may range from 1 to 20 drug moieties (D) per antibody. ADCs of Formula I include collections of antibodies conjugated with a range of drug moieties, from 1 to 20. The average number of drug moieties per antibody in preparations of ADC from conjugation reactions may be characterized by conventional means such as mass spectroscopy, ELISA assay, and HPLC. The quantitative distribution of ADC in terms of p may also be determined. In some instances, separation, purification, and characterization of homogeneous ADC where p is a certain value from ADC with other drug loadings may be achieved by means such as reverse phase HPLC or electrophoresis.

For some antibody-drug conjugates, p may be limited by the number of attachment sites on the antibody. For

example, where the attachment is a cysteine thiol, as in certain exemplary embodiments above, an antibody may have only one or several cysteine thiol groups, or may have only one or several sufficiently reactive thiol groups through which a linker may be attached. In certain embodiments, higher drug loading, e.g. p >5, may cause aggregation, insolubility, toxicity, or loss of cellular permeability of certain antibody-drug conjugates. In certain embodiments, the average drug loading for an ADC ranges from 1 to about 8; from about 2 to about 6; or from about 3 to about 5. Indeed, it has been shown that for certain ADCs, the optimal ratio of drug moieties per antibody may be less than 8, and may be about 2 to about 5 (US 7498298).

In certain embodiments, fewer than the theoretical maximum of drug moieties are conjugated to an antibody during a conjugation reaction. An antibody may contain, for example, lysine residues that do not react with the drug-linker intermediate or linker reagent, as discussed below. Generally, antibodies do not contain many free and reactive cysteine thiol groups which may be linked to a drug moiety; indeed most cysteine thiol residues in antibodies exist as disulfide bridges. In certain embodiments, an antibody may be reduced with a reducing agent such as dithiothreitol (DTT) or tricarbonylethylphosphine (TCEP), under partial or total reducing conditions, to generate reactive cysteine thiol groups. In certain embodiments, an antibody is subjected to denaturing conditions to reveal reactive nucleophilic groups such as lysine or cysteine.

The loading (drug/antibody ratio) of an ADC may be controlled in different ways, and for example, by: (i) limiting the molar excess of drug-linker intermediate or linker reagent relative to antibody, (ii) limiting the conjugation reaction time or temperature, and (iii) partial or limiting reductive conditions for cysteine thiol modification.

It is to be understood that where more than one nucleophilic group reacts with a drug-linker intermediate or linker reagent, then the resulting product is a mixture of ADC compounds with a distribution of one or more drug moieties attached to an antibody. The average number of drugs per antibody may be calculated from the mixture by a dual ELISA antibody assay, which is specific for antibody and specific for the drug. Individual ADC molecules may be identified in the mixture by mass spectroscopy and separated by HPLC, e.g. hydrophobic interaction chromatography (*see, e.g.,* McDonagh et al (2006) Prot. Engr. Design & Selection 19(7):299-307; Hamblett et al (2004) Clin. Cancer Res. 10:7063-7070; Hamblett, K.J., et al. "Effect of drug loading on the pharmacology, pharmacokinetics, and toxicity of an anti-CD30 antibody-drug conjugate," Abstract No. 624, American Association for Cancer Research, 2004 Annual Meeting, March 27-31, 2004, Proceedings of the AACR, Volume 45, March 2004; Alley, S.C., et al. "Controlling the location of drug attachment in antibody-drug conjugates," Abstract No. 627, American Association for Cancer Research, 2004 Annual Meeting, March 27-31, 2004, Proceedings of the AACR, Volume 45, March 2004). In certain embodiments, a homogeneous ADC with a single loading value may be isolated from the conjugation mixture by electrophoresis or chromatography.

### d) Certain Methods of Preparing Immunoconjugates

An ADC of Formula I may be prepared by several routes employing organic chemistry reactions, conditions, and reagents known to those skilled in the art, including: (1) reaction of a nucleophilic group of an antibody with a bivalent linker reagent to form Ab-L via a covalent bond, followed by reaction with a drug moiety D; and (2) reaction of a nucleophilic group of a drug moiety with a bivalent linker reagent, to form D-L, via a covalent bond, followed by reaction with a nucleophilic group of an antibody. Exemplary methods for preparing an ADC of Formula I via the latter route are described in US 7498298.

Nucleophilic groups on antibodies include: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; and (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol) or tricarbonylethylphosphine (TCEP), such that the antibody is fully or partially reduced. Each cysteine bridge will thus form, theoretically, two reactive thiol nucleophiles. Additional nucleophilic groups can be introduced into antibodies through modification of lysine residues, e.g., by reacting lysine residues with 2-iminothiolane (Traut's reagent), resulting in conversion of an amine into a thiol. Reactive thiol groups may also be introduced into an antibody by introducing one, two, three, four, or more cysteine residues (e.g., by preparing variant antibodies comprising one or more non-native cysteine amino acid residues).

Antibody-drug conjugates of the invention may also be produced by reaction between an electrophilic group on an antibody, such as an aldehyde or ketone carbonyl group, with a nucleophilic group on a linker reagent or drug. Useful nucleophilic groups on a linker reagent include, but are not limited to, hydrazide, oxime, amino, hydrazine, thiosemicarbazone, hydrazine carboxylate, and arylhydrazide. In one embodiment, an antibody is modified to introduce electrophilic moieties that are capable of reacting with nucleophilic substituents on the linker reagent or drug. In another

embodiment, the sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either galactose oxidase or sodium meta-periodate may yield carbonyl (aldehyde and ketone) groups in the antibody that can react with appropriate groups on the drug (Hermanson, Bioconjugate Techniques). In another embodiment, antibodies containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such an aldehyde can be reacted with a drug moiety or linker nucleophile.

Exemplary nucleophilic groups on a drug moiety include, amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Exemplary cross-linker reagents that may be used to prepare ADC are described herein in the section titled "Exemplary Linkers." Methods of using such crosslinker reagents to link two moieties, including a proteinaceous moiety and a chemical moiety, are known in the art. In some embodiments, a fusion protein comprising an antibody and a cytotoxic agent may be made, e.g., by recombinant techniques or peptide synthesis. A recombinant DNA molecule may comprise regions encoding the antibody and cytotoxic portions of the conjugate either adjacent to one another or separated by a region encoding a linker peptide which does not destroy the desired properties of the conjugate.

In yet another embodiment, an antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a drug or radionucleotide).

### E. Methods and Compositions for Diagnostics and Detection

In certain embodiments, any of the anti-CD79b antibodies provided herein is useful for detecting the presence of CD79b in a biological sample. The term "detecting" as used herein encompasses quantitative or qualitative detection. A "biological sample" comprises, e.g., a cell or tissue (e.g., biopsy material, including cancerous or potentially cancerous lymph tissue, including tissue from subjects having or suspected of having a B cell disorder and/or a B cell proliferative disorder, including, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

In one embodiment, an anti-CD79b antibody for use in a method of diagnosis or detection is provided. In a further aspect, a method of detecting the presence of CD79b in a biological sample is provided. In certain embodiments, the method comprises contacting the biological sample with an anti-CD79b antibody as described herein under conditions permissive for binding of the anti-CD79b antibody to CD79b, and detecting whether a complex is formed between the anti-CD79b antibody and CD79b in the biological sample. Such method may be an *in vitro* or *in vivo* method. In one embodiment, an anti-CD79b antibody is used to select subjects eligible for therapy with an anti-CD79b antibody, e.g. where CD79b is a biomarker for selection of patients. In a further embodiment, the biological sample is a cell or tissue (e.g., cancerous or potentially cancerous lymph tissue, including tissue of subjects having or suspected of having a B cell disorder and/or a B cell proliferative disorder, including, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

In a further embodiment, an anti-CD79b antibody is used in vivo to detect, e.g., by in vivo imaging, a CD79b-positive cancer in a subject, e.g., for the purposes of diagnosing, prognosing, or staging cancer, determining the appropriate course of therapy, or monitoring response of a cancer to therapy. One method known in the art for in vivo detection is immuno-positron emission tomography (immuno-PET), as described, e.g., in van Dongen et al., The Oncologist 12:1379-1389 (2007) and Verel et al., J. Nucl. Med. 44:1271-1281 (2003). In such embodiments, a method is provided for detecting a CD79b-positive cancer in a subject, the method comprising administering a labeled anti-CD79b antibody to a subject having or suspected of having a CD79b-positive cancer, and detecting the labeled anti-CD79b antibody in the subject, wherein detection of the labeled anti-CD79b antibody indicates a CD79b-positive cancer in the subject. In certain of such embodiments, the labeled anti-CD79b antibody comprises an anti-CD79b antibody conjugated to a positron emitter, such as $^{68}Ga$, $^{18}F$, $^{64}Cu$, $^{86}Y$, $^{76}Br$, $^{89}Zr$, and $^{124}I$. In a particular embodiment, the positron emitter is $^{89}Zr$.

A method of diagnosis or detection may comprise contacting a first anti-CD79b antibody immobilized to a substrate with a biological sample to be tested for the presence of CD79b, exposing the substrate to a second anti-CD79b antibody, and detecting whether the second anti-CD79b is bound to a complex between the first anti-CD79b antibody and CD79b in the biological sample. A substrate may be any supportive medium, e.g., glass, metal, ceramic, polymeric beads, slides, chips, and other substrates. In certain embodiments, a biological sample comprises a cell or tissue (e.g., biopsy material, including cancerous or potentially cancerous lymph tissue, including tissue from subjects having or suspected of having a B cell disorder and/or a B cell proliferative disorder, including, but not limited to, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma). In certain embodiments, the first or second anti-CD79b antibody is any of the antibodies described herein.

Exemplary disorders that may be diagnosed or detected according to any of the above embodiments include CD79b-positive cancers, such as CD79b-positive lymphoma, CD79b-positive non-Hogkins lymphoma (NHL; including, CD79b-positive aggressive NHL, CD79b-positive relapsed aggressive NHL, CD79b-positive relapsed indolent NHL, CD79b-positive refractory NHL, and CD79b-positive refractory indolent NHL), CD79b-positive chronic lymphocytic leukemia (CLL), CD79b-positive small lymphocytic lymphoma, CD79b-positive leukemia, CD79b-positive hairy cell leukemia (HCL), CD79b-positive acute lymphocytic leukemia (ALL), CD79b-positive Burkitt's lymphoma, and CD79b-positive mantle cell lymphoma. In some embodiments, a CD79b-positive cancer is a cancer that receives an anti-CD79b immunohistochemistry (IHC) score greater than "0," which corresponds to very weak or no staining in >90% of tumor cells. In some embodiments, a CD79b-positive cancer expresses CD79b at a 1+, 2+ or 3+ level, wherein 1+ corresponds to weak staining in >50% of neoplastic cells, 2+ corresponds to moderate staining in >50% neoplastic cells, and 3+ corresponds to strong staining in >50% of neoplastic cells. In some embodiments, a CD79b-positive cancer is a cancer that expresses CD79b according to an in situ hybridization (ISH) assay. In some such embodiments, a scoring system similar to that used for IHC is used. In some embodiments, a CD79b-positive cancer is a cancer that expresses CD79b according to a reverse-transcriptase PCR (RT-PCR) assay that detects CD79b mRNA. In some embodiments, the RT-PCR is quantitative RT-PCR.

Labeled anti-CD79b antibodies are provided. Labels include, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, the radioisotopes $^{32}P$, $^{14}C$, $^{125}I$, $^{3}H$, and $^{131}I$, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like. In another embodiment, a label is a positron emitter. Positron emitters include but are not limited to $^{68}Ga$, $^{18}F$, $^{64}Cu$, $^{86}Y$, $^{76}Br$, $^{89}Zr$, and $^{124}I$. In a particular embodiment, a positron emitter is $^{89}Zr$.

### F. Pharmaceutical Formulations

Pharmaceutical formulations of an anti-CD79b antibody or immunoconjugate as described herein are prepared by mixing such antibody or immunoconjugate having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody or immunoconjugate formulations are described in US Patent No. 6,267,958.

Aqueous antibody or immunoconjugate formulations include those described in US Patent No. 6,171,586 and WO2006/044908, the latter formulations including a histidine-acetate buffer.

The formulation herein may also contain more than one active ingredient as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody or immunoconjugate, which matrices are in the form of shaped articles, *e.g*. films, or microcapsules.

The formulations to be used for *in vivo* administration are generally sterile. Sterility may be readily accomplished, e.g., by filtration through sterile filtration membranes.

**G. Therapeutic Methods and Compositions**

Any of the anti-CD79b antibodies or immunoconjugates provided herein may be used in methods, e.g., therapeutic methods.

In one aspect, an anti-CD79b antibody or immunoconjugate provided herein is used in a method of inhibiting proliferation of a CD79b-positive cell, the method comprising exposing the cell to the anti-CD79b antibody or immunoconjugate under conditions permissive for binding of the anti-CD79b antibody or immunoconjugate to CD79b on the surface of the cell, thereby inhibiting the proliferation of the cell. In certain embodiments, the method is an *in vitro* or an *in vivo* method In some embodiments, the cell is a B cell. In some embodiments, the cell is a neoplastic B cell, such as a lymphoma cell or a leukemia cell.

Inhibition of cell proliferation in vitro may be assayed using the CellTiter-Glo™ Luminescent Cell Viability Assay, which is commercially available from Promega (Madison, WI). That assay determines the number of viable cells in culture based on quantitation of ATP present, which is an indication of metabolically active cells. *See* Crouch et al. (1993) J. Immunol. Meth. 160:81-88, US Pat. No. 6602677. The assay may be conducted in 96- or 384-well format, making it amenable to automated high-throughput screening (HTS). *See* Cree et al. (1995) AntiCancer Drugs 6:398-404. The assay procedure involves adding a single reagent (CellTiter-Glo® Reagent) directly to cultured cells. This results in cell lysis and generation of a luminescent signal produced by a luciferase reaction. The luminescent signal is proportional to the amount of ATP present, which is directly proportional to the number of viable cells present in culture. Data can be recorded by luminometer or CCD camera imaging device. The luminescence output is expressed as relative light units (RLU).

In another aspect, an anti-CD79b antibody or immunoconjugate for use as a medicament is provided. In further aspects, an anti-CD79b antibody or immunoconjugate for use in a method of treatment is provided. In certain embodiments, an anti-CD79b antibody or immunoconjugate for use in treating CD79b-positive cancer is provided. In certain embodiments, the invention provides an anti-CD79b antibody or immunoconjugate for use in a method of treating an individual having a CD79b-positive cancer, the method comprising administering to the individual an effective amount of the anti-CD79b antibody or immunoconjugate. In one such embodiment, the method further comprises administering to the individual an effective amount of at least one additional therapeutic agent, e.g., as described below.

In a further aspect, the invention provides for the use of an anti-CD79b antibody or immunoconjugate in the manufacture or preparation of a medicament. In one embodiment, the medicament is for treatment of CD79b-positive cancer. In a further embodiment, the medicament is for use in a method of treating CD79b-positive cancer, the method comprising administering to an individual having CD79b-positive cancer an effective amount of the medicament. In one such embodiment, the method further comprises administering to the individual an effective amount of at least one additional therapeutic agent, e.g., as described below.

In a further aspect, the invention provides a method for treating CD79b-positive cancer. In one embodiment, the method comprises administering to an individual having such CD79b-positive cancer an effective amount of an anti-CD79b antibody or immunoconjugate. In one such embodiment, the method further comprises administering to the individual an effective amount of at least one additional therapeutic agent, as described below.

A CD79b-positive cancer according to any of the above embodiments may be, e.g., lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma). In some embodiments, a CD79b-positive cancer is a cancer that receives an anti-CD79b immunohistochemistry (IHC) or in situ hybridization (ISH) score greater than "0," which corresponds to very weak or no staining in >90% of tumor cells. In another embodiment, a CD79b-positive cancer expresses CD79b at a 1+, 2+ or 3+ level, wherein 1+ corresponds to weak staining in >50% of neoplastic cells, 2+ corresponds to moderate staining in >50% neoplastic cells, and 3+ corresponds to strong staining in >50% of neoplastic cells. In some embodiments, a CD79b-positive cancer is a cancer that expresses CD79b according to a reverse-transcriptase PCR (RT-PCR) assay that detects CD79b mRNA. In some embodiments, the RT-PCR is quantitative RT-PCR.

In some embodiments, immunoconjugates comprising a nemorubicin derivative covalently attached to the anti-CD79b antibody through a protease-cleavable linker are particularly useful for treating diffuse large B-cell lymphomas, mantle cell lymphomas, and Burkitt's lymphoma as evidenced, for example, by the xenograft models shown in Examples B, C, D, E, and G. The immunoconjugate for use in treating diffuse large B-cell lymphomas, mantle cell lymphomas, and/or Burkitt's lymphoma, may, in some embodiments, comprise PNU-159682. In some such embodiments, the immunoconjugate comprises PNU-159682 and a linker comprising val-cit, such as the immunoconjugate shown in Figure 5B.

In some embodiments, methods of treating an individual having an CD79b-positive cancer are provided, wherein the CD79b-positive cancer is resistant to a first therapeutic. In some embodiments, the CD79b-positive cancer that is resistant to a first therapeutic expresses P-glycoprotein (P-gp). In some embodiments, the method comprises administering to the individual an effective amount of an immunoconjugate comprising an antibody that binds to CD79b. In some embodiments, the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, the first therapeutic comprises a first antibody that binds an antigen other than CD79b. In some embodiments, the first therapeutic is a first immunoconjugate comprising a first antibody that binds an antigen other than CD79b and a first cytotoxic agent. In some embodiments, the first antibody binds CD22. In some embodiments, the first cytotoxic agent and the cytotoxic agent of the immunoconjugate comprising an antibody that binds to CD79b are different. In some such embodiments, the first cytotoxic agent is selected from MMAE, a calicheamicin, a maytansinoid, and a pyrrolobenzodiazepine. In some such embodiments, the first cytotoxic agent is MMAE and the cytotoxic agent of the immunoconjugate comprising an antibody that binds to CD79b is a nemorubicin derivative. In some such embodiments, the first cytotoxic agent is a pyrrolobenzodiazepine and the cytotoxic agent of the immunoconjugate comprising an antibody that binds to CD79b is a nemorubicin derivative. In some such embodiments, the first cytotoxic agent is a maytansinoid and the cytotoxic agent of the immunoconjugate comprising an antibody that binds to CD79b is a nemorubicin derivative.

In some embodiments, the first antibody binds CD79b. In some such embodiments, the first cytotoxic agent is selected from MMAE, a calicheamicin, and a pyrrolobenzodiazepine and the cytotoxic agent of the immunoconjugate described herein is a nemorubicin derivative. In some embodiments, the first cytotoxic agent is MMAE and the cytotoxic agent of the immunoconjugate described herein is a nemorubicin derivative. In some embodiments, the first cytotoxic agent is a pyrrolobenzodiazepine and the cytotoxic agent of the immunoconjugate described herein is a nemorubicin derivative. In some embodiments, the first cytotoxic agent is a maytansinoid and the cytotoxic agent of the immunoconjugate described herein is a nemorubicin derivative.

In some embodiments, a method of treating an individual having a CD79b-positive / P-gp positive cancer is provided. In some embodiments, the method comprises administering to the individual an effective amount of an immunoconjugate described herein. In some embodiments, the CD79b-positive / P-gp positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, a cancer is considered to be P-gp positive when the cancer expresses higher levels of P-gp mRNA and/or protein than control cells or tissue. The control cells or tissue may, in various embodiments, be non-cancerous cells or tissue from the same patient; non-cancerous or cancerous cells or tissue from a different patient or healthy individual, or from a pool of patients or healthy individuals; non-cancerous or cancerous cells or tissue from the same patient taken at an earlier point in time, such as, for example, prior to an initial treatment regiment; or from healthy individuals; etc.

In some embodiments, methods of treating an individual with cancer are provided, wherein the cancer is resistant to a first therapeutic. In some embodiments, the first therapeutic is a first immunoconjugate comprising a first antibody linked to a first cytotoxic agent through a first linker. In some embodiments, a method of treating an individual with a cancer that is resistant to a first therapeutic (such as a first immunoconjugate) comprises administering a second immunoconjugate comprising a second antibody linked to a second cytotoxic agent through a second linker. In some embodiments, the first antibody and the second antibody bind to different antigens and the first cytotoxic agent and the second cytotoxic agents are the same or different. In some embodiments, the first antibody and the second antibody bind to different antigens that are present on at least some of the same cells. In some embodiments, the first antibody and the second antibody bind to different antigens and the first cytotoxic agent and the second cytotoxic agents are different. In some embodiments, the first antibody and the second antibody bind to the same antigens, and the first

cytotoxic agent and the second cytotoxic agent are different. In any of the foregoing embodiments, the first linker and the second linker may be the same or different. In some embodiments, the first antibody and the second antibody bind to different antigens, the first and second linkers are different, and the first and second cytotoxic agents are different.

An "individual" according to any of the above embodiments may be a human.

In a further aspect, the invention provides pharmaceutical formulations comprising any of the anti-CD79b antibodies or immunoconjugate provided herein, e.g., for use in any of the above therapeutic methods. In one embodiment, a pharmaceutical formulation comprises any of the anti-CD79b antibodies or immunoconjugates provided herein and a pharmaceutically acceptable carrier. In another embodiment, a pharmaceutical formulation comprises any of the anti-CD79b antibodies or immunoconjugates provided herein and at least one additional therapeutic agent, e.g., as described below.

Antibodies or immunoconjugates of the invention can be used either alone or in combination with other agents in a therapy. For instance, an antibody or immunoconjugate of the invention may be co-administered with at least one additional therapeutic agent.

In some embodiments, an anti-CD79b immunoconjugate is administered in combination with an anti-CD22 antibody or immunoconjugate. An exemplary anti-CD22 antibody or immunoconjugate comprises the hypervariable regions of 10F4v3, such that the anti-CD22 antibody or immunoconjugate comprises (i) HVR H1 having the sequence of SEQ ID NO: 42, (ii) HVR H2 having the sequence of SEQ ID NO: 43, (iii) HVR H3 having the sequence of SEQ ID NO: 44, (iv) HVR L1 having the sequence of SEQ ID NO: 45, (v) HVR L2 having the sequence of SEQ ID NO: 46, and (vi) HVR L3 having the sequence of SEQ ID NO: 47. In some embodiments, an anti-CD22 antibody or immunoconjugate comprises the heavy chain variable region and light chain variable region of 10F4v3. In some such embodiments, the anti-CD22 antibody or immunoconjugate comprises a heavy chain variable region having the sequence of SEQ ID NO: 48 and a light chain variable region having the sequence of SEQ ID NO: 49. An anti-CD22 immunoconjugate comprises, in some embodiments, a cytotoxic agent selected from an auristatin, a nemorubicin derivative, and a pyrrolobenzodiazepine. In some embodiments, an anti-CD22 immunoconjugate comprises a cytotoxic agent selected from MMAE, PNU-159682, and a PBD dimer having the structure:

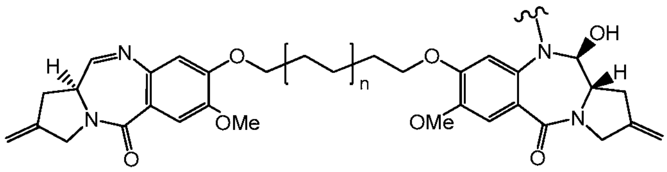
;

wherein the wavy line indicates the attachment to a linker, which is attached to the anti-CD79b antibody, and wherein n is 0 or 1. Exemplary linkers that may be used in the anti-CD79b immunoconjugates include those described herein. In some embodiments, an anti-CD22 immunoconjugate is selected from a Thio Hu anti-CD22 10F4v3 HC A118C-MC-val-cit-PAB-MMAE, a Thio Hu anti-CD22 10F4v3 HC S400C-MC-val-cit-PAB-MMAE, and a Thio Hu anti-CD22 10F4v3 LC V205C-MC-val-cit-PAB-MMAE immunoconjugate, which are described, e.g., in US 2008/0050310; a Thio Hu anti-CD22 10F4v3 HC A118C-MC-val-cit-PAB-PNU-159682, a Thio Hu anti-CD22 10F4v3 HC A118C-MC-acetal-PNU-159682, a Thio Hu anti-CD22 10F4v3 HC A118C-MC-val-cit-PAB-PBD, a Thio Hu anti-CD22 10F4v3 HC S400C-MC-val-cit-PAB-PNU-159682, a Thio Hu anti-CD22 10F4v3 HC S400C-MC-acetal-PNU-159682, a Thio Hu anti-CD22 10F4v3 HC S400C-MC-val-cit-PAB-PBD, a Thio Hu anti-CD22 10F4v3 LC V205C-MC-val-cit-PAB-PNU-159682, a Thio Hu anti-CD22 10F4v3 LC V205C-MC-acetal-PNU-159682, and a Thio Hu anti-CD22 10F4v3 LC V205C-MC-val-cit-PAB-PBD. The heavy chain and light chain sequences for Thio Hu anti-CD22 10F4v3 HC A118C are shown in SEQ ID NOs: 50 and 51, respectively. The heavy chain and light chain sequences for Thio Hu anti-CD22 10F4v3 HC S400C are shown in SEQ ID NOs: 52 and 51, respectively. The heavy chain and light chain sequences for Thio Hu anti-CD22 10F4v3 LC V205C are shown in SEQ ID NOs: 56 and 53, respectively. Apart from the specific antibody sequence, the structures of the anti-CD22 immunoconjugates are analogous to the structures of the anti-CD79b immunoconjugates described herein.

In some embodiments, an anti-CD22 immunoconjugate is administered in combination with an anti-CD20 antibody (either a naked antibody or an ADC). In some embodiments, the anti-CD20 antibody is rituximab (Rituxan®) or 2H7 (Genentech, Inc., South San Francisco, CA). In some embodiments, an anti-CD22 immunoconjugate is administered in combination with an anti-VEGF antibody (e.g, bevicizumab, trade name Avastin®).

Other therapeutic regimens may be combined with the administration of an anti-CD22 immunoconjugate, including, radiation therapy and/or bone marrow and peripheral blood transplants, and/or a cytotoxic agent. In some embodiments, a cytotoxic agent is an agent or a combination of agents such as, for example, cyclophosphamide, hydroxydaunorubicin, adriamycin, doxorubincin, vincristine (Oncovin™), prednisolone, CHOP (combination of cyclophosphamide, doxorubicin, vincristine, and prednisolone), CVP (combination of cyclophosphamide, vincristine, and

prednisolone), or immunotherapeutics such as anti-CD20 (e.g., rituximab, trade name Rituxan®), anti-VEGF (e.g., bevicizumab, trade name Avastin®), taxanes (such as paclitaxel and docetaxel) and anthracycline antibiotics.

Such combination therapies noted above encompass combined administration (where two or more therapeutic agents are included in the same or separate formulations), and separate administration, in which case, administration of the antibody or immunoconjugate of the invention can occur prior to, simultaneously, and/or following, administration of the additional therapeutic agent and/or adjuvant. Antibodies or immunoconjugates of the invention can also be used in combination with radiation therapy.

An antibody or immunoconjugate of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies or immunoconjugates of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody or immunoconjugate need not be, but is optionally formulated with one or more agents currently used to prevent or treat the disorder in question. The effective amount of such other agents depends on the amount of antibody or immunoconjugate present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody or immunoconjugate of the invention (when used alone or in combination with one or more other additional therapeutic agents) will depend on the type of disease to be treated, the type of antibody or immunoconjugate, the severity and course of the disease, whether the antibody or immunoconjugate is administered for preventive or therapeutic purposes, previous therapy, the patient's clinical history and response to the antibody or immunoconjugate, and the discretion of the attending physician. The antibody or immunoconjugate is suitably administered to the patient at one time or over a series of treatments. Depending on the type and severity of the disease, about 1 μg/kg to 15 mg/kg (e.g. 0.1mg/kg-10mg/kg) of antibody or immunoconjugate can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody or immunoconjugate would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

In some embodiments, a lower dose of a huMA79b (such as huMA79bv28) ADC comprising a nemorubicin derivative, such as PNU-159682, may be used to achieve the same efficacy as a higher dose of a huMA79b ADC comprising an MMAE moiety.

It is understood that any of the above formulations or therapeutic methods may be carried out using both an immunoconjugate of the invention and an anti-CD79b antibody.

### H. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the disorder and may have a sterile access port (for example the container may be an intravenous solution bag or a vial having a stopper pierceable by a hypodermic injection needle). At least one active agent in the composition is an antibody or immunoconjugate of the invention. The label or package insert indicates that the composition is used for treating the condition of choice. Moreover, the article of manufacture may comprise (a) a

first container with a composition contained therein, wherein the composition comprises an antibody or immunoconjugate of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

## III. EXAMPLES

The following are examples of methods and compositions of the invention.

### A. Production of Anti-CD79b Antibody Drug Conjugates

Anti-CD79b antibody MA79b and certain variants, including humanized huMA79b graft and humanized variants huMA79bv17, huMA79bv18, huMA79bv28, and huMA79bv32, are described, e.g., in US 8,088,378 B2. Table 2 shows the SEQ ID NOs corresponding to the heavy chain, light chain, and hypervariable regions (HVRs) for each antibody.

Table 2: Sequences corresponding to MA79b and humanized variants

| Antibody | HC variable region SEQ ID NO: | LC variable region SEQ ID NO: | HVR H1 SEQ ID NO: | HVR H2 SEQ ID NO: | HVR H3 SEQ ID NO: | HVR L1 SEQ ID NO: | HVR L2 SEQ ID NO: | HVR L3 SEQ ID NO: |
|---|---|---|---|---|---|---|---|---|
| MA79b | 3 | 4 | 15 | 16 | 17 | 18 | 19 | 20 |
| huMA79b graft | 5 | 6 | 15 | 16 | 17 | 18 | 19 | 20 |
| huMA79bv17 | 7 | 8 | 15 | 16 | 17 | 18 | 19 | 20 |
| huMA79bv18 | 9 | 10 | 15 | 16 | 23 | 18 | 19 | 20 |
| huMA79bv28 | 11 | 12 | 21 (identical to 15) | 22 (identical to 16) | 23 | 24 | 25 (identical to 19) | 26 (identical to 20) |
| huMA79bv32 | 13 | 14 | 15 | 16 | 23 | 35 | 19 | 20 |

The heavy chain framework regions for antibodies huMA79bv17, huMA79bv18, huMA79bv28, and huMA79bv32, HC FR1 to FR4, are shown in SEQ ID NOs: 27 to 30, respectively. The light chain framework regions for antibodies huMA79bv17, huMA79bv18, and huMA79bv28, LC FR1 to FR4, are shown in SEQ ID NOs: 31 to 34, respectively. The light chain framework regions for antibody huMA79bv32, LC FR1 to FR4, are shown in SEQ ID NOs: 31, 36, 33, and 34, respectively. The binding affinity of huMA79b was found to be about 0.4 nM using Scatchard analysis. *See, e.g.,* US 8,088,378 B2.

For larger scale antibody production, antibodies were produced in CHO cells. Vectors coding for VL and VH were transfected into CHO cells and IgG was purified from cell culture media by protein A affinity chromatography.

Anti-CD79b antibody-drug conjugates (ADCs) were produced by conjugating Thio huMA79bv28 HC A118C antibodies to certain drug moieties. Thio huMA79bv28 HC A118C is a huMA79bv28 antibody with an A118C mutation in the heavy chain that adds a conjugatable thiol group. *See, e.g.,* US 8,088,378 B2. The amino acid sequence of the heavy chain of Thio huMA79bv28 HC A118C is shown in SEQ ID NO: 39 (*see* Figure 4), and the amino acid sequence of the light chain of Thio huMA79bv28 HC A118C is shown in SEQ ID NO: 37 (*see* Figure 3). The immunoconjugates were prepared as follows.

<u>Thio huMA79bv28 HC A118C-MC-acetal-PNU-159682</u> ("huMA79bv28-PNU-2")

Prior to conjugation, the antibody was reduced with dithiothreitol (DTT) to remove blocking groups (e.g. cysteine) from the engineered cysteines of the thio-antibody. This process also reduces the interchain disulfide bonds of the

antibody. The reduced antibody was purified to remove the released blocking groups and the interchain disulfides were reoxidized using dehydro-ascorbic acid (dhAA). The intact antibody was then combined with the drug-linker moiety MC-acetal-PNU-159682 to allow conjugation of the drug-linker moiety to the engineered cysteine residues of the antibody. The conjugation reaction was quenched by adding excess N-acetyl-cysteine to react with any free linker-drug moiety, and the ADC was purified. The drug load (average number of drug moieties per antibody) for the ADC was about 1.86, as indicated in the Examples below. huMA79bv28-PNU-2 has the structure shown in Figure 5A (p = drug load).

Thio huMA79bv28 HC A118C-MC-val-cit-PAB-PNU-159682 ("huMA79bv28-PNU-1")

Prior to conjugation, the antibody was reduced with dithiothreitol (DTT) to remove blocking groups (e.g. cysteine) from the engineered cysteines of the thio-antibody. This process also reduces the interchain disulfide bonds of the antibody. The reduced antibody was purified to remove the released blocking groups and the interchain disulfides were reoxidized using dehydro-ascorbic acid (dhAA). The intact antibody was then combined with the drug-linker moiety MC-val-cit-PAB-PNU-159682 ("val-cit" may also be referred to herein as "vc") to allow conjugation of the drug-linker moiety to the engineered cysteine residues of the antibody. The conjugation reaction was quenched by adding excess N-acetyl-cysteine to react with any free linker-drug moiety, and the ADC was purified. The drug load (average number of drug moieties per antibody) for the ADC was in the range of about 1.8 to about 1.9, as indicated in the Example below. huMA79bv28-PNU-1 has the structure shown in Figure 5B (p = drug load).

Thio huMA79bv28 HC A118C-MC-val-cit-PAB-MMAE ("huMA79bv28-MMAE")

Prior to conjugation, the antibody was reduced with dithiothreitol (DTT) to remove blocking groups (e.g. cysteine) from the engineered cysteines of the thio-antibody. This process also reduces the interchain disulfide bonds of the antibody. The reduced antibody was purified to remove the released blocking groups and the interchain disulfides were reoxidized using dehydro-ascorbic acid (dhAA). The intact antibody was then combined with the drug-linker moiety MC-val-cit-PAB-MMAE ("val-cit" may also be referred to herein as "vc") to allow conjugation of the drug-linker moiety to the engineered cysteine residues of the antibody. The conjugation reaction was quenched by adding excess N-acetyl-cysteine to react with any free linker-drug moiety, and the ADC was purified. The drug load (average number of drug moieties per antibody) for the ADC was determined to be about 2, as indicated in the examples below. Thio huMA79bv28 HC A118C-MC-val-cit-PAB-MMAE is described, e.g., in US 8,088,378 B2.

**B. *In vivo* anti-tumor activity of humanized anti-CD79b antibody drug conjugates in a WSU-DLCL2 xenograft model**

To test the efficacy of Thio huMA79bv28 HC A118C conjugates with PNU-159682 ("huMA79bv28-PNU-1" and "huMA79bv28-PNU-2"), the effects of the conjugated antibodies in a mouse xenograft model of WSU-DLCL2 tumors (diffuse large B-cell lymphoma cell line) was examined.

Female CB17 ICR SCID mice (11-12 weeks of age from Charles Rivers Laboratories; Hollister, CA) were each inoculated subcutaneously in the flank with 2 x $10^7$ WSU-DLCL2 cells (DSMZ, German Collection of Microorganisms and Cell Cultures, Braunschweig, Germany). When the xenograft tumors reached an average tumor volume of 150-250 $mm^3$ (referred to as Day 0), the first and only dose of treatment was administered. Tumor volume was calculated based on two dimensions, measured using calipers, and was expressed in $mm^3$ according to the formula: $V = 0.5a \times b^2$, wherein a and b are the long and the short diameters of the tumor, respectively. To analyze the repeated measurement of tumor volumes from the same animals over time, a mixed modeling approach was used (*see, e.g.*, Pinheiro J, et al. nlme: linear and nonlinear mixed effects models. 2009; R package, version 3.1-96). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 2 mg ADC/kg of Thio huMA79bv28 HC A118C immunoconjugate or control antibody-drug conjugates (control ADCs). The control ADCs bind to a protein that is not expressed on the surface of WSU-DLCL2 cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 3 and Figure 6. Table 3 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration

dropped to 0 $mm^3$), the drug dose for each group, the antibody dose for each group, and the drug load for each ADC administered.

Table 3: Anti-CD79b ADC administration to mice with WSU-DLCL2 xenografts

| Antibody administered (Treatment) | TI | PR | CR | Drug Dose (μg//kg) | Ab Dose (mg/kg) | Drug Load (Drug /Ab) |
|---|---|---|---|---|---|---|
| Vehicle* | 8/8 | 0 | 0 | n/a | n/a | n/a |
| huMA79bv28-PNU-1 | 8/8 | 2 | 0 | 15.66 | 2 | 1.83 |
| Control ADC-A118C-MC-val-cit-PAB-PNU-159682 ("Control-PNU-1") | 8/8 | 0 | 0 | 15.83 | 2 | 1.85 |
| huMA79bv28-PNU-2 | 8/8 | 0 | 0 | 15.91 | 2 | 1.86 |
| Control ADC-A118C-MC-acetal-PNU-159682 ("Control-PNU-2") | 8/8 | 0 | 0 | 16.25 | 2 | 1.9 |
| Thio huMA79bv28 HC A118C-MC-vc-PAB-MMAE ("huMA79bv28-MMAE") | 8/8 | 0 | 0 | 19.24 | 2 | 2.01 |
| * Vehicle = 20 mM histidine acetate, pH 5.5, 240 mM sucrose, 0.02% PS20; n/a = not applicable. | | | | | | |

In a 35 day time course with drug conjugates and doses as shown in Table 3, thio huMA79bv28 ADC conjugated through a protease cleavable linker with PNU-159682 ("huMA79bv28-PNU-1") showed inhibition of tumor growth in SCID mice with WSU-DLCL2 tumors compared to the vehicle and the control ADC ("Control-PNU-1"). *See* Figure 6. Thio huMA79bv28 conjugated through an acid-labile linker with PNU-159682 ("huMA79bv28-PNU-2") also showed inhibition of tumor growth in SCID mice with WSU-DLCL2 tumors compared to the vehicle and the control ADC ("Control-PNU-2").

Furthermore, when administered at 2 mg/kg, huMA79bv28-PNU-1 better inhibited tumor growth than huMA79bv28 conjugated with the auristatin drug MMAE ("huMA79bv28-MMAE"). *See* Figure 6.

In this study, the percent body weight change was determined in each dosage group. The results indicated that administration of the huMA79bv28 ADCs did not cause a significant decrease in body weight during the study.

**C. *In vivo* anti-tumor activity of humanized anti-CD79b antibody drug conjugates in a Granta-519 xenograft model**

To test the efficacy of Thio huMA79bv28 HC A118C conjugates with PNU-159682 ("huMA79bv28-PNU-1" and "huMA79bv28-PNU-2"), the effects of the conjugated antibodies in a mouse xenograft model of Granta-519 tumors (human mantle cell lymphoma cell line) was examined.

Female CB17 ICR SCID mice (10-11 weeks of age from Charles Rivers Laboratories; Hollister, CA) were each inoculated subcutaneously in the flank with 2 x $10^7$ Granta-519 cells (DSMZ, German Collection of Microorganisms and Cell Cultures, Braunschweig, Germany). When the xenograft tumors reached an average tumor volume of 150-250 $mm^3$ (referred to as Day 0), the first and only dose of treatment was administered. Tumor volume was calculated based on two dimensions, measured using calipers, and was expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach was used (*see, e.g.*, Pinheiro et al. 2009). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 1 mg ADC/kg of huMA79bv28 immunoconjugate or control antibody-drug conjugates (control ADCs). The control ADCs bind to a protein that is not expressed on the surface of Grant-519 cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 4 and Figure 7. Table 4 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 $mm^3$), the drug dose for each group, the antibody dose for each group, and the drug load for each ADC

administered.

Table 4: Anti-CD79b ADC administration to mice with Grant-519 xenografts

| Antibody administered (Treatment) | TI | PR | CR | Drug Dose-(μg//kg) | Ab Dose (mg/kg) | Drug Load (Drug /Ab) |
|---|---|---|---|---|---|---|
| Vehicle* | 8/8 | 0 | 0 | n/a | n/a | n/a |
| huMA79bv28-PNU-1 | 4/8 | 4 | 4 | 7.83 | 1 | 1.83 |
| Control-PNU-1 | 8/8 | 1 | 0 | 7.91 | 1 | 1.85 |
| huMA79bv28-PNU-2 | 8/8 | 2 | 0 | 7.96 | 1 | 1.86 |
| Control-PNU-2 | 8/8 | 0 | 0 | 8.13 | 1 | 1.9 |
| huMA79bv28-MMAE | 6/8 | 1 | 2 | 9.62 | 1 | 2.01 |
| * Vehicle = 20 mM histidine acetate, pH 5.5, 240 mM sucrose, 0.02% PS20; n/a = not applicable. | | | | | | |

In a 31 day time course with the ADCs and doses shown in Table 4, Thio huMA79bv28 conjugated through a protease cleavable linker with PNU-159682 ("huMA79bv28-PNU-1") showed inhibition of tumor growth in SCID mice with Granta-519 tumors compared to vehicle and the control ADC ("Control-PNU-1"). *See* Figure 7. Thio huMA79bv28 conjugated through an acid-labile linker with PNU-159682 ("huMA79bv28-PNU-2") also showed inhibition of tumor growth in SCID mice with Granta-519 tumors compared to the vehicle and the control ADC ("Control-PNU-2"). When given at 1 mg/kg, huMA79bv28-PNU-1 shows slightly more effective tumor growth inhibition than the humanized anti-CD79b thiomab conjugated with auristatin drug MMAE ("huMA79bv28-MMAE").

Four mice that received 1 mg/kg huMA79bv28-PNU-1 showed complete response, while only two mice that received 1 mg/kg huMA79bv28-MMAE showed complete response. See Table 4.

In this study, the percent body weight change was determined in each dosage group. The results indicated that administration of the huMA79bv28 ADCs did not cause a significant decrease in body weight during the study.

**D. *In vivo* anti-tumor activity of humanized anti-CD79b antibody drug conjugates in a SuDHL4-luc xenograft model**

To test the efficacy of Thio huMA79bv28 HC A118C conjugates with PNU-159682 ("huMA79bv28-PNU-1" and "huMA79bv28-PNU-2"), the effects of the conjugated antibodies in a mouse xenograft model of SuDHL4-luc tumors (diffuse large B-cell lymphoma cell line) was examined.

Female CB17 ICR SCID mice (10-11 weeks of age from Charles Rivers Laboratories; Hollister, CA) were each inoculated subcutaneously in the flank with 2 x $10^7$ SuDHL4-luc cells (obtained from DSMZ, German Collection of Microorganisms and Cell Cultures, Braunschweig, Germany, and engineered at Genentech to stably express a luciferase gene). When the xenograft tumors reached an average tumor volume of 150-250 $mm^3$ (referred to as Day 0), the first and only dose of treatment was administered. Tumor volume was calculated based on two dimensions, measured using calipers, and was expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach was used *(see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 7 mice were treated with a single intravenous (i.v.) dose of 1 mg ADC/kg of huMA79bv28 immunoconjugate or control antibody-drug conjugates (control ADCs). The control ADCs bind to a protein that is not expressed on the surface of SuDHL4-luc cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 5 and Figure 8. Table 5 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 $mm^3$), the drug dose for each group, the antibody dose for each group, and the drug load for each ADC administered.

Table 5: Anti-CD79b ADC administration to mice with SuDHL4-luc xenografts

| Antibody administered (Treatment) | TI | PR | CR | Drug Dose (μg//kg) | Ab Dose (mg/kg) | Drug Load (Drug /Ab) |
|---|---|---|---|---|---|---|
| Vehicle* | 7/7 | 0 | 0 | n/a | n/a | n/a |
| huMA79bv28-PNU-1 | 7/7 | 2 | 0 | 7.83 | 1 | 1.83 |
| Control -PNU-1 | 7/7 | 0 | 0 | 7.91 | 1 | 1.85 |
| huMA79bv28-PNU-2 | 7/7 | 1 | 0 | 7.96 | 1 | 1.86 |
| Control-PNU-2 | 7/7 | 0 | 0 | 8.13 | 1 | 1.9 |
| huMA79bv28-MMAE | 5/7 | 1 | 3 | 9.62 | 1 | 2.01 |
| Control-ADC-A118C-MC-vc-PAB-MMAE ("Control MMAE") | 7/7 | 0 | 0 | 10.05 | 1 | 2.1 |
| * Vehicle = 20 mM histidine acetate, pH 5.5, 240 mM sucrose, 0.02% PS20; n/a = not applicable. | | | | | | |

In a 21 day time course with drug conjugates and doses as shown in Table 5, Thio Hu anti-CD79b ADCs conjugated through a protease cleavable linker with PNU-159682 ("huMA79bv28-PNU-1") showed inhibition of tumor growth in SCID mice with SuDHL4-luc tumors compared to the vehicle and the control ADC ("Control-PNU-1"). *See* Figure 8. Thio huMA79bv28 conjugated through an acid-labile linker with PNU-159682 ("huMA79bv28-PNU-2") also showed inhibition of tumor growth in SCID mice with SuDHL4-luc tumors compared to the vehicle and the control ADC ("Control-PNU-2").

Furthermore, 1 mg/kg huMA79bv28-PNU-1 showed comparable anti-tumor activity to the humanized anti-CD79b thiomab conjugated with auristatin drug MMAE ("huMA79bv28-MMAE").

In this study, the percent body weight change was determined in each dosage group. The results indicated that administration of the huMA79bv28 ADCs did not cause a significant decrease in body weight during the study.

**E. Dose escalation study of huMA79bv28-PNU-1 in a SuDHL4-luc xenograft model**

The efficacy of huMA79bv28-PNU-1 at various dose levels in a mouse xenograft model of SuDHL4-luc tumors (diffuse large B-cell lymphoma cell line) was examined.

Female CB17 ICR SCID mice (10-11 weeks of age from Charles Rivers Laboratories; Hollister, CA) were each inoculated subcutaneously in the flank with 2 x $10^7$ SuDHL4-luc cells (obtained from DSMZ, German Collection of Microorganisms and Cell Cultures, Braunschweig, Germany, and engineered at Genentech to stably express a luciferase gene). When the xenograft tumors reached an average tumor volume of 150-300 $mm^3$ (referred to as Day 0), the first and only dose of treatment was administered. Tumor volume was calculated based on two dimensions, measured using calipers, and was expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 7 mice were treated with a single intravenous (i.v.) dose of 0.2, 0.5, 1, or 2 mg ADC/kg of huMA79bv28-PNU-1 or Control-PNU-1, which binds to a protein that is not expressed on the surface of SuDHL4-luc cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 6 and Figure 9. Table 6 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 $mm^3$), the drug dose for each group, the antibody dose for each group, and the drug load for each ADC administered.

Table 6: Anti-CD79b ADC administration to mice with SuDHL4-luc xenografts

| **Antibody administered (Treatment)** | **TI** | **PR** | **CR** | **Drug Dose (μg//kg)** | **Ab Dose (mg/kg)** | **Drug Load (Drug /Ab)** |
|---|---|---|---|---|---|---|
| Vehicle* | 7/7 | 0 | 0 | n/a | n/a | n/a |
| huMA79bv28-PNU-1 | 7/7 | 1 | 0 | 1.63 | 0.2 | 1.9 |
| huMA79bv28-PNU-1 | 7/7 | 1 | 0 | 4.06 | 0.5 | 1.9 |
| huMA79bv28-PNU-1 | 6/7 | 1 | 1 | 8.13 | 1 | 1.9 |
| huMA79bv28-PNU-l | 4/7 | 4 | 3 | 16.25 | 2 | 1.9 |
| Control-PNU-1 | 7/7 | 0 | 0 | 7.91 | 1 | 1.85 |
| Control-PNU-1 | 7/7 | 0 | 0 | 15.83 | 2 | 1.85 |
| * Vehicle = 20 mM histidine acetate, pH 5.5, 240 mM sucrose, 0.02% PS20; n/a = not applicable. | | | | | | |

In a 49 day time course with drug conjugates and doses as shown in Table 6, huMA79bv28-PNU-1 showed dose-dependent inhibition of tumor growth in SCID mice with SuDHL4-luc tumors. When administered at 0.2 mg/kg or higher dose, huMA79bv28-PNU-1 showed clear inhibitory activity compared to vehicle or the control ADC. *See* Figure 9. At 2 mg/kg, three mice administered huMA79bv28-PNU-1 showed a complete response and four mice showed a partial response. *See* Table 6.

In this study, the percent body weight change was determined in each dosage group. The results indicated that administration of huMA79bv28-PNU-1 did not cause a significant decrease in body weight during the study. Unrelated to treatment, one mouse given 1 mg/kg huMA79bv28-PNU-1 became anorexic and was euthanized before body weight loss reached 15%.

**F. Dose escalation study of huMA79bv28-PNU-1 in a BJAB-luc xenograft model**

The efficacy of huMA79bv28-PNU-1 at various dose levels in a mouse xenograft model of BJAB-luc tumors (Burkitt's lymphoma cell line) was examined.

Female CB17 ICR SCID mice (12-13 weeks of age from Charles Rivers Laboratories; Hollister, CA) were each inoculated subcutaneously in the flank with 2 x $10^7$ BJAB-luc cells (available, e.g., from Lonza, Basel, Switzerland, and engineered at Genentech to stably express a luciferase gene). When the xenograft tumors reached an average tumor volume of 150-300 $mm^3$ (referred to as Day 0), the first and only dose of treatment was administered. Tumor volume was calculated based on two dimensions, measured using calipers, and was expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.2, 0.5, 1, or 2 mg ADC/kg of huMA79bv28-PNU-1 or Control-PNU-1, which binds to a protein that is not expressed on the surface of BJAB-luc cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 7 and Figure 10. Table 8 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 $mm^3$), the drug dose for each group, the antibody dose for each group, and the drug load for each ADC administered.

Table 7: Anti-CD79b ADC administration to mice with BJAB-luc xenografts

| Antibody administered (Treatment) | TI | PR | CR | Drug Dose (μg//kg) | Ab Dose (mg/kg) | Drug Load (Drug /Ab) |
|---|---|---|---|---|---|---|
| Vehicle* | 8/8 | 0 | 0 | n/a | n/a | n/a |
| huMA79bv28-PNU-1 | 8/8 | 1 | 0 | 1.63 | 0.2 | 1.9 |
| huMA79bv28-PNU-1 | 7/8 | 2 | 1 | 4.06 | 0.5 | 1.9 |
| huMA79bv28-PNU-1 | 0/8 | 0 | 8 | 8.13 | 1 | 1.9 |
| huMA79bv28-PNU-1 | 0/8 | 0 | 8 | 16.25 | 2 | 1.9 |
| Control-PNU-1 | 8/8 | 0 | 0 | 7.91 | 1 | 1.85 |
| Control-PNU-1 | 8/8 | 0 | 0 | 15.83 | 2 | 1.85 |
| * Vehicle = 20 mM histidine acetate, pH 5.5, 240 mM sucrose, 0.02% PS20; n/a = not applicable. | | | | | | |

In a 41 day time course with drug conjugates and doses as shown in Table 8, huMA79bv28-PNU-1 showed dose-dependent inhibition of tumor growth in SCID mice with BJAB-luc tumors. When administered at 0.2 mg/kg or higher dose, huMA79bv28-PNU-1 showed clear inhibitory activity compared to vehicle or the control ADC. *See* Figure 10. In addition, a single dose of 1 or 2 mg/kg huMA79bv28-PNU-1 resulted in complete tumor remission in all treated animals.

In this study, the percent body weight change was determined in each dosage group. The results indicated that administration of huMA79bv28-PNU-1 did not cause a significant decrease in body weight during the study.

**G. Dose escalation study of huMA79bv28-MMAE in a BJAB-luc xenograft model**

The efficacy of huMA79bv28-MMAE at various dose levels in a mouse xenograft model of BJAB-luc tumors (Burkitt's lymphoma cell line) was examined.

Female CB17 ICR SCID mice (13-14 weeks of age from Charles Rivers Laboratories; Hollister, CA) were each inoculated subcutaneously in the flank with 2 x $10^7$ BJAB-luc cells (available, e.g., from Lonza, Basel, Switzerland, and engineered at Genentech to stably express a luciferase gene). When the xenograft tumors reached an average tumor volume of 150-300 $mm^3$ (referred to as Day 0), the first and only dose of treatment was administered. Tumor volume was calculated based on two dimensions, measured using calipers, and was expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.1, 0.5, 1, 2, OR 4 mg ADC/kg of huMA79bv28-MMAE, unconjugated huMA79bv28, or Control-MMAE, which binds to a protein that is not expressed on the surface of BJAB-luc cells. Tumors and body weights of mice were measured 1-2 times a week throughout the experiment. Mice were euthanized before tumor volumes reached 3000 $mm^3$ or when tumors showed signs of impending ulceration. All animal protocols were approved by an Institutional Animal Care and Use Committee (IACUC).

The results of that experiment are shown in Table 8 and Figure 11. Table 10 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 $mm^3$), the drug dose for each group, the antibody dose for each group, and the drug load for each ADC administered.

Table 8: Anti-CD79a ADC administration to mice with BJAB-luc xenografts

| Antibody administered (Treatment) | TI | PR | CR | Drug Dose (μg//kg) | Ab Dose (mg/kg) | Drug Load (Drug /Ab) |
|---|---|---|---|---|---|---|
| Vehicle* | 8/8 | 0 | 0 | n/a | n/a | n/a |

(continued)

| Antibody administered (Treatment) | TI | PR | CR | Drug Dose (μg//kg) | Ab Dose (mg/kg) | Drug Load (Drug /Ab) |
|---|---|---|---|---|---|---|
| huMA79bv28-MMAE | 8/8 | 0 | 0 | 1.77 | 0.1 | 3.7 |
| huMA79bv28-MMAE | 8/8 | 0 | 0 | 8.86 | 0.5 | 3.7 |
| huMA79bv28-MMAE | 8/8 | 4 | 0 | 17.71 | 1 | 3.7 |
| huMA79bv28-MMAE | 1/8 | 1 | 7 | 35.42 | 2 | 3.7 |
| huMA79bv28-MMAE | 0/8 | 0 | 8 | 70.84 | 4 | 3.7 |
| Control-MMAE | 8/8 | 0 | 0 | 57.37 | 4 | 2.9 |
| huMA79bv28 | 8/8 | 0 | 0 | n/a | 4 | n/a |
| * Vehicle = 20 mM histidine acetate, pH 5.5, 240 mM sucrose, 0.02% PS20; n/a = not applicable. | | | | | | |

In a 42 day time course with drug conjugates and doses as shown in Table 10, huMA79bv28-MMAE showed dose-dependent inhibition of tumor growth in SCID mice with BJAB-luc tumors. In contrast to huMA79bv28-PNU-1, which showed complete inhibition at 1 mg ADC/kg, and very strong inhibition at 0.5 mg ADC/kg (see Figure 10), huMA79bv28-MMAE did not show complete inhibition until a dose of 2 mg ADC/kg.

In this study, the percent body weight change was determined in each dosage group. The results indicated that administration of huMA79bv28-MMAE did not cause a significant decrease in body weight during the study.

**H. Efficacy of Anti-CD79b Immunoconjugates Comprising a Nemorubicin Derivative in Bjab-luc Cells Resistant to anti-CD79b-vc-MMAE**

To determine the efficacy of an anti-CD79b immunoconjugate comprising a nemorubicin derivative in Non-Hodgkin's Lymphoma that had developed resistance to anti-CD79b-vc-MMAE, Bjab-luc cells that are resistant to anti-CD79b-vc-MMAE were developed *in vivo.*

CB17 SCID mice (Charles River Laboratories, Hollister, CA) were inoculated subcutaneously in the dorsal right flank with 20 million Bjab-luc cells in HBSS. Twenty mice inoculated with Bjab-luc cells were dosed with 1.5 mg/kg huMA79bv28-MC-vc-PAB-MMAE intravenously on day 0. To determine when the mice would be dosed again, and at what doses, the following was taken into consideration: whether or not tumors re-grew after the initial treatment (i.e., tumors that grew back to initial tumor volume size at day 0), and the rate of regrowth. Frequency of doses administered varied over time but did not exceed 2 doses/week. Intravenous doses did not exceed 300 μL. The range of doses administered were 1.5, 2, 3, 3.5, 4, 5, 6, 8, 10, 15, 18, 20 and 30 mg/kg. Dosing was discontinued once a tumor no longer responded (i.e., it showed resistance to) a series of increasing doses.

Two resistant lines were generated, referred to as BJAB.Luc_SN8v28-vcE(CD79b)_T1.1X1 and BJAB.Luc_SN8v28-vcE(CD79b)_T1.2X1. CD79b was expressed on the surface of the resistant cells, and anti-CD79b antibodies were internalized by the resistant cells. Resistance *in vivo* to thio huMA79bv28 HC A118C-MC-val-cit-PAB-PNU-159682 was confirmed in BJAB.Luc_SN8v28-vcE(CD79b)_T1.1X1 xenograft model.

Expression of P-glycoprotein (P-gp, also referred to as multidrug resistance protein 1, or MDR1) in the resistant cells and the parental Bjab-luc cells was determined by RT-PCR, FACS, and surface plasmon resonance. All three methods showed that P-gp was upregulated in BJAB.Luc_SN8v28-vcE(CD79b)_T1.1X1 and BJAB.Luc_SN8v28-vcE(CD79b)_T1.2X1 cells, as compared to parental Bjab-luc cells. Figure 12 shows the expression of P-gp in the resistant cells as determined by FACS.

To support the hypothesis that P-gp is at least partially responsible for the huMA79bv28-MMAE resistance observed in BJAB.Luc_SN8v28-vcE(CD79b)_T1.1X1 and BJAB.Luc_SN8v28-vcE(CD79b)_T1.2X1 cells, Bjab-luc cells stably expressing P-gp were developed. Figure 12 shows expression of P-gp in two stably expressing Bjab-luc cell lines (a high-expressing cell line and a low-expressing cell line). The Bjab-luc_P-gp cells were also found to be resistant to huMA79bv28-MMAE.

To determine whether an anti-CD79b immunoconjugate comprising a nemorubicin derivative may be effective against the resistant cells, the efficacy of PNU-159682 was tested in the P-gp-expressing Bjab-luc cell lines. As shown in Figure 13, although PNU-159682 is an anthracycline analog, it appeared not to be a substrate of P-gp. The P-gp high-expressing Bjab-luc cells and the P-gp low-expressing Bjab-luc cells were both sensitive to PNU-159682.

Efficacy of thio huMA79bv28 HC A118C-MC-val-cit-PAB-PNU-159682 ("huMA79bv28-PNU-1") is deteremined in a BJAB.Luc_SN8v28-vcE(CD79b)_T1.2X1 xenograft model. Female CB17 ICR SCID mice (10 weeks of age from

Charles Rivers Laboratories; Hollister, CA) are each inoculated subcutaneously in the flank with 2 x $10^7$ BJAB.Luc_SN8v28-vcE(CD79b)_T1.2X1 cells. When the xenograft tumors reach an average tumor volume of 100-200 $mm^3$ (referred to as Day 0), the first and only dose of treatment is administered. Tumor volume is calculated based on two dimensions, measured using calipers, and is expressed in $mm^3$ according to the formula: $V= 0.5a \times b^2$, wherein a and b are the long and the short diameters of the tumor, respectively. To analyze the repeated measurement of tumor volumes from the same animals over time, a mixed modeling approach may be used (*see, e.g.,* Pinheiro et al. 2008). This approach can address both repeated measurements and modest dropout rates due to non-treatment related removal of animals before the study end. Cubic regression splines are used to fit a non-linear profile to the time courses of log2 tumor volume at each dose level. These non-linear profiles are then related to dose within the mixed model.

Groups of 8 mice are treated with a single intravenous (i.v.) dose of 1-2 mg ADC/kg of huMA79bv28-PNU-1 or 8-12 mg ADC/kg huMA79bv28-MC-vc-PAB-MMAE. Tumors and body weights of mice are measured 1-2 times a week throughout the experiment. Mice are euthanized before tumor volumes reached 3000 $mm^3$ or when tumors show signs of impending ulceration.

It is expected that BJAB.Luc_SN8v28-vcE(CD79b)_T1.1X1 cells are resistant to huMA79bv28-MC-vc-PAB-MMAE *in vivo,* but are sensitive to huMA79bv28-PNU-1.

**Table of Sequences**

| SEQ ID NO | Description | Sequence |
|---|---|---|
| 1 | humIII variable region sequence | EVQLVESGGG LVQPGGSLRL SCAASGFTFS SYAMSWVRQA PGKGLEWVSV ISGDGGSTYY ADSVKGRFTI SRDNSKNTLY LQMNSLRAED TAVYYCARGF DYWGQGTLVT VSS |
| 2 | humκ1 variable region sequence | DIQMTQSPSS LSASVGDRVT ITCRASQSIS NYLAWYQQKP GKAPKLLIYA ASSLESGVPS RFSGSGSGTD FTLTISSLQP EDFATYYCQQ YNSLPWTFGQ GTKVEIKR |
| 3 | MA79b heavy chain variable region | EVQLQQSGAE LMKPGASVKI SCKATGYTFS SYWIEWVKQR PGHGLEWIGE ILPGGGDTNY NEIFKGKATF TADTSSNTAY MQLSSLTSED SAVYYCTRRV PVYFDYWGQG TSVTVSS |
| 4 | MA79b light chain variable region | DIVLTQSPAS LAVSLGQRAT ISCKASQSVD YDGDSFLNWY QQKPGQPPKL FIYAASNLES GIPARFSGSG SGTDFTLNIH PVEEEDAATY YCQQSNEDPL TFGAGTELEL KR |
| 5 | huMA79b graft heavy chain variable region | EVQLVESGGG LVQPGGSLRL SCAASGYTFS SYWIEWVRQA PGKGLEWVGE ILPGGGDTNY NEIFKGRFTI SADTSKNTAY LQMNSLRAED TAVYYCTRRV PVYFDYWGQG TLVTVSS |
| 6 | huMA79b graft light chain variable region | DIQMTQSPSS LSASVGDRVT ITCKASQSVD YDGDSFLNWY QQKPGKAPKL LIYAASNLES GVPSRFSGSG SGTDFTLTIS SLQPEDFATY YCQQSNEDPL TFGQGTKVEI KR |
| 7 | huMA79bv17 heavy chain variable region | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PVYFDYWGQG TLVTVSS |
| 8 | huMA79bv17 light chain variable region | DIQLTQSPSS LSASVGDRVT ITCKASQSVD YDGDSFLNWY QQKPGKAPKL LIYAASNLES GVPSRFSGSG SGTDFTLTIS SLQPEDFATY YCQQSNEDPL TFGQGTKVEI KR |
| 9 | huMA79bv18 heavy chain variable region | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSS |
| 10 | huMA79bv18 light chain variable region | DIQLTQSPSS LSASVGDRVT ITCKASQSVD YDGDSFLNWY QQKPGKAPKL LIYAASNLES GVPSRFSGSG SGTDFTLTIS SLQPEDFATY YCQQSNEDPL TFGQGTKVEI KR |
| 11 | huMA79bv28 heavy chain variable region | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSS |

(continued)

| SEQ ID NO | Description | Sequence |
|---|---|---|
| 12 | huMA79bv28 light chain variable region | DIQLTQSPSS LSASVGDRVT ITCKASQSVD YEGDSFLNWY QQKPGKAPKL LIYAASNLES GVPSRFSGSG SGTDFTLTIS SLQPEDFATY YCQQSNEDPL TFGQGTKVEI KR |
| 13 | huMA79bv32 heavy chain variable region | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSS |
| 14 | huMA79bv32 light chain | DIQLTQSPSS LSASVGDRVT ITCKASQSVD YSGDSFLNWY QQKPGKAPKL FIYAASNLES GVPSRFSGSG SGTDFTLTIS SLQPEDFATY YCQQSNEDPL |
| | variable region | TFGQGTKVEI KR |
| 15 | MA79b HVR H1 | GYTFSSYWIE |
| 16 | MA79b HVR H2 | GEILPGGGDTNYNEIFKG |
| 17 | MA79b HVR H3 | TRRVPVYFDY |
| 18 | MA79b HVR L1 | KASQSVDYDGDSFLN |
| 19 | MA79b HVR L2 | AASNLES |
| 20 | MA79b HVR L3 | QQSNEDPLT |
| 21 | huMA79bv28 HVR H1 | GYTFSSYWIE |
| 22 | huMA79bv28 HVR H2 | GEILPGGGDTNYNEIFKG |
| 23 | huMA79bv28 HVR H3 | TRRVPIRLDY |
| 24 | huMA79bv28 HVR L1 | KASQSVDYEGDSFLN |
| 25 | huMA79bv28 HVR L2 | AASNLES |
| 26 | huMA79bv28 HVR L3 | QQSNEDPLT |
| 27 | huMA79bv28 heavy chain (HC) framework region (FR) 1 | EVQLVESGGGLVQPGGSLRLSCAAS |
| 28 | huMA79bv28 HC FR2 | WVRQAPGKGLEWI |
| 29 | huMA79bv28 HC FR3 | RATFSADTSKNTAYLQMNSLRAEDTAVYYC |
| 30 | huMA79bv28 HC FR4 | WGQGTLVTVSS |
| 31 | huMA79bv28 light chain (LC) FR1 | DIQLTQSPSSLSASVGDRVTITC |
| 32 | huMA79bv28 LC FR2 | WYQQKPGKAPKLLIY |

(continued)

| SEQ ID NO | Description | Sequence |
|---|---|---|
| 33 | huMA79bv28 LC FR3 | GVPSRFSGSGSGTDFTLTISSLQPEDFATYYC |
| 34 | huMA79bv28 LC FR4 | FGQGTKVEIKR |
| 35 | huMA79bv32 HVR L1 | KASQSVDYSGDSFLN |
| 36 | huMA79bv32 LC FR2 | WYQQKPGKAPKLLFY |
| 37 | huMA79bv28 light chain (Igκ) | DIQLTQSPSS LSASVGDRVT ITCKASQSVD YEGDSFLNWY QQKPGKAPKL LIYAASNLES GVPSRFSGSG SGTDFTLTIS SLQPEDFATY YCQQSNEDPL TFGQGTKVEI KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| 38 | huMA79bv28 heavy chain (IgG1) | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSSAST KGPSVFPLAP SSKSTSGGTA ALGCLVKDYF PEPVTVSWNS GALTSGVHTF PAVLQSSGLY SLSSVVTVPS SSLGTQTYIC NVNHKPSNTK VDKKVEPKSC DKTHTCPPCP APELLGGPSV FLFPPKPKDT LMISRTPEVT CVVVDVSHED PEVKFNWYVD GVEVHNAKTK PREEQYNSTY |
| | | RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPG |
| 39 | huMA79bv28 A118C cysteine engineered heavy chain (IgG1) | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSSCST KGPSVFPLAP SSKSTSGGTA ALGCLVKDYF PEPVTVSWNS GALTSGVHTF PAVLQSSGLY SLSSVVTVPS SSLGTQTYIC NVNHKPSNTK VDKKVEPKSC DKTHTCPPCP APELLGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPG |
| 40 | Human CD79b precursor; Acc. No. NP_000617.1; signal sequence = amino acids 1 to 28 | MARLALSPVP SHWMVALLLL LSAEPVPAAR SEDRYRNPKG SACSRIWQSP RFIARKRGFT VKMHCYMNSA SGNVSWLWKQ EMDENPQQLK LEKGRMEESQ NESLATLTIQ GIRFEDNGIY FCQQKCNNTS EVYQGCGTEL RVMGFSTLAQ LKQRNTLKDG IIMIQTLLII LFIIVPIFLL LDKDDSKAGM EEDHTYEGLD IDQTATYEDI VTLRTGEVKW SVGEHPGQE |
| 41 | Human mature CD79b, without signal sequence; amino acids 29 to 229 | AR SEDRYRNPKG SACSRIWQSP RFIARKRGFT VKMHCYMNSA SGNVSWLWKQ EMDENPQQLK LEKGRMEESQ NESLATLTIQ GIRFEDNGIY FCQQKCNNTS EVYQGCGTEL RVMGFSTLAQ LKQRNTLKDG IIMIQTLLII LFIIVPIFLL LDKDDSKAGM EEDHTYEGLD IDQTATYEDI VTLRTGEVKW SVGEHPGQE |
| 42 | Anti-CD22 10F4v3 HVR H1 | GYEFSRSWMN |
| 43 | Anti-CD22 10F4v3 HVR H2 | GRIYPGDGDTNYSGKFKG |
| 44 | Anti-CD22 10F4v3 HVR H3 | DGSSWDWYFDV |

(continued)

| SEQ ID NO | Description | Sequence |
|---|---|---|
| 45 | Anti-CD22 10F4v3 HVR L1 | RSSQSIVHSVGNTFLE |
| 46 | Anti-CD22 10F4v3 HVR L2 | KVSNRFS |
| 47 | Anti-CD22 10F4v3 HVR L3 | FQGSQFPYT |
| 48 | Anti-CD22 hu10F4v3 heavy chain variable region | EVQLVESGGG LVQPGGSLRL SCAASGYEFS RSWMNWVRQA PGKGLEWVGR IYPGDGDTNY SGKFKGRFTI SADTSKNTAY LQMNSLRAED TAVYYCARDG SSWDWYFDVW GQGTLVTVSS |
| 49 | Anti-CD22 hu10F4v3 light chain variable region | DIQMTQSPSS LSASVGDRVT ITCRSSQSIV HSVGNTFLEW YQQKPGKAPK LLIYKVSNRF SGVPSRFSGS GSGTDFTLTI SSLQPEDFAT YYCFQGSQFP YTFGQGTKVE IK |
| 50 | Anti-CD22 hu10F4v3 A118C cysteine engineered heavy chain | EVQLVESGGG LVQPGGSLRL SCAASGYEFS RSWMNWVRQA PGKGLEWVGR IYPGDGDTNY SGKFKGRFTI SADTSKNTAY LQMNSLRAED TAVYYCARDG SSWDWYFDVW GQGTLVTVSS CSTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKVEP KSCDKTHTCP PCPAPELLGG PSVFLFPPKP KDTLMISRTP EVTCVVVDVS HEDPEVKFNW YVDGVEVHNA KTKPREEQYN |
| | (IgG1) | STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPGK |
| 51 | Anti-CD22 hu10F4v3 light chain (Igκ) | DIQMTQSPSS LSASVGDRVT ITCRSSQSIV HSVGNTFLEW YQQKPGKAPK LLIYKVSNRF SGVPSRFSGS GSGTDFTLTI SSLQPEDFAT YYCFQGSQFP YTFGQGTKVE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC |
| 52 | Anti-CD22 hu10F4v3 S400C cysteine engineered heavy chain Fc region (IgG1) | EVQLVESGGG LVQPGGSLRL SCAASGYEFS RSWMNWVRQA PGKGLEWVGR IYPGDGDTNY SGKFKGRFTI SADTSKNTAY LQMNSLRAED TAVYYCARDG SSWDWYFDVW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDCDGSFFLY SKLTVDKSRW QQGNVFSCSV MHEALHNHYT QKSLSLSPGK |
| 53 | Anti-CD22 hu10F4v3 V205C cysteine engineered light chain (Igκ) | DIQMTQSPSS LSASVGDRVT ITCRSSQSIV HSVGNTFLEW YQQKPGKAPK LLIYKVSNRF SGVPSRFSGS GSGTDFTLTI SSLQPEDFAT YYCFQGSQFP YTFGQGTKVE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPC TKSFNRGEC |

(continued)

| SEQ ID NO | Description | Sequence |
|---|---|---|
| 56 | Anti-CD22 hu10F4v3 heavy chain Fc region (IgG1) | EVQLVESGGG LVQPGGSLRL SCAASGYEFS RSWMNWVRQA PGKGLEWVGR IYPGDGDTNY SGKFKGRFTI SADTSKNTAY LQMNSLRAED TAVYYCARDG SSWDWYFDVW GQGTLVTVSS ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKVEP KSCDKTHTCP PCPAPELLGG PSVFLFPPKP KDTLMISRTP EVTCVVVDVS HEDPEVKFNW YVDGVEVHNA KTKPREEQYN STYRVVSVLT VLHQDWLNGK EYKCKVSNKA LPAPIEKTIS KAKGQPREPQ VYTLPPSREE MTKNQVSLTC LVKGFYPSDI AVEWESNGQP ENNYKTTPPV LDSDGSFFLY SKLTVDKSRW QQGNVFSCSV MHEALHNHYT QKSLSLSPGK |
| 54 | huMA79bv28 V205C cysteine engineered light chain (Igκ) | DIQLTQSPSS LSASVGDRVT ITCKASQSVD YEGDSFLNWY QQKPGKAPKL LIYAASNLES GVPSRFSGSG SGTDFTLTIS SLQPEDFATY YCQQSNEDPL TFGQGTKVEI KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPCT KSFNRGEC |
| 55 | huMA79bv28 S400C cysteine engineered heavy chain (IgG1) | EVQLVESGGG LVQPGGSLRL SCAASGYTFS SYWIEWVRQA PGKGLEWIGE ILPGGGDTNY NEIFKGRATF SADTSKNTAY LQMNSLRAED TAVYYCTRRV PIRLDYWGQG TLVTVSSAST KGPSVFPLAP SSKSTSGGTA ALGCLVKDYF PEPVTVSWNS GALTSGVHTF PAVLQSSGLY SLSSVVTVPS SSLGTQTYIC NVNHKPSNTK VDKKVEPKSC DKTHTCPPCP APELLGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDC DGSFFLYSKL TVDKSRWQQG NVFSCSVMHE ALHNHYTQKS LSLSPGK |

## Claims

1. An immunoconjugate having the formula Ab-(L-D)p, wherein:

   (a) Ab is the antibody;
   (b) L is a linker;
   (c) D is the cytotoxic agent; and
   (d) p ranges from 1-8;

   wherein D has a structure selected from:

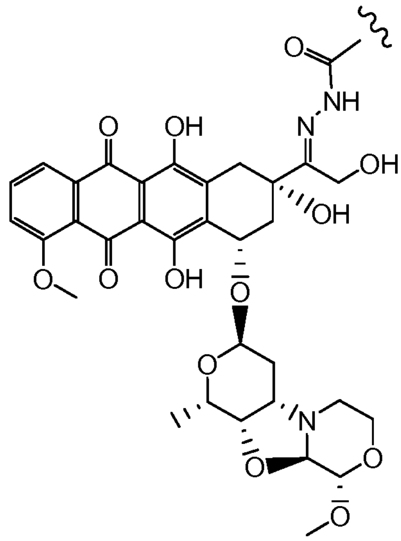

;

and

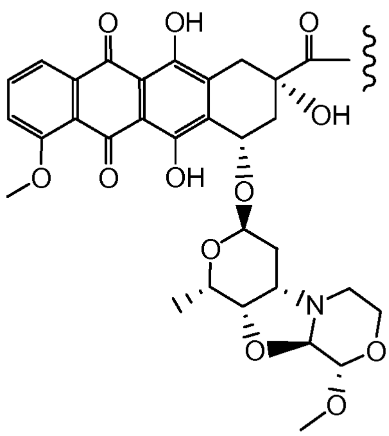

;

and
Ab is an antibody that binds human CD79b, wherein the antibody comprises:

a) (i) HVR-H1 comprising the amino acid sequence of SEQ ID NO: 21, (ii) HVR-H2 comprising the amino acid sequence of SEQ ID NO: 22, and (iii) HVR-H3 comprising the amino acid sequence of SEQ ID NO: 23; (iv) HVR-L1 comprising an amino acid sequence selected from SEQ ID NOs: 18, 24, and 35, (v) HVR-L2 comprising the amino acid sequence of SEQ ID NO: 25, and (vi) HVR-L3 comprising the amino acid sequence of SEQ ID NO: 26; or
b) (i) HVR-H1 comprising the amino acid sequence of SEQ ID NO: 21, (ii) HVR-H2 comprising the amino acid sequence of SEQ ID NO: 22, and (iii) HVR-H3 comprising the amino acid sequence of SEQ ID NO: 23; (iv)

HVR-L1 comprising the amino acid sequence of SEQ ID NO: 24, (v) HVR-L2 comprising the amino acid sequence of SEQ ID NO: 25, and (vi) HVR-L3 comprising the amino acid sequence of SEQ ID NO: 26; or
c) a VH sequence having at least 95% sequence identity to the amino acid sequence of SEQ ID NO: 11; or
d) a VL sequence having at least 95% sequence identity to the amino acid sequence of SEQ ID NO: 12; or
e) a VH sequence as in (c) and a VL sequence as in (d);
f) a VH sequence having an amino acid sequence selected from SEQ ID NOs: 9, 11, and 13; or
g) a VL sequence having an amino acid sequence selected from SEQ ID NOs: 8, 10, 12, and 14; or
h) a VH sequence as in (f) and a VL sequence as in (g); or
i) a VH sequence having the amino acid sequence of SEQ ID NO: 11; or
j) a VL sequence having the amino acid sequence of SEQ ID NO: 12; or
k) a VH sequence as in (i) and a VL sequence as in (j).

**2.** The immunoconjugate of claim 1, wherein the linker is cleavable by a protease, wherein the linker optionally comprises a val-cit dipeptide.

**3.** The immunoconjugate of claim 1, wherein the linker is acid-labile, wherein the linker optionally comprises hydrazone.

**4.** The immunoconjugate of claim 1 having a formula selected from:

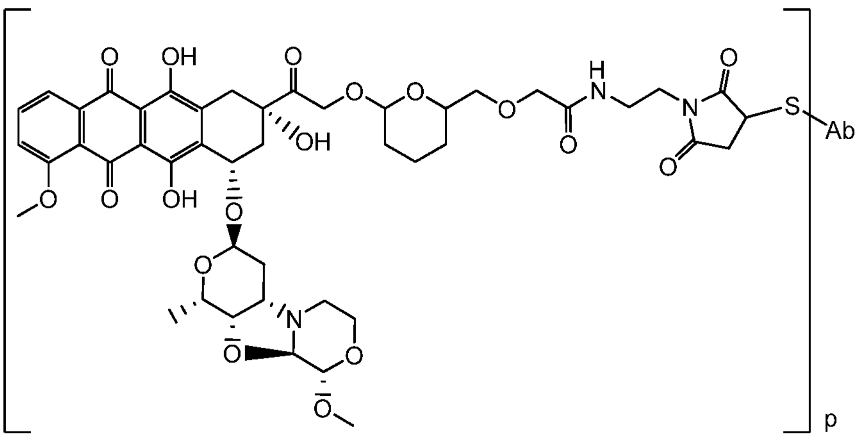

;

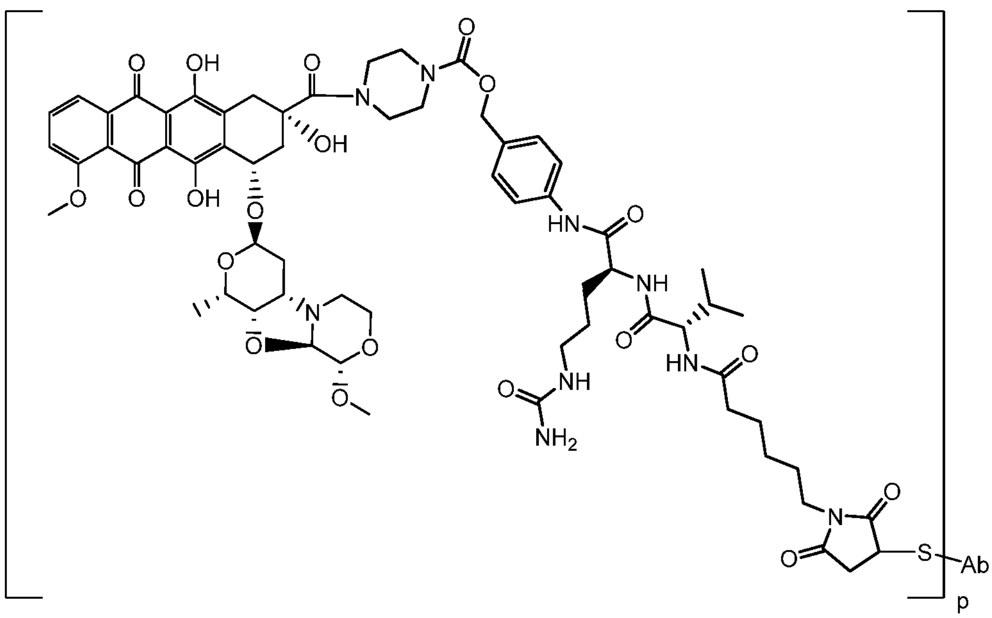

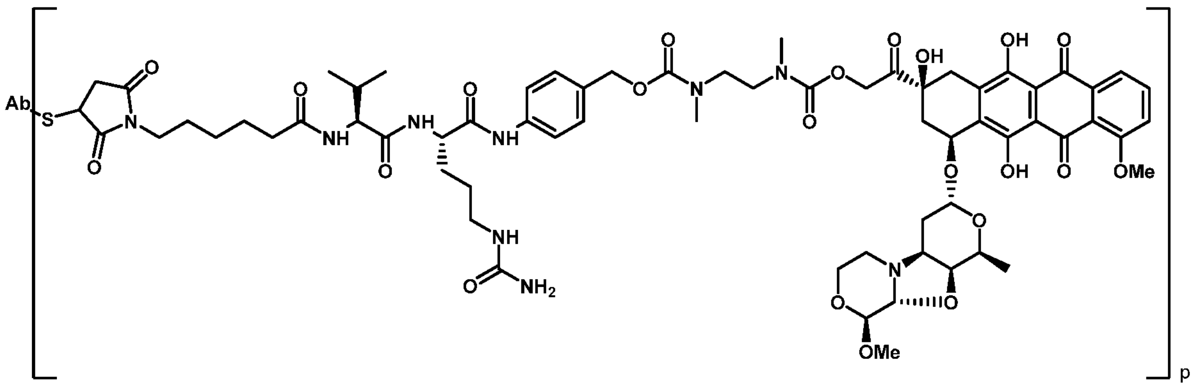

;

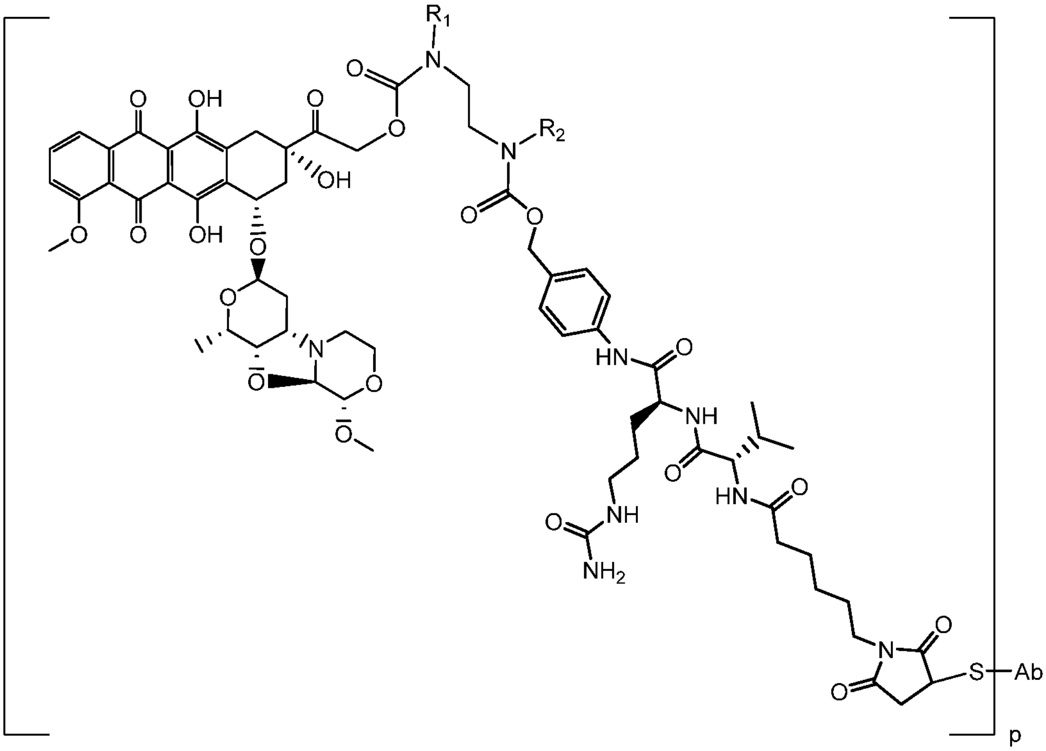

;

and

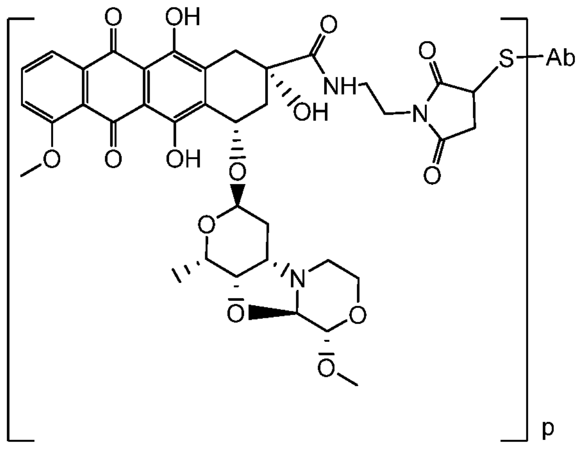

.

5. The immunoconjugate of claim 4, wherein Ab is an antibody comprising (i) HVR-H1 comprising the amino acid sequence of SEQ ID NO: 21, (ii) HVR-H2 comprising the amino acid sequence of SEQ ID NO: 22, (iii) HVR-H3 comprising the amino acid sequence of SEQ ID NO: 23, (iv) HVR-L1 comprising the amino acid sequence of SEQ ID NO: 24, (v) HVR-L2 comprising the amino acid sequence of SEQ ID NO: 25, and (vi) HVR-L3 comprising the amino acid sequence of SEQ ID NO: 26; and wherein p ranges from 1 to 3.

6. The immunoconjugate of claim 5, wherein the antibody comprises a VH sequence of SEQ ID NO: 11 and a VL sequence of SEQ ID NO: 12.

7. The immunoconjugate of claim 6, wherein the antibody comprises a heavy chain of SEQ ID NO: 39 and a light chain of SEQ ID NO: 37.

8. The immunoconjugate of any one of the preceding claims, wherein human CD79b has the sequence of SEQ ID NO: 40 or SEQ ID NO: 41.

9. A pharmaceutical formulation comprising the immunoconjugate of any one of the preceding claims and a pharmaceutically acceptable carrier.

10. The immunoconjugate of any one of claims 1 to 8 for use in a method of treating an individual having a CD79b-positive cancer.

11. The immunoconjugate for use of claim 10, wherein the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

12. The immunoconjugate for use of claim 10 or claim 11, wherein the CD79b-positive cancer is resistant to a first therapeutic, wherein the first therapeutic is a first immunoconjugate comprising a first antibody and a first cytotoxic agent.

13. The immunoconjugate for use of claim 12, wherein the first antibody binds CD22.

14. The immunoconjugate for use of claim 12 or claim 13, wherein the first cytotoxic agent and the cytotoxic agent of the immunoconjugate of any one of claims 1 to 8 are different.

15. The immunoconjugate for use of claim 14, wherein the first cytotoxic agent is MMAE.

16. The immunoconjugate for use of any one of claims 10 to 15, wherein the CD79b-positive cancer expresses P-glycoprotein (P-gp).

**Patentansprüche**

1. Immunkonjugat der Formel Ab-(L-D)p, worin:

   (a) Ab der Antikörper ist;
   (b) L ein Linker ist;
   (c) D das zytotoxische Mittel ist und
   (d) p im Bereich von 1 bis 8 liegt;

   worin D eine Struktur ausgewählt aus

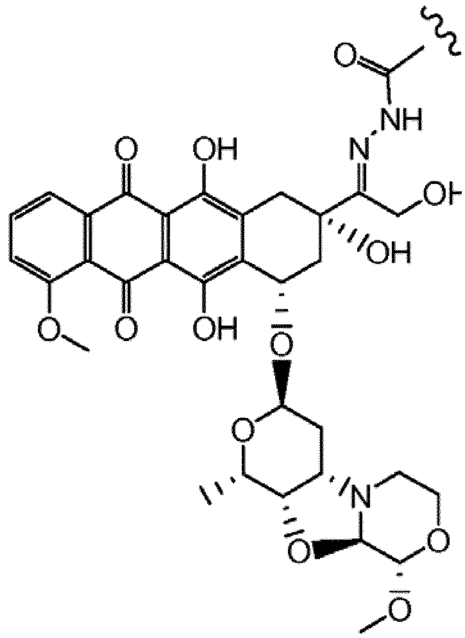

und

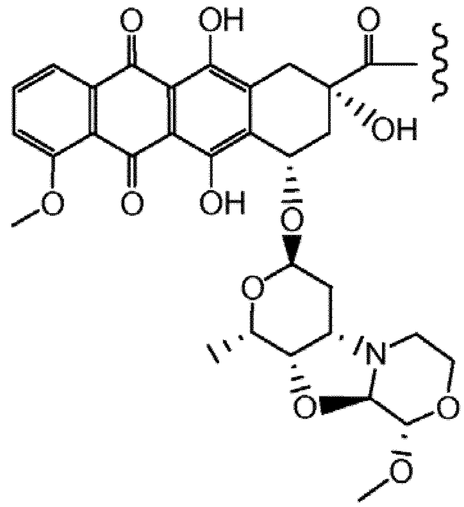

aufweist, und
Ab ein Antikörper ist, der menschliches CD79b bindet, wobei der Antikörper Folgendes umfasst:

a) (i) HVR-H1, umfassend die Aminosäuresequenz von Seq.-ID Nr. 21, (ii) HVR-H2, umfassend die Aminosäuresequenz von Seq.-ID Nr. 22 und (iii) HVR-H3, umfassend die Aminosäuresequenz von Seq.-ID Nr. 23; (iv)

HVR-L1, umfassend eine Aminosäuresequenz, ausgewählt aus Seq.-ID Nr. 18, 24 und 35, (v) HVR-L2, umfassend die Aminosäuresequenz von Seq.-ID Nr. 25 und (vi) HVR-L3, umfassend die Aminosäuresequenz von Seq.-ID Nr. 26; oder

b) (i) HVR-H1, umfassend die Aminosäuresequenz von Seq.-ID Nr. 21, (ii) HVR-H2, umfassend die Aminosäuresequenz von Seq.-ID Nr. 22 und (iii) HVR-H3, umfassend die Aminosäuresequenz von Seq.-ID Nr. 23; (iv) HVR-L1, umfassend die Aminosäuresequenz von Seq.-ID Nr. 24, (v) HVR-L2, umfassend die Aminosäuresequenz von Seq.-ID Nr. 25 und (vi) HVR-L3, umfassend die Aminosäuresequenz von Seq.-ID Nr. 26; oder

c) eine VH-Sequenz, die zumindest 95 % Sequenzidentität mit der Aminosäuresequenz von Seq.-ID Nr. 11 aufweist; oder

d) eine VL-Sequenz, die zumindest 95 % Sequenzidentität mit der Aminosäuresequenz von Seq.-ID Nr. 12 aufweist; oder

e) eine VH-Sequenz gemäß (c) und eine VL-Sequenz gemäß (d);

f) eine VH-Sequenz mit einer Aminosäuresequenz, ausgewählt aus Seq.-ID Nr. 9, 11 und 13; oder

g) eine VL-Sequenz mit einer Aminosäuresequenz, ausgewählt aus Seq.-ID Nr. 8, 10, 12 und 14; oder

h) eine VH-Sequenz gemäß (f) und eine VL-Sequenz gemäß (g); oder

i) eine VH-Sequenz mit der Aminosäuresequenz von Seq.-ID Nr. 11; oder

j) eine VL-Sequenz mit der Aminosäuresequenz von Seq.-ID Nr. 12; oder

k) eine VH-Sequenz gemäß (i) und eine VL-Sequenz gemäß (j).

**2.** Immunkonjugat nach Anspruch 1, wobei der Linker mittels einer Protease abspaltbar ist, wobei der Linker gegebenenfalls ein Val-Cit-Dipeptid umfasst.

**3.** Immunkonjugat nach Anspruch 1, wobei der Linker säurelabil ist, wobei der Linker gegebenenfalls Hydrazon umfasst.

**4.** Immunkonjugat nach Anspruch 1 mit einer Formel, ausgewählt aus:

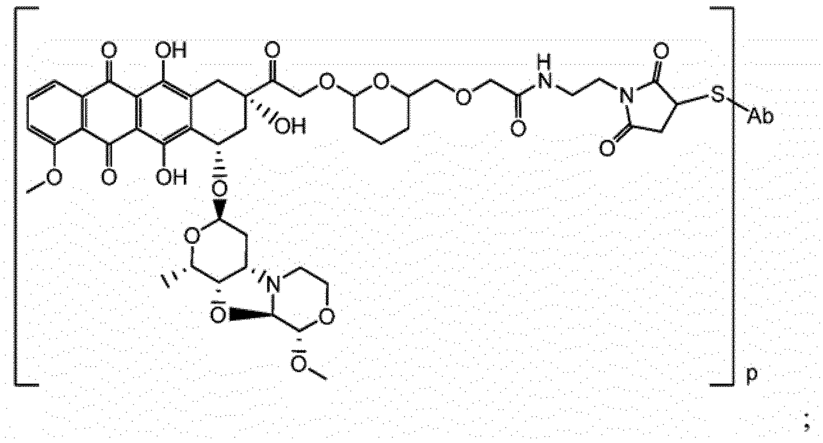

;

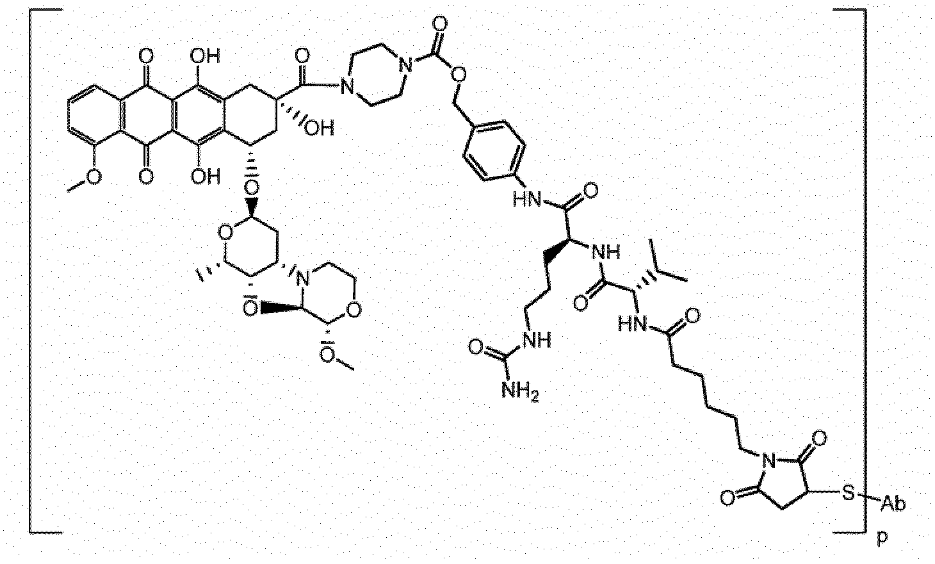

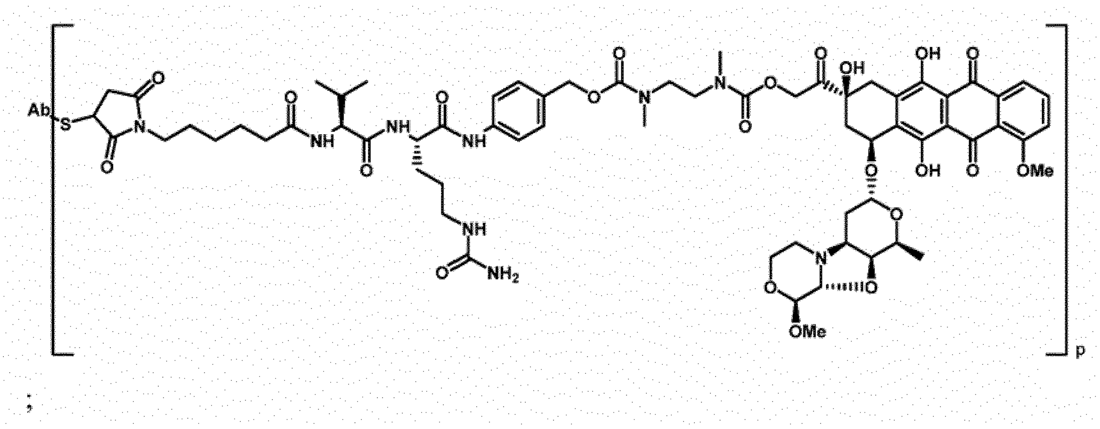

;

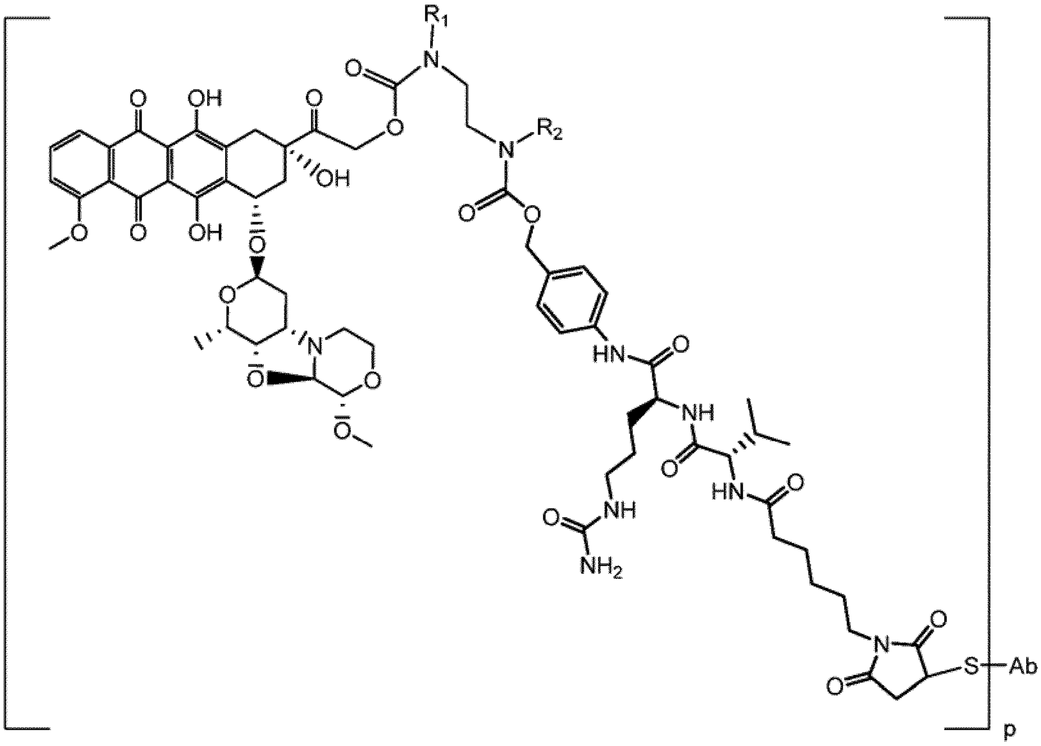

;

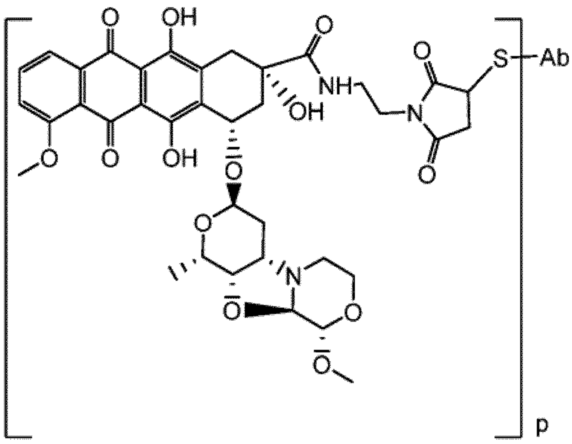

.

5. Immunkonjugat nach Anspruch 4, wobei Ab ein Antikörper ist, der Folgendes umfasst: (i) HVR-H1, umfassend die Aminosäuresequenz von Seq.-ID Nr. 21, (ii) HVR-H2, umfassend die Aminosäuresequenz von Seq.-ID Nr. 22 und (iii) HVR-H3, umfassend die Aminosäuresequenz von Seq.-ID Nr. 23; (iv) HVR-L1, umfassend die Aminosäuresequenz von Seq.-ID Nr. 24, (v) HVR-L2, umfassend die Aminosäuresequenz von Seq.-ID Nr. 25 und (vi) HVR-L3, umfassend die Aminosäuresequenz von Seq.-ID Nr. 26, wobei p im Bereich von 1 bis 3 liegt.

6. Immunkonjugat nach Anspruch 5, wobei der Antikörper eine VH-Sequenz von Seq.-ID Nr. 11 und eine VL-Sequenz von Seq.-ID Nr. 12 umfasst.

7. Immunkonjugat nach Anspruch 6, wobei der Antikörper eine Schwerkette von Seq.-ID Nr. 39 und eine Leichtkette von Seq.-ID Nr. 37 umfasst.

8. Immunkonjugat nach einem der vorangegangenen Ansprüche, wobei menschliches CD79b die Sequenz von Seq.-ID Nr. 40 oder Seq.-ID Nr. 41 aufweist.

9. Pharmazeutische Formulierung, die ein Immunkonjugat nach einem der vorangegangenen Ansprüche und einen pharmazeutisch annehmbaren Träger umfasst.

10. Immunkonjugat nach einem der Ansprüche 1 bis 8 zur Verwendung in einem Verfahren zur Behandlung eines Individuums mit einem CD79b-positiven Krebs.

11. Immunkonjugat zur Verwendung nach Anspruch 10, wobei der CD79b-positive Krebs ausgewählt ist aus Lyphom, Non-Hodgkins-Lymphom (NHL), aggressivem NHL, rezidivierendem aggressivem NHL, rezidivierendem indolentem NHL, refraktärem NHL, refraktärem indolentem NHL, chronischer lymphatischer Leukämie (CLL), kleinem lymphozytischem Lymphom, Leukämie, Haarzellleukämie (HCL), akuter lymphatischer Leukämie (ALL), Burkitt-Lymphom und Mantelzelllymphom.

12. Immunkonjugat zur Verwendung nach Anspruch 10 oder 11, wobei der CD79b-positive Krebs gegenüber einem ersten Therapeutikum resistent ist, wobei das erste Therapeutikum ein erstes Immunkonjugat ist, das einen ersten Antikörper und ein erstes zytotoxisches Mittel umfasst.

13. Immunkonjugat zur Verwendung nach Anspruch 12, wobei der erste Antikörper CD22 bindet.

14. Immunkonjugat zur Verwendung nach Anspruch 12 oder 13, wobei das erste zytotoxische Mittel und das zytotoxische Mittel des Immunkonjugats nach einem der Ansprüche 1 bis 8 unterschiedlich sind.

15. Immunkonjugat zur Verwendung nach Anspruch 14, wobei das erste zytotoxische Mittel MMAE ist.

16. Immunkonjugat zur Verwendung nach einem der Ansprüche 10 bis 15, wobei der CD79b-positive Krebs P-Glykoprotein (P-gp) exprimiert.

## Revendications

1. Immunoconjugué ayant la formule Ab-(L-D)p, dans laquelle :

(a) Ab est un anticorps ;
(b) L est un lieur ;
(c) D est un agent cytotoxique ; et
(d) p se situe dans la plage allant de 1 à 8 ;

où D a une structure choisie parmi :

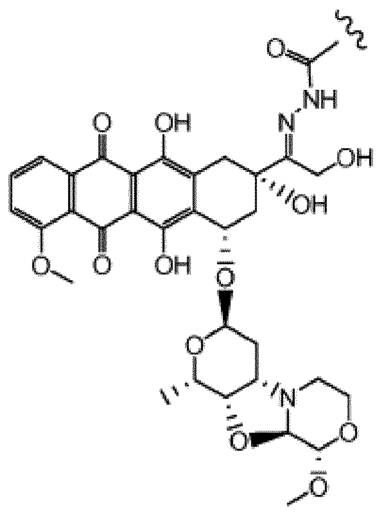
;

et

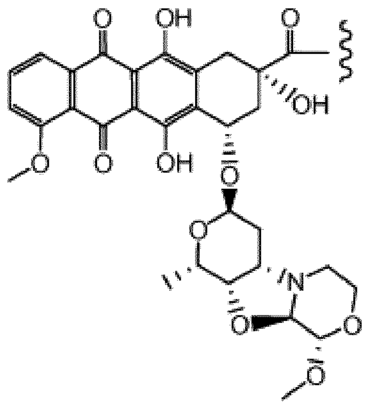

;

et

Ab est un anticorps qui se lie au CD79b humain, où l'anticorps comprend :

a) (i) une HVR-H1 comprenant la séquence d'acides aminés de la SEQ ID n° 21, (ii) une HVR-H2 comprenant la séquence d'acides aminés de la SEQ ID n° 22, et (iii) une HVR-H3 comprenant la séquence d'acides aminés de la SEQ ID n° 23 ; (iv) une HVR-L1 comprenant une séquence d'acides aminés choisie parmi les SEQ ID n° 18, 24 et 35, (v) une HVR-L2 comprenant la séquence d'acides aminés de la SEQ ID n° 25, et (vi) une HVR-L3 comprenant la séquence d'acides aminés de la SEQ ID n° 26 ; ou
b) (i) une HVR-H1 comprenant la séquence d'acides aminés de la SEQ ID n° 21, (ii) une HVR-H2 comprenant la séquence d'acides aminés de la SEQ ID n° 22, et (iii) une HVR-H3 comprenant la séquence d'acides aminés de la SEQ ID n° 23 ; (iv) une HVR-L1 comprenant une séquence d'acides aminés de la SEQ ID n° 24, (v) une HVR-L2 comprenant la séquence d'acides aminés de la SEQ ID n° 25, et (vi) une HVR-L3 comprenant la séquence d'acides aminés de la SEQ ID n° 26 ; ou
c) une séquence VH ayant au moins 95 % d'identité de séquence avec la séquence d'acides aminés de la SEQ ID n° 11 ; ou
d) une séquence VL ayant au moins 95 % d'identité de séquence avec la séquence d'acides aminés de la SEQ ID n° 12 ; ou
e) une séquence VH comme dans (c) et une séquence VL comme dans (d) ;
f) une séquence VH ayant une séquence d'acides aminés choisie parmi les SEQ ID n° 9, 11 et 13 ; ou
g) une séquence VL ayant une séquence d'acides aminés choisie parmi les SEQ ID n° 8, 10, 12 et 14 ; ou
h) une séquence VH comme dans (f) et une séquence VL comme dans (g) ; ou
i) une séquence VH ayant la séquence d'acides aminés de SEQ ID n° 11 ; ou
j) une séquence VL ayant la séquence d'acides aminés de SEQ ID n° 12 ; ou
k) une séquence VH comme dans (i) et une séquence VL comme dans (j).

**2.** Immunoconjugué selon la revendication 1, dans lequel le lieur est clivable par une protéase, où le lieur comprend éventuellement un dipeptide val-cit.

**3.** Immunoconjugué selon la revendication 1, dans lequel le lieur est labile pour un acide, où le lieur comprend éventuellement de l'hydrazone.

**4.** Immunoconjugué selon la revendication 1 ayant une formule choisie parmi :

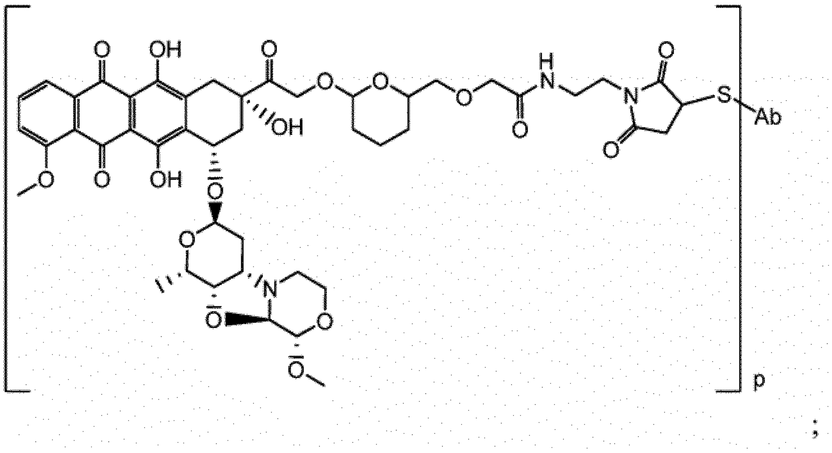

;

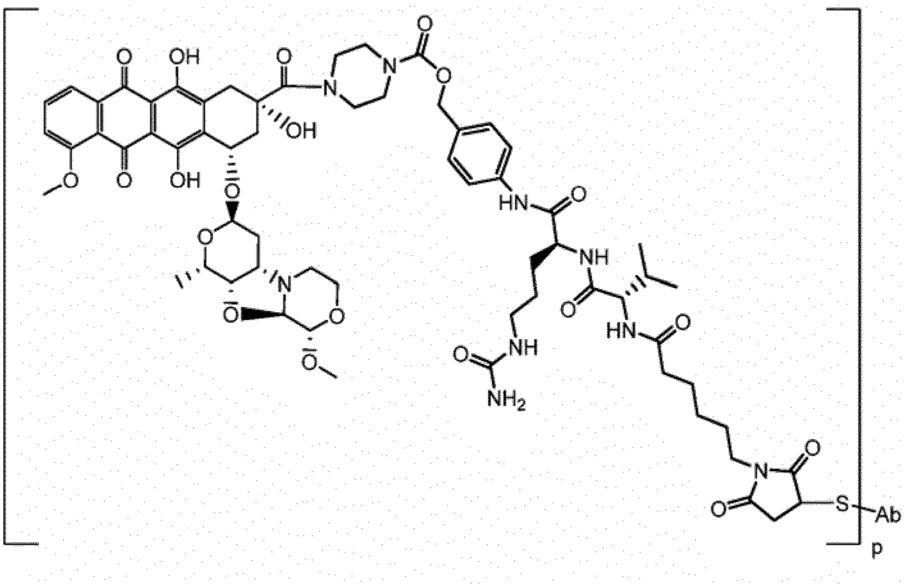

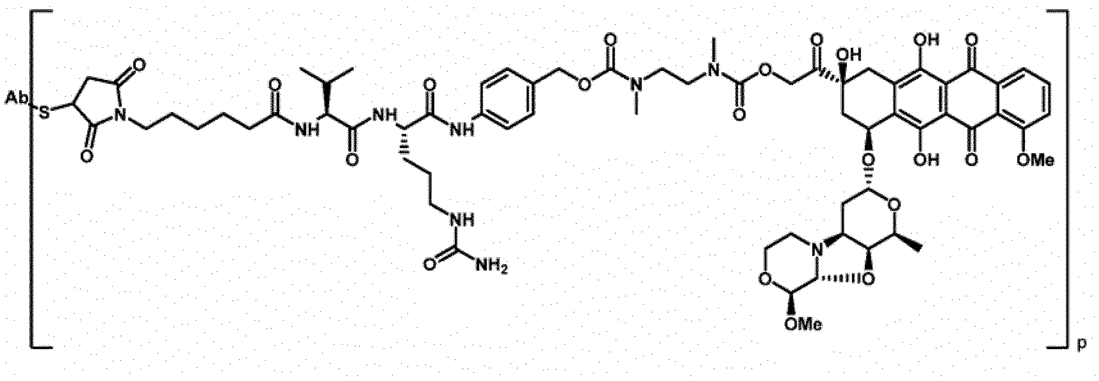

;

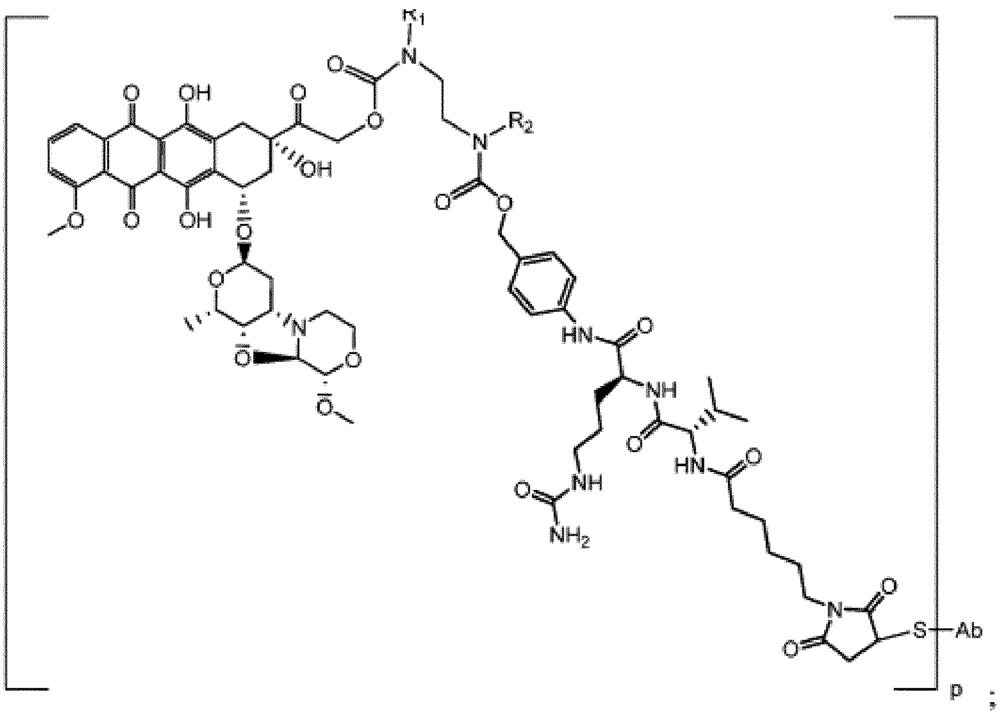

et

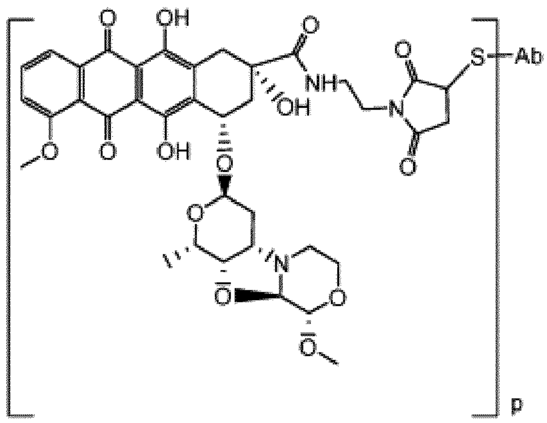

5. Immunoconjugué selon la revendication 4, dans lequel Ab est un anticorps comprenant (i) une HVR-H1 comprenant la séquence d'acides aminés de la SEQ ID n° 21, (ii) une HVR-H2 comprenant la séquence d'acides aminés de la SEQ ID n° 22, et (iii) une HVR-H3 comprenant la séquence d'acides aminés de la SEQ ID n° 23 ; (iv) une HVR-L1 comprenant la séquence d'acides aminés de la SEQ ID n° 24, (v) une HVR-L2 comprenant la séquence d'acides aminés de la SEQ ID n° 25, et (vi) une HVR-L3 comprenant la séquence d'acides aminés de la SEQ ID n° 26 ; et où p se situe dans la plage allant de 1 à 3.

6. Immunoconjugué selon la revendication 5, dans lequel l'anticorps comprend une séquence VH de la SEQ ID n° 11 et une séquence VL de la SEQ ID n° 12.

7. Immunoconjugué selon la revendication 6, dans lequel l'anticorps comprend une chaîne lourde de la SEQ ID n° 39 et une chaîne légère de la SEQ ID n° 37.

8. Immunoconjugué selon l'une quelconque des revendications précédentes, le CD79b humain ayant la séquence de la SEQ ID n° 40 ou de la SEQ ID n° 41.

9. Formulation pharmaceutique comprenant l'immunoconjugué selon l'une quelconque des revendications précédentes et un véhicule pharmaceutiquement acceptable.

**10.** Immunoconjugué selon l'une quelconque des revendications 1 à 8 destiné à être utilisé dans un procédé de traitement d'un individu ayant un cancer positif pour le CD79b.

**11.** Immunoconjugué destiné à être utilisé selon la revendication 10, le cancer positif pour le CD79b étant choisi parmi un lymphome, un lymphome non hodgkinien (NHL), un NHL agressif, un NHL agressif en rechute, un NHL indolent en rechute, un NHL réfractaire, un NHL indolent réfractaire, une leucémie lymphocytaire chronique (CLL), un lymphome lymphocytaire petit, une leucémie, une leucémie à cellules trichodermiques (HCL), une leucémie lymphocytaire aiguë (ALL), le lymphome de Burkitt, et un lymphome à cellules du manteau.

**12.** Immunoconjugué destiné à être utilisé selon la revendication 10 ou la revendication 11, le cancer positif pour le CD79b étant résistant à un premier agent thérapeutique, où le premier agent thérapeutique est un premier immunoconjugué comprenant un premier anticorps et un premier agent cytotoxique.

**13.** Immunoconjugué destiné à être utilisé selon la revendication 12, le premier anticorps se liant au CD22.

**14.** Immunoconjugué destiné à être utilisé selon la revendication 12 ou la revendication 13, le premier agent cytotoxique et l'agent cytotoxique de l'immunoconjugué selon l'une quelconque des revendications 1 à 8 étant différents.

**15.** Immunoconjugué destiné à être utilisé selon la revendcation 14, le premier agent cytotoxique étant le MMAE.

**16.** Immunoconjugué destiné à être utilisé selon l'une quelconque des revendications 10 à 15, le cancer positif pour le CD79b exprimant la glycoprotéine P (P-gp).

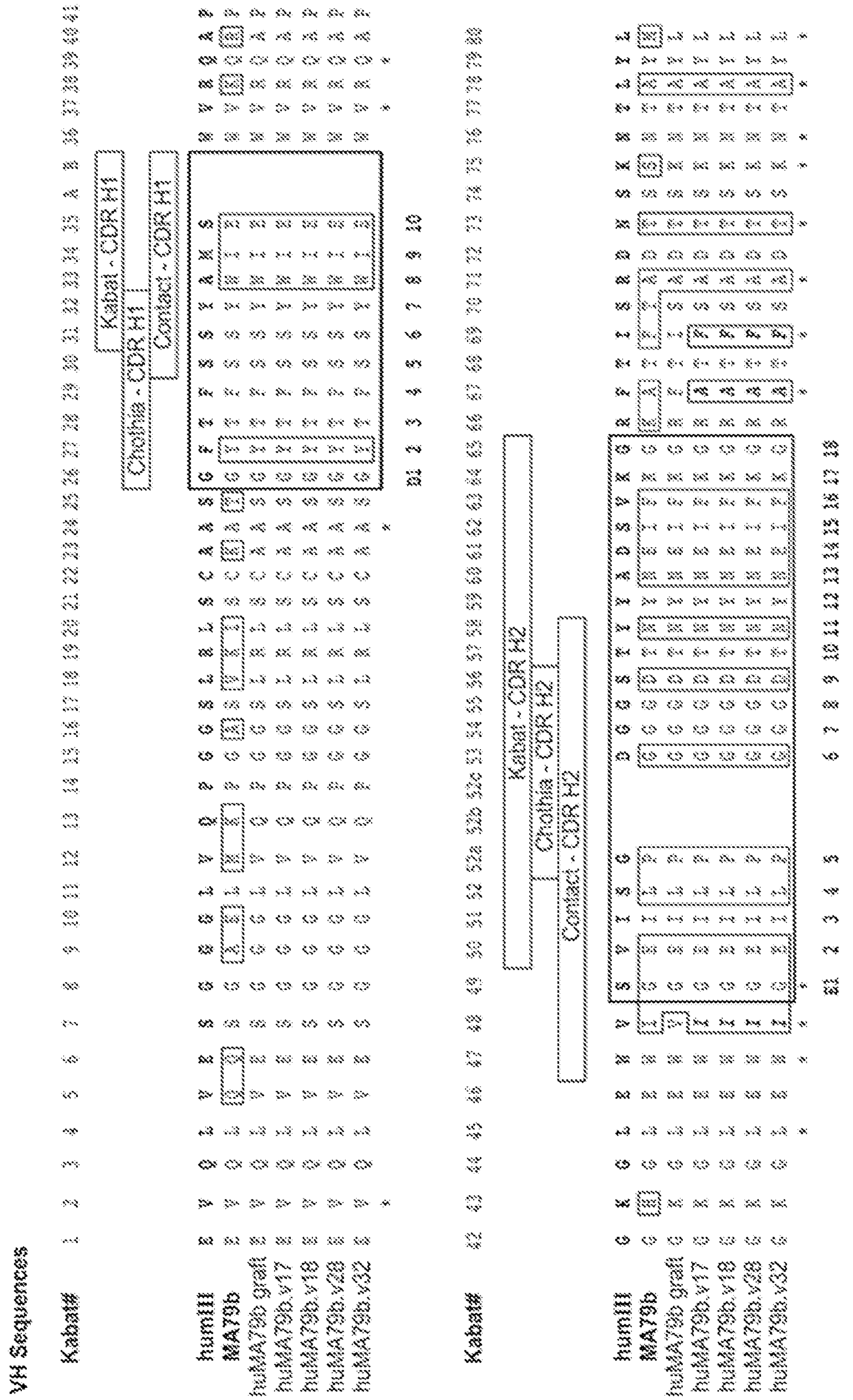
VH Sequences
Kabat#
humIII
MA79b
huMA79b graft
huMA79b.v17
huMA79b.v18
huMA79b.v28
huMA79b.v32
Kabat - CDR H1
Chothia - CDR H1
Contact - CDR H1
Kabat - CDR H2
Chothia - CDR H2
Contact - CDR H2

FIG. 1A

**VH Sequences**

| Kabat# | 81 | 82 | A | B | C | 83 | 84 | 85 | 86 | 87 | 88 | 89 | 90 | 91 | 92 | 93 | 94 |
|---|---|---|---|---|---|---|---|---|---|---|---|---|---|---|---|---|---|
| **humIII** | Q | M | N | S | L | R | A | E | D | T | A | V | Y | Y | C | A | R |
| **MA79b** | Q | L | S | S | L | T | S | E | D | S | A | V | Y | Y | C | T | R |
| huMA79b graft | Q | M | N | S | L | R | A | E | D | T | A | V | Y | Y | C | T | R |
| huMA79b.v17 | Q | M | N | S | L | R | A | E | D | T | A | V | Y | Y | C | T | R |
| huMA79b.v18 | Q | M | N | S | L | R | A | E | D | T | A | V | Y | Y | C | T | R |
| huMA79b.v28 | Q | M | N | S | L | R | A | E | D | T | A | V | Y | Y | C | T | R |
| huMA79b.v32 | Q | M | N | S | L | R | A | E | D | T | A | V | Y | Y | C | T | R |
| | | | | | | | | | | | | | | * | | * | * |
| | | | | | | | | | | | | | | | | H1 | 2 |

Kabat - CDR H3

Chothia - CDR H3

Contact - CDR H3

| Kabat# | 95 | 96 | 97 | 98 | 99 | 100 | A | B | C | D | E | F | G | H | I | J | K | 101 | 102 | 103 | 104 |
|---|---|---|---|---|---|---|---|---|---|---|---|---|---|---|---|---|---|---|---|---|---|
| **humIII** | G | | | | | | | | | | | | | | | | F | D | Y | W | G |
| **MA79b** | R | V | P | V | Y | | | | | | | | | | | | F | D | Y | W | G |
| huMA79b graft | R | V | P | V | Y | | | | | | | | | | | | F | D | Y | W | G |
| huMA79b.v17 | R | V | P | V | Y | | | | | | | | | | | | F | D | Y | W | G |
| huMA79b.v18 | R | V | P | I | R | | | | | | | | | | | | L | D | Y | W | G |
| huMA79b.v28 | R | V | P | I | R | | | | | | | | | | | | L | D | Y | W | G |
| huMA79b.v32 | R | V | P | I | R | | | | | | | | | | | | L | D | Y | W | G |
| | | | | | | | | | | | | | | | | | | | | * | |
| | 3 | 4 | 5 | 6 | 7 | | | | | | | | | | | | 8 | 9 | 10 | | |

| Kabat# | 105 | 106 | 107 | 108 | 109 | 110 | 111 | 112 | 113 | |
|---|---|---|---|---|---|---|---|---|---|---|
| **humIII** | Q | G | T | L | V | T | V | S | S | SEQ ID NO: 1 |
| **MA79b** | Q | G | T | S | V | T | V | S | S | SEQ ID NO: 3 |
| huMA79b graft | Q | G | T | L | V | T | V | S | S | SEQ ID NO: 5 |
| huMA79b.v17 | Q | G | T | L | V | T | V | S | S | SEQ ID NO: 7 |
| huMA79b.v18 | Q | G | T | L | V | T | V | S | S | SEQ ID NO: 9 |
| huMA79b.v28 | Q | G | T | L | V | T | V | S | S | SEQ ID NO: 11 |
| huMA79b.v32 | Q | G | T | L | V | T | V | S | S | SEQ ID NO: 13 |

*FIG. 1B*

VL Sequences

Kabat - CDR L1 (Kabat 24–34); Chothia - CDR L1 (Kabat 24–34); Contact - CDR L1 (Kabat 30–36)

| Kabat# | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | A | B | C | D | E | F | 28 | 29 | 30 | 31 | 32 | 33 | 34 | 35 | 36 | 37 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| hukI | D | I | Q | M | T | Q | S | P | S | S | L | S | A | S | V | G | D | R | V | T | I | T | C | R | A | S | Q | | | | | | | S | I | S | N | Y | L | A | W | Y | Q |
| MA79b | D | I | V | L | T | Q | S | P | A | S | L | A | V | S | L | G | Q | R | A | T | I | S | C | K | A | S | Q | S | V | D | Y | | | D | G | D | S | F | L | N | W | Y | Q |
| huMA79b graft | D | I | Q | M | T | Q | S | P | S | S | L | S | A | S | V | G | D | R | V | T | I | T | C | K | A | S | Q | S | V | D | Y | | | D | G | D | S | F | L | N | W | Y | Q |
| huMA79b.v17 | D | I | Q | L | T | Q | S | P | S | S | L | S | A | S | V | G | D | R | V | T | I | T | C | K | A | S | Q | S | V | D | Y | | | D | G | D | S | F | L | N | W | Y | Q |
| huMA79b.v18 | D | I | Q | L | T | Q | S | P | S | S | L | S | A | S | V | G | D | R | V | T | I | T | C | K | A | S | Q | S | V | D | Y | | | D | G | D | S | F | L | N | W | Y | Q |
| huMA79b.v28 | D | I | Q | L | T | Q | S | P | S | S | L | S | A | S | V | G | D | R | V | T | I | T | C | K | A | S | Q | S | V | D | Y | | | E | G | D | S | F | L | N | W | Y | Q |
| huMA79b.v32 | D | I | Q | L | T | Q | S | P | S | S | L | S | A | S | V | G | D | R | V | T | I | T | C | K | A | S | Q | S | V | D | Y | | | S | G | D | S | F | L | N | W | Y | Q |
| | | * | | * | | | | | | | | | | | | | | | | | | | | | | | | | | | | | | | | | | | | | * | * | |
| | | | | | | | | | | | | | | | | | | | | | | | | A1 | 2 | 3 | 4 | 5 | 6 | 7 | 8 | | | 9 | 10 | 11 | 12 | 13 | 14 | 15 | | | |

Kabat - CDR L2 (Kabat 50–56); Chothia - CDR L2 (Kabat 50–56); Contact - CDR L2 (Kabat 46–55)

| Kabat# | 38 | 39 | 40 | 41 | 42 | 43 | 44 | 45 | 46 | 47 | 48 | A | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| hukI | Q | K | P | G | K | A | P | K | L | L | I | | Y | A | A | S | S | L | E | S | G | V | P | S | R | F | S | G | S | G | S | G | T | D | F | T | L | T | I | S | S | L | Q | P |
| MA79b | Q | K | P | G | Q | P | P | K | L | F | I | | Y | A | A | S | N | L | E | S | G | I | P | A | R | F | S | G | S | G | S | G | T | D | F | T | L | N | I | H | P | V | E | E |
| huMA79b graft | Q | K | P | G | K | A | P | K | L | L | I | | Y | A | A | S | N | L | E | S | G | V | P | S | R | F | S | G | S | G | S | G | T | D | F | T | L | T | I | S | S | L | Q | P |
| huMA79b.v17 | Q | K | P | G | K | A | P | K | L | L | I | | Y | A | A | S | N | L | E | S | G | V | P | S | R | F | S | G | S | G | S | G | T | D | F | T | L | T | I | S | S | L | Q | P |
| huMA79b.v18 | Q | K | P | G | K | A | P | K | L | L | I | | Y | A | A | S | N | L | E | S | G | V | P | S | R | F | S | G | S | G | S | G | T | D | F | T | L | T | I | S | S | L | Q | P |
| huMA79b.v28 | Q | K | P | G | K | A | P | K | L | L | I | | Y | A | A | S | N | L | E | S | G | V | P | S | R | F | S | G | S | G | S | G | T | D | F | T | L | T | I | S | S | L | Q | P |
| huMA79b.v32 | Q | K | P | G | K | A | P | K | L | F | I | | Y | A | A | S | N | L | E | S | G | V | P | S | R | F | S | G | S | G | S | G | T | D | F | T | L | T | I | S | S | L | Q | P |
| | * | | | | | | * | | * | * | * | | * | | | | | | | | | | | | | | | * | | * | | * | * | | * | | | | | | | | | |
| | | | | | | | | | | | | | | B1 | 2 | 3 | 4 | 5 | 6 | 7 | | | | | | | | | | | | | | | | | | | | | | | | |

FIG. 2A

## VL Sequences

Kabat - CDR L3 (Kabat 89–97); Chothia - CDR L3 (Kabat 89–97); Contact - CDR L3 (Kabat 89–96)

| Kabat# | 81 | 82 | 83 | 84 | 85 | 86 | 87 | 88 | 89 | 90 | 91 | 92 | 93 | 94 | 95 | A | B | C | D | E | F | 96 | 97 | 98 | 99 | 100 | 101 | 102 | 103 | 104 | 105 | 106 | 107 | 108 | |
|---|---|---|---|---|---|---|---|---|---|---|---|---|---|---|---|---|---|---|---|---|---|---|---|---|---|---|---|---|---|---|---|---|---|---|---|
| **huKI** | E | D | F | A | T | Y | Y | C | Q | Q | Y | N | S | L | P | | | | | | | W | T | F | G | Q | G | T | K | V | E | I | K | R | SEQ ID NO: 2 |
| **MA79b** | E | D | A | A | T | Y | Y | C | Q | Q | S | N | E | D | P | | | | | | | L | T | F | G | A | G | T | E | L | E | L | K | R | SEQ ID NO: 4 |
| huMA79b graft | E | D | F | A | T | Y | Y | C | Q | Q | S | N | E | D | P | | | | | | | L | T | F | G | Q | G | T | K | V | E | I | K | R | SEQ ID NO: 6 |
| huMA79b.v17 | E | D | F | A | T | Y | Y | C | Q | Q | S | N | E | D | P | | | | | | | L | T | F | G | Q | G | T | K | V | E | I | K | R | SEQ ID NO: 8 |
| huMA79b.v18 | E | D | F | A | T | Y | Y | C | Q | Q | S | N | E | D | P | | | | | | | L | T | F | G | Q | G | T | K | V | E | I | K | R | SEQ ID NO: 10 |
| huMA79b.v28 | E | D | F | A | T | Y | Y | C | Q | Q | S | N | E | D | P | | | | | | | L | T | F | G | Q | G | T | K | V | E | I | K | R | SEQ ID NO: 12 |
| huMA79b.v32 | E | D | F | A | T | Y | Y | C | Q | Q | S | N | E | D | P | | | | | | | L | T | F | G | Q | G | T | K | V | E | I | K | R | SEQ ID NO: 14 |
| | | | | | | | * | | | | | | | | | | | | | | | | | * | | | | | | | | | | | |
| | | | | | | | | | C1 | 2 | 3 | 4 | 5 | 6 | 7 | | | | | | | 8 | 9 | | | | | | | | | | | | |

*FIG. 2B*

**huMA79bv28 Light Chain**

DIQLTQSPSSLSASVGDRVTITCKASQSVDYEGDSFLNWYQQKPGKAPKLLIYAASNLESGVPSRFSG
SGSGTDFTLTISSLQPEDFATYYCQQSNEDPLTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC **SEQ ID NO: 37**

**huMA79bv28 Heavy Chain**

EVQLVESGGGLVQPGGSLRLSCAASGYTFSSYWIEWVRQAPGKGLEWIGEILPGGGDTNYNEIFKGRA
TFSADTSKNTAYLQMNSLRAEDTAVYYCTRRVPIRLDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISKAK
GQPREPQVYTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPG **SEQ ID NO: 38**

***FIG. 3***

**Thio-huMA79b.v28-HC-A118C (HC)**

EVQLVESGGGLVQPGGSLRLSCAASGYTFSSYWIEWVRQAPGKGLEWIGEILPGGGDTNYNEIFKGRAT
FSADTSKNTAYLQMNSLRAEDTAVYYCTRRVPIRLDYWGQGTLVTVSS**C**STKGPSVFPLAPSSKSTSGG
TAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPG **SEQ ID NO: 39**

**Thio-huMA79b.v28-LC-V205C (LC)**

DIQLTQSPSSLSASVGDRVTITCKASQSVDYEGDSFLNWYQQKPGKAPKLLIYAASNLESGVPSRFSGSG
SGTDFTLTISSLQPEDFATYYCQQSNEDPLTFGQGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP**C**T
KSFNRGEC **SEQ ID NO: 54**

**Thio-huMA79b.v28-HC-S400C (HC)**

EVQLVESGGGLVQPGGSLRLSCAASGYTFSSYWIEWVRQAPGKGLEWIGEILPGGGDTNYNEIFKGRATF
SADTSKNTAYLQMNSLRAEDTAVYYCTRRVPIRLDYWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLD**C**DGSFFLYSKLTVDKSRWQQG
NVFSCSVMHEALHNHYTQKSLSLSPGK **SEQ ID NO: 55**

***FIG. 4***

**A** Thio huMA79bv28 HC A118C-MC-acetal-PNU-159682 (“huMA79bv28-PNU-2”)

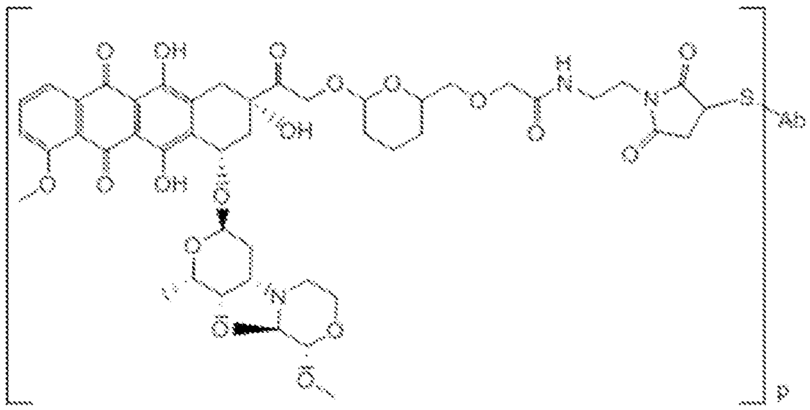

**B** Thio huMA79bv28 HC A118C-MC-val-cit-PAB-PNU-159682 (“huMA79bv28-PNU-1”)

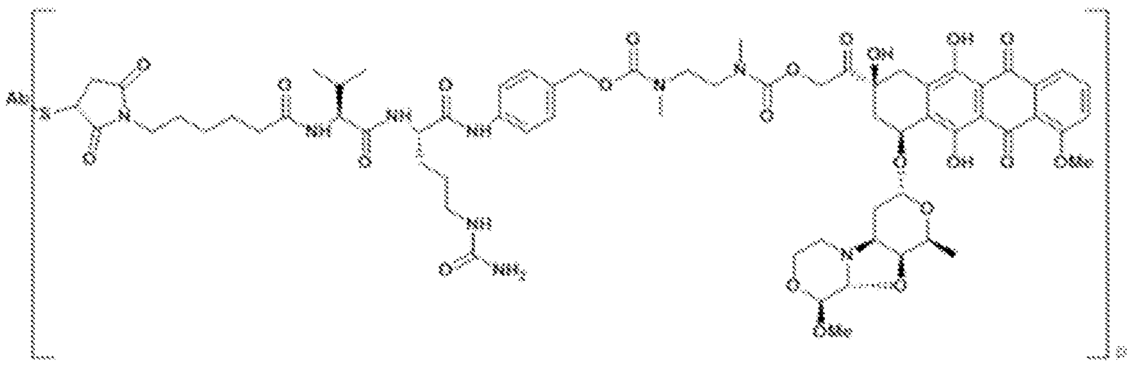

*FIG. 5*

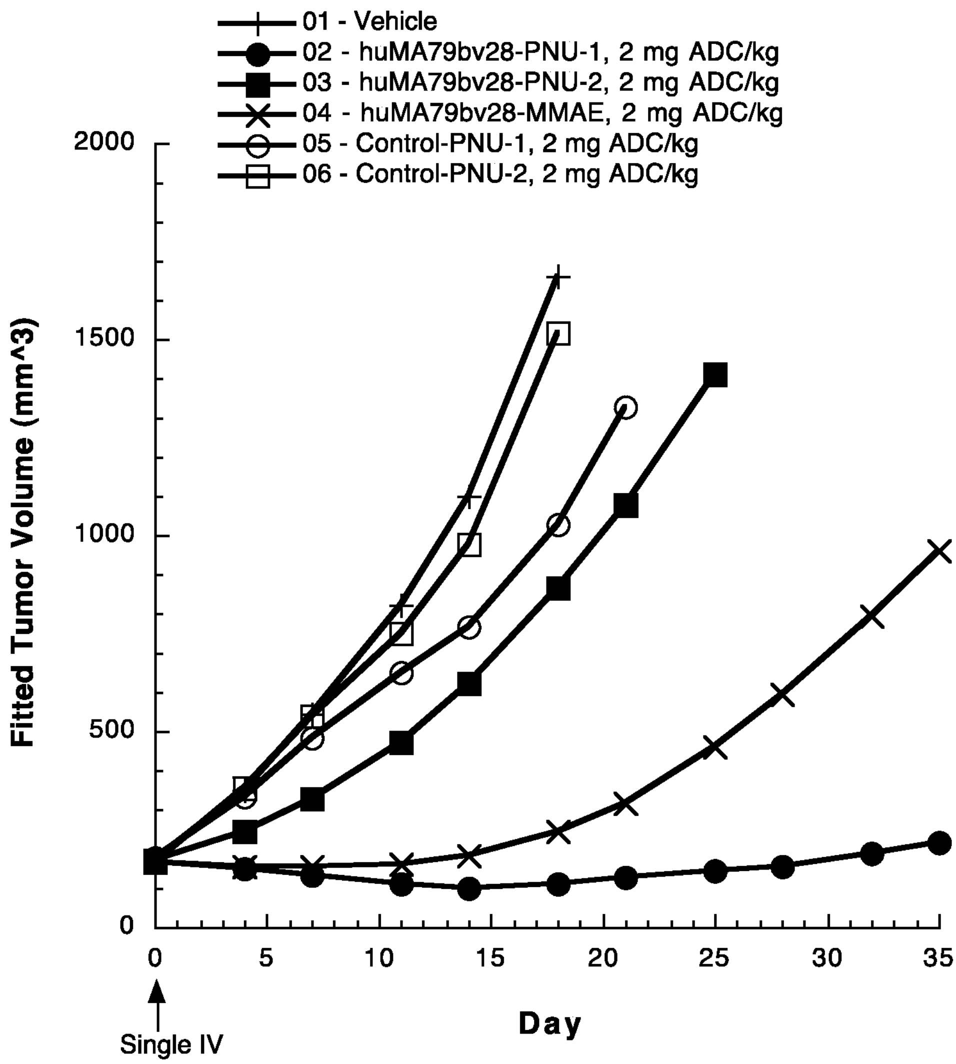
01 - Vehicle
02 - huMA79bv28-PNU-1, 2 mg ADC/kg
03 - huMA79bv28-PNU-2, 2 mg ADC/kg
04 - huMA79bv28-MMAE, 2 mg ADC/kg
05 - Control-PNU-1, 2 mg ADC/kg
06 - Control-PNU-2, 2 mg ADC/kg
Fitted Tumor Volume (mm^3)
2000
1500
1000
500
0
0
5
10
15
20
25
30
35
Day
Single IV

*FIG. 6*

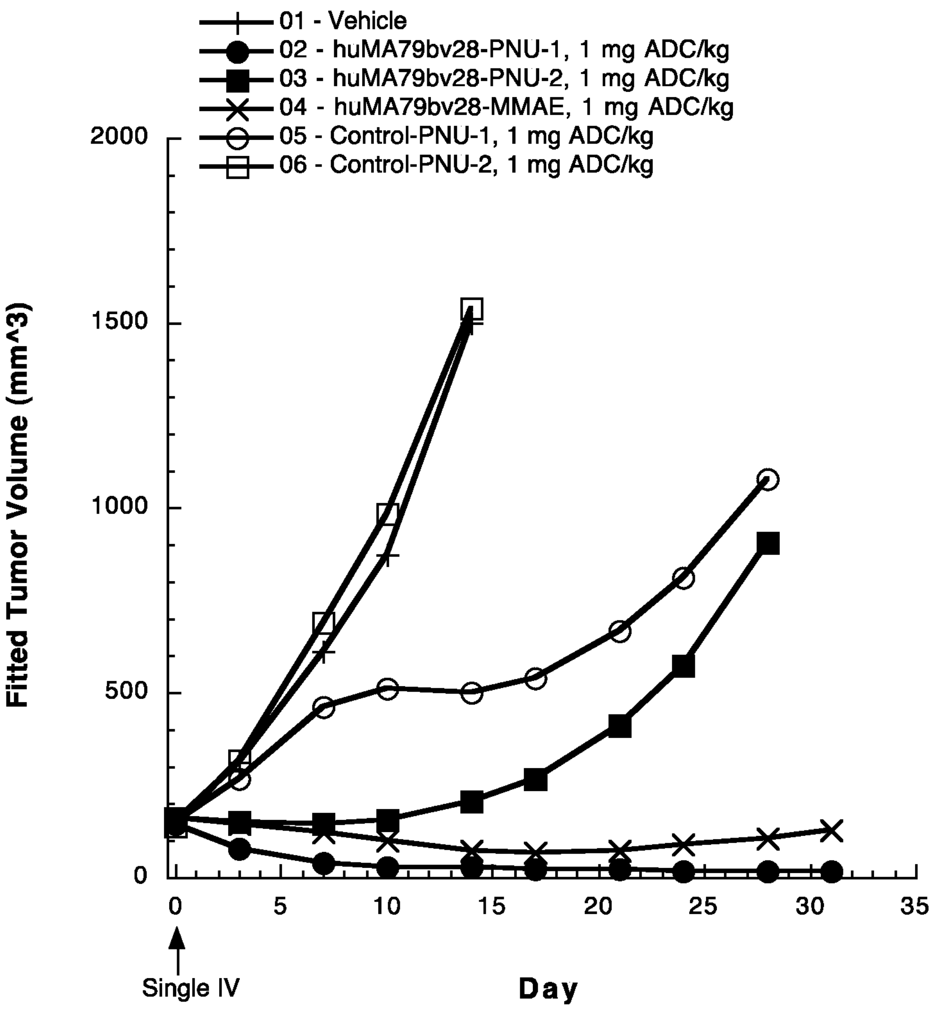
01 - Vehicle
02 - huMA79bv28-PNU-1, 1 mg ADC/kg
03 - huMA79bv28-PNU-2, 1 mg ADC/kg
04 - huMA79bv28-MMAE, 1 mg ADC/kg
05 - Control-PNU-1, 1 mg ADC/kg
06 - Control-PNU-2, 1 mg ADC/kg
Fitted Tumor Volume (mm^3)
2000
1500
1000
500
0
0
5
10
15
20
25
30
35
Single IV
Day

*FIG. 7*

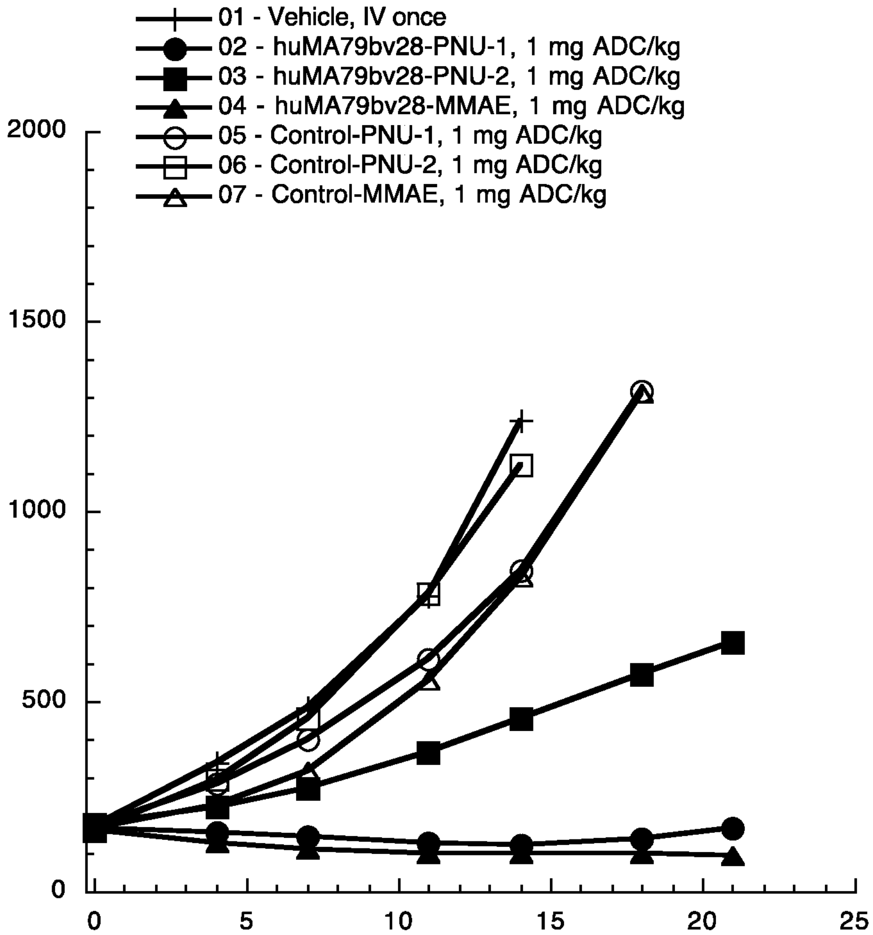
01 - Vehicle, IV once
02 - huMA79bv28-PNU-1, 1 mg ADC/kg
03 - huMA79bv28-PNU-2, 1 mg ADC/kg
04 - huMA79bv28-MMAE, 1 mg ADC/kg
05 - Control-PNU-1, 1 mg ADC/kg
06 - Control-PNU-2, 1 mg ADC/kg
07 - Control-MMAE, 1 mg ADC/kg
2000
1500
1000
500
0
0
5
10
15
20
25

*FIG. 8*

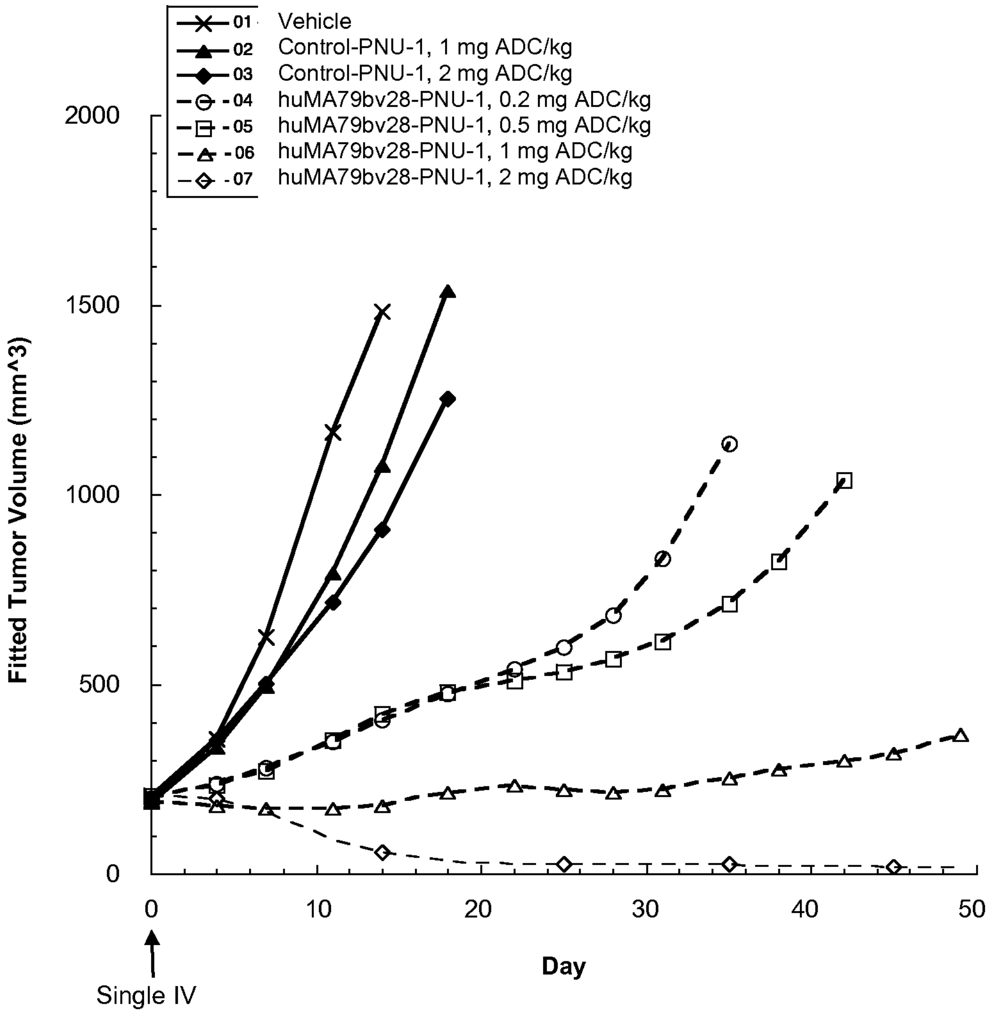
01 Vehicle
02 Control-PNU-1, 1 mg ADC/kg
03 Control-PNU-1, 2 mg ADC/kg
04 huMA79bv28-PNU-1, 0.2 mg ADC/kg
05 huMA79bv28-PNU-1, 0.5 mg ADC/kg
06 huMA79bv28-PNU-1, 1 mg ADC/kg
07 huMA79bv28-PNU-1, 2 mg ADC/kg
Fitted Tumor Volume (mm^3)
2000
1500
1000
500
0
0
10
20
30
40
50
Day
Single IV

*FIG. 9*

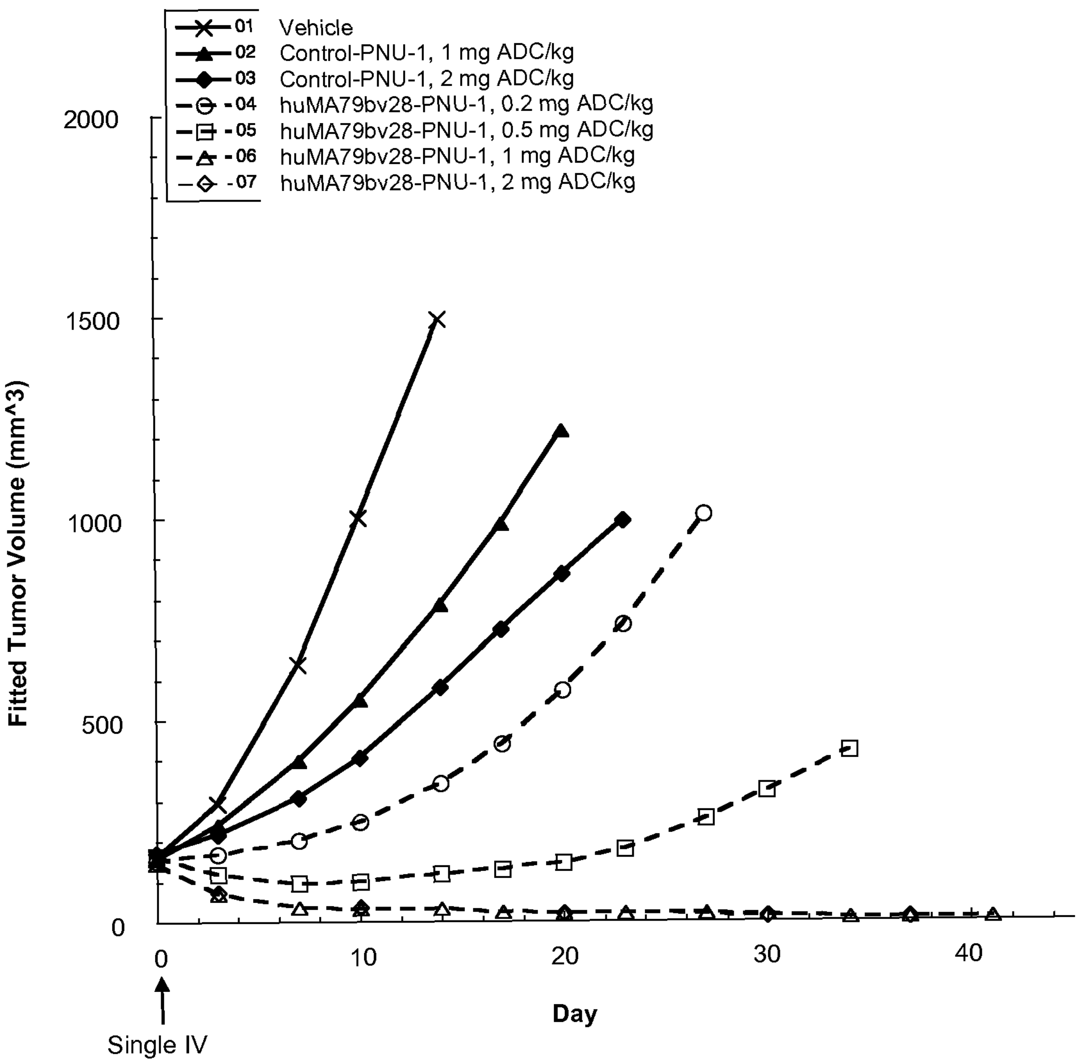
01 Vehicle
02 Control-PNU-1, 1 mg ADC/kg
03 Control-PNU-1, 2 mg ADC/kg
04 huMA79bv28-PNU-1, 0.2 mg ADC/kg
05 huMA79bv28-PNU-1, 0.5 mg ADC/kg
06 huMA79bv28-PNU-1, 1 mg ADC/kg
07 huMA79bv28-PNU-1, 2 mg ADC/kg
2000
1500
1000
500
0
Fitted Tumor Volume (mm^3)
0
10
20
30
40
Day
Single IV

*FIG. 10*

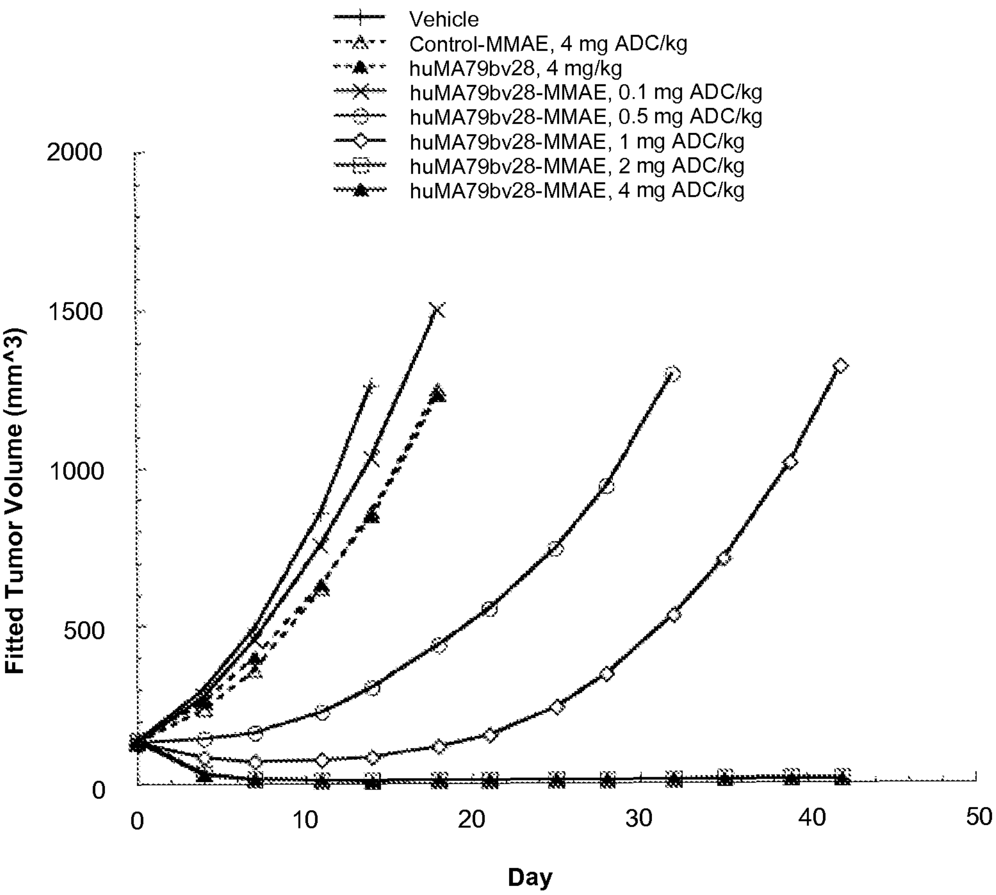
Vehicle
Control-MMAE, 4 mg ADC/kg
huMA79bv28, 4 mg/kg
huMA79bv28-MMAE, 0.1 mg ADC/kg
huMA79bv28-MMAE, 0.5 mg ADC/kg
huMA79bv28-MMAE, 1 mg ADC/kg
huMA79bv28-MMAE, 2 mg ADC/kg
huMA79bv28-MMAE, 4 mg ADC/kg
Fitted Tumor Volume (mm^3)
2000
1500
1000
500
0
0
10
20
30
40
50
Day

*FIG. 11*

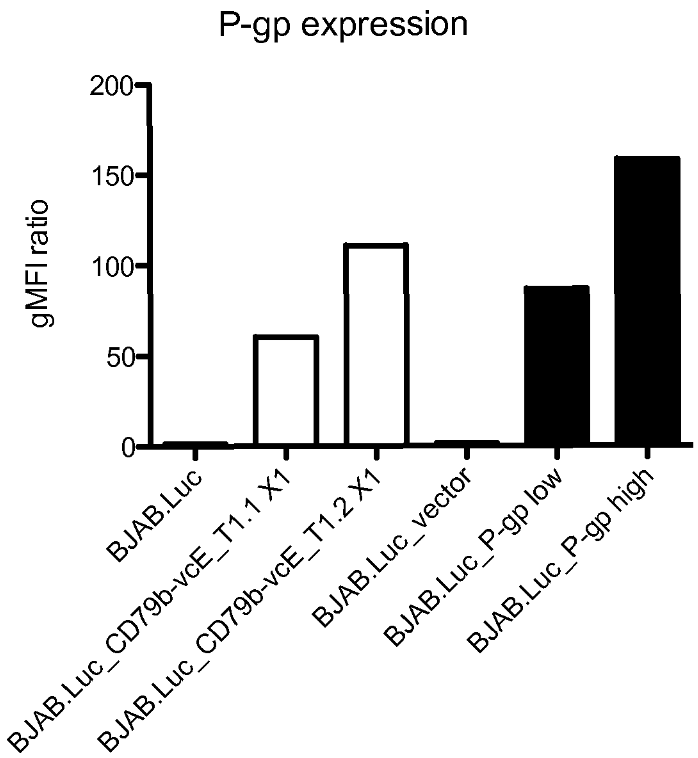
P-gp expression
gMFI ratio
200
150
100
50
0
BJAB.Luc
BJAB.Luc_CD79b-vcE_T1.1 X1
BJAB.Luc_CD79b-vcE_T1.2 X1
BJAB.Luc_vector
BJAB.Luc_P-gp low
BJAB.Luc_P-gp high

*FIG. 12*

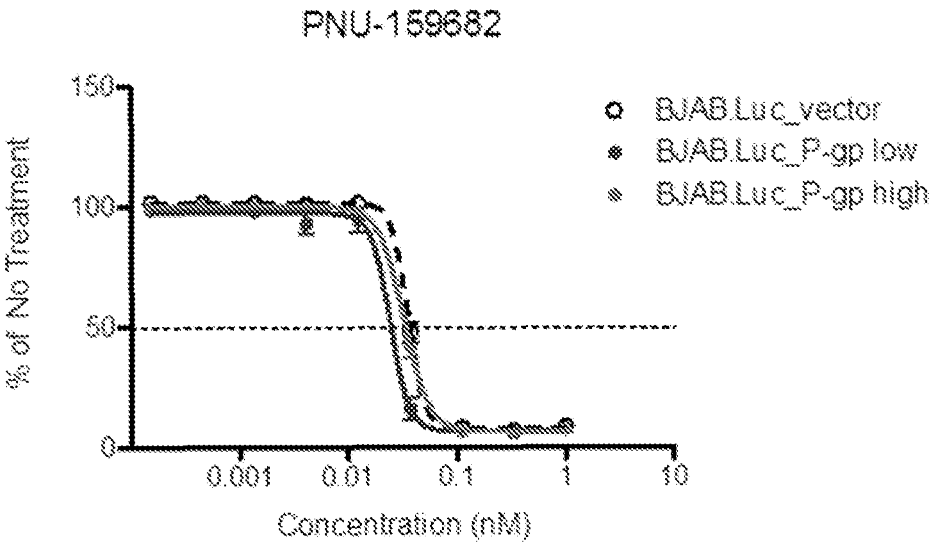
PNU-159682
% of No Treatment
150
100
50
0
0.001
0.01
0.1
1
10
Concentration (nM)
BJAB.Luc_vector
BJAB.Luc_P-gp low
BJAB.Luc_P-gp high

*FIG. 13*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8088378 B2
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 5500362 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 7498298 B
- US 7375078 B
- US 6214345 B
- WO 02088172 A
- US 2003130189 A
- US 2003096743 A
- WO 03026577 A
- WO 03043583 A
- WO 04032828 A
- WO 9411026 A
- EP 0328147 A
- US 6630579 B
- US 20110076287 A
- WO 2009099741 A

- US 20100034837 A
- WO 2010009124 A
- US 5362852 A
- US 4737456 A
- US 20050260186 A
- US 20060104968 A
- US 6267958 B
- US 6171586 B
- WO 2006044908 A
- US 6602677 B
- US 20080050310 A
- US 61726742 B
- US 61669268 B

**Non-patent literature cited in the description**


- **CABEZUDO et al.** *Haematologica,* 1999, vol. 84, 413-418
- **D'ARENA et al.** *Am. J. Hematol.,* 2000, vol. 64, 275-281
- **OLEJNICZAK et al.** *Immunol. Invest.,* 2006, vol. 35, 93-114
- **MATSUUCHI et al.** *Curr. Opin. Immunol.,* vol. 13 (3), 270-7
- **MATSUUCHI et al.** *Curr. Opin. Immunol.,* 2001, vol. 13 (3), 270-7
- Color Atlas of Clinical Hematology. Harcourt Publishers Ltd, 2000
- Agnew, Chem Intl. Ed. Engl. 1994, vol. 33, 183-186
- **KABAT et al.** Sequences of Proteins of Immunological Interest. Public Health Service, National Institutes of Health, 1991
- **KABAT et al.** Sequences of Proteins of Immunological Interest. NIH, 1991, vol. 1-3
- **CHOTHIA ; LESK.** *J. Mol. Biol.,* 1987, vol. 196, 901-917
- **ALMAGRO ; FRANSSON.** *Front. Biosci.,* 2008, vol. 13, 1619-1633
- **FLATMAN et al.** *J. Chromatogr. B,* 2007, vol. 848, 79-87
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **PAQUETTE, LEO A.** Principles of Modern Heterocyclic Chemistry. W.A. Benjamin, 1968
- The Chemistry of Heterocyclic Compounds, A series of Monographs. John Wiley & Sons, 1950
- *J. Am. Chem. Soc.,* 1960, vol. 82, 5566
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **T. W. GREENE.** Protective Groups in Organic Synthesis. John Wiley & Sons, 1991
- **MUNSON et al.** *Anal Biochem,* 1980, vol. 107, 220-239
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol,* 1991, vol. 147, 86

- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- **POLAKIS P.** *Current Opinion in Pharmacology,* 2005, vol. 5, 382-387
- **TEICHER, B.A.** *Current Cancer Drug Targets,* 2009, vol. 9, 982-1004
- **CARTER, P.J. ; SENTER P.D.** *The Cancer Jour.,* 2008, vol. 14 (3), 154-169
- **CHARI, R.V.** *Acc. Chem. Res.,* 2008, vol. 41, 98-107
- **LYON, R. et al.** *Methods in Enzym.,* 2012, vol. 502, 123-138
- **KLUSSMAN et al.** *Bioconjugate Chemistry,* 2004, vol. 15 (4), 765-773

- **CHARI et al.** *Cancer Research,* 1992, vol. 52, 127-131
- **DORONINA et al.** *Nat. Biotechnol.,* 2003, vol. 21, 778-784
- **E. SCHRÖDER ; K. LÜBKE.** The Peptides. Academic Press, 1965, vol. 1, 76-136
- **HAMANN et al.** *Expert Opin. Ther. Patents,* 2005, vol. 15, 1087-1103
- **HAY et al.** *Bioorg. Med. Chem. Lett.,* 1999, vol. 9, 2237
- **RODRIGUES et al.** *Chemistry Biology,* 1995, vol. 2, 223
- **STORM et al.** *J. Amer. Chem. Soc.,* 1972, vol. 94, 5815
- **AMSBERRY et al.** *J. Org. Chem.,* 1990, vol. 55, 5867
- **KINGSBURY et al.** *J. Med. Chem.,* 1984, vol. 27, 1447
- **SUN et al.** *Bioorganic & Medicinal Chemistry Letters,* 2002, vol. 12, 2213-2215
- **SUN et al.** *Bioorganic & Medicinal Chemistry,* 2003, vol. 11, 1761-1768
- **TOKI et al.** *J. Org. Chem.,* 2002, vol. 67, 1866-1872
- **DUBOWCHIK et al.** *Tetrahedron Letters,* 1997, vol. 38, 5257-60
- **WALKER, M.A.** *J. Org. Chem.,* 1995, vol. 60, 5352-5355
- **FRISCH et al.** *Bioconjugate Chem.,* 1996, vol. 7, 180-186
- **PETERSON et al.** Transport And Storage Of Anthracycline In Experimental Systems And Human Leukemia. *Anthracycline Antibiotics In Cancer Therapy*
- **N.R. BACHUR.** *Free Radical Damage,* 97-102
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **SALEH et al.** *J. Clin. Oncology,* 2000, vol. 18, 2282-2292
- **AJANI et al.** *Cancer Jour.,* 2000, vol. 6, 78-81
- **TOLCHER et al.** *J. Clin. Oncology,* 1999, vol. 17, 478-484
- **QUINTIERI et al.** *Clinical Cancer Research,* 2005, vol. 11 (4), 1608-1617
- **GRANDI et al.** *Cancer Treat. Rev.,* 1990, vol. 17, 133
- **RIPAMONTI et al.** *Brit. J. Cancer,* 1992, vol. 65, 703
- **SUN et al.** *Proceedings of the American Society for Clinical Oncology,* 2003, vol. 22
- **QUINTIERI.** Proceedings of the American Association of Cancer Research. 2003, vol. 44
- **PACCIARINI et al.** *Four. Clin. Oncology,* 2006, vol. 24, 14116
- **MCDONAGH et al.** *Prot. Engr. Design & Selection,* 2006, vol. 19 (7), 299-307
- **HAMBLETT et al.** *Clin. Cancer Res.,* 2004, vol. 10, 7063-7070
- Effect of drug loading on the pharmacology, pharmacokinetics, and toxicity of an anti-CD30 antibody-drug conjugate. **HAMBLETT, K.J. et al.** 2004 Annual Meeting, March 27-31, 2004, Proceedings of the AACR. American Association for Cancer Research, 27 March 2004, vol. 45
- Controlling the location of drug attachment in antibody-drug conjugates. **ALLEY, S.C. et al.** 2004 Annual Meeting, March 27-31, 2004, Proceedings of the AACR. American Association for Cancer Research, March 2004, vol. 45
- **GEOGHEGAN ; STROH.** *Bioconjugate Chem.,* 1992, vol. 3, 138-146
- **VAN DONGEN et al.** *The Oncologist,* 2007, vol. 12, 1379-1389
- **VEREL et al.** *J. Nucl. Med.,* 2003, vol. 44, 1271-1281
- Remington's Pharmaceutical Sciences. 1980
- **CROUCH et al.** *J. Immunol. Meth.,* 1993, vol. 160, 81-88
- **CREE et al.** *AntiCancer Drugs,* 1995, vol. 6, 398-404
- **PINHEIRO J et al.** nlme: linear and nonlinear mixed effects models. *R package,* 2009, 1-96